# FASCICULE DE BREVET EUROPEEN

(11) **EP 3 720 471 B1**
(45) Date de publication et mention de la délivrance du brevet: **29.11.2023**
(21) Numéro de dépôt: 18826205.9
(22) Date de dépôt: 07.12.2018
(51) Int. Cl.: A61K 38/26, A61K 47/34, A61K 9/08, A61P 3/10

(54) **COMPOSITIONS SOUS FORME D'UNE SOLUTION AQUEUSE INJECTABLE COMPRENANT DU GLUCAGON HUMAIN ET UN CO-POLYAMINOACIDE**
ZUSAMMENSETZUNGEN IN FORM EINER INJIZIERBAREN WÄSSRIGEN LÖSUNG MIT MENSCHLICHEM GLUCAGON UND EINER COPOLYAMINOSÄURE
COMPOSITIONS IN THE FORM OF AN INJECTABLE AQUEOUS SOLUTION COMPRISING HUMAN GLUCAGON AND A COPOLYAMINO ACID

(30) Priorité: 07.12.2017 FR 1761809; 07.12.2017 US 201762606137 P; 29.06.2018 FR 1855939
(43) Date de publication de la demande: 14.10.2020
(73) Titulaire: Adocia, 69003 Lyon (FR)
(72) Inventeur: CHAN, You-Ping, 69360 Ternay (FR); GEISSLER, Alexandre, 69007 Lyon (FR); NOEL, Romain, 69100 Villeurbanne (FR); CHARVET, Richard, 69140 Rillieux La Pape (FR); LAURENT, Nicolas, 01700 Miribel (FR)
(74) Mandataire: Tripoz, Inès
(86) Numéro de dépôt international: PCT/EP2018/084065
(87) Numéro de publication internationale: WO 2019/110837

(56) Documents cités:
- WO-A1-2013/104861
- WO-A1-2017/211918
- GB-A- 1 202 607

## Description

Le glucagon humain est une hormone hyperglycémiante d'action brève qui permet d'augmenter la glycémie, corrigeant ainsi un niveau hypoglycémique pouvant résulter d'un excès d'insuline. Il permet la libération de glucose par stimulation de la glycogénolyse hépatique, et possède des propriétés antagonistes de l'insuline (hypoglycémiante). Le glucagon humain est normalement sécrété par les cellules alpha des ilots de Langerhans dans le pancréas lorsqu'une hypoglycémie est détectée.

Le glucagon humain est utilisé à des fins thérapeutiques, comme le traitement d'urgence d'hypoglycémies sévères, encore appelé « rescue », mais également dans un cadre diagnostique lors de la réalisation d'examens médicaux, par exemple pour inhiber la motilité gastro-intestinale. D'autres applications sont également envisagées pour le glucagon humain, en particulier son utilisation dans un système de régulation bi-hormonal de la glycémie aussi appelé pancréas artificiel et dans l'hyperinsulinisme congénital qui est une maladie rare caractérisée par des niveaux très élevés d'insuline.

L'utilisation clinique du glucagon humain a été limitée à cause de certaines de ses propriétés peu favorables pour développer un produit pharmaceutique stable à visée thérapeutique. En effet, le glucagon humain présente une très faible solubilité à pH physiologique, une forte instabilité physique, à cause de sa propension à former des fibrilles sur une large gamme de pH. C'est pour cette raison que les seuls produits commerciaux à base de glucagon humain (Glucagen^{®}, NOVO NORDISK et Glucagon pour injection, ELI LILLY) sont des formes lyophilisées à reconstituer extemporanément.

Les travaux d'Onoue et al. (Pharm. Res. 2004, 21(7), 1274-83) ont montré le caractère potentiellement dangereux de ces fibrilles : le glucagon humain fibrillé étant cytotoxique dans des cellules de mammifères en culture.

Outre son instabilité physique, le glucagon humain subit divers types de dégradation chimique. En solution aqueuse, il se dégrade rapidement pour former plusieurs produits de dégradation. Au moins 16 produits de dégradation du glucagon humain ont été identifiés par Kirsh et al. (International Journal of Pharmaceutics, 2000, 203, 115-125). La dégradation chimique de ce glucagon humain est donc rapide et complexe.

La mauvaise stabilité chimique et physique du glucagon humain en solution a conduit des sociétés pharmaceutiques comme NOVO NORDISK, ELI LILLY et plus récemment FRESENIUS KABI à commercialiser ce glucagon humain sous la forme d'un lyophilisat à reconstituer à pH acide (pH<3) juste avant injection. Le glucagon humain sous forme de lyophilisat est plus stable, et la préparation de la formulation à pH acide juste avant utilisation permet d'obtenir une solution limpide. Cependant, une fois le produit reconstitué celui-ci doit être utilisé rapidement car il subit une dégradation chimique et physique extrêmement rapide dans le tampon acide de reconstitution, avec apparition de fibrilles de glucagon humain dans les 24 heures suivant la reconstitution, et/ou une gélification de la composition. Cette présentation du produit est cependant insatisfaisante car elle oblige à une utilisation très rapide de la formulation. Cette instabilité rend non seulement l'utilisation en pompe impossible, mais elle présente également l'inconvénient de conduire à des pertes de produit importantes dans l'utilisation diagnostique. En effet, une composition de ce type n'étant plus utilisable quelques heures après préparation cela cause du gaspillage.

Enfin, même dans l'application de traitement d'urgence des réactions hypoglycémiques sévères, pouvant survenir lors d'une insulinothérapie chez les patients diabétiques, la formulation à reconstituer n'est pas non plus idéale, car elle implique une préparation longue et compliquée, par exemple la notice de GlucaGen^{®} décrit un procédé en 5 étapes pour procéder à l'injection de la dose préconisée. D'ailleurs, une étude de la société LOCEMIA démontre que très peu de personnes (environ 10% des participants) devant réaliser la reconstitution dans l'urgence étaient capables de délivrer la dose adéquate. Enfin, le pH acide des solutions de glucagon humain peut générer des douleurs à l'injection chez le patient.

Il y a donc un besoin d'une solution de glucagon humain prête à l'emploi. Aujourd'hui, les solutions les plus avancées d'un point de vue clinique pour permettre la délivrance de glucagon humain contournent le problème de stabilité du glucagon humain en solution aqueuse de différentes manières.

La société LOCEMIA a mis au point un spray de glucagon humain lyophilisé, actuellement testé en étude clinique de phase 3, qui est destiné à être administré par voie intranasale. Ce spray est adapté à une utilisation dite « rescue », c'est-à-dire dans le cas d'une hypoglycémie sévère, car il est prêt à l'emploi et donc d'utilisation facile, contrairement aux solutions à reconstituer. Cependant, ce produit n'est pas adapté à une utilisation en pompe ou à une utilisation nécessitant un contrôle précis de la quantité de glucagon humain délivrée.

Pour sa part, XERIS a mis au point une formulation liquide du glucagon humain basée sur un solvant aprotique polaire, comme le DMSO, actuellement testée en études cliniques. Cependant, si l'injection de solution de solvants organiques pour une utilisation de type « rescue » est envisageable, il est largement préférable d'avoir une solution aqueuse de glucagon humain pour une utilisation chronique. Des compositions comprenant une association avec d'autres peptides sont envisagées notamment l'amyline ou un GLP-1 RA (Glucagon like peptide-1 receptor agonist).

Enfin, face aux difficultés de formulation du glucagon humain, des analogues du glucagon humain sont en cours de développement par des grandes sociétés pharmaceutiques, comme NOVO NORDISK, SANOFI OU ELI LILLY, afin d'obtenir des formulations ayant une stabilité compatible avec une utilisation pharmaceutique. Cependant, ces peptides dont la séquence primaire a été modifiée par rapport au peptide d'origine humaine peuvent présenter un risque de sécurité pour les patients.

Il y a donc un intérêt majeur pour une solution permettant d'améliorer la solubilisation et la stabilité, à la fois chimique et physique, du glucagon humain en solution aqueuse à un pH proche du pH physiologique, c'est-à-dire compris entre 6,0 et 8,0. Ceci pourrait permettre d'obtenir un produit pharmaceutique plus facilement utilisable par le patient en cas d'urgence, mais également d'ouvrir le champ à de nouvelles applications thérapeutiques du glucagon humain, comme par exemple son utilisation dans un pancréas artificiel bihormonal.

L'art antérieur propose des solutions pour tenter de résoudre ce problème.

Certains documents proposent de se placer à pH basique. Par exemple US2015291680 enseigne la solubilisation de glucagon humain à 1 mg/ml en se plaçant à un pH compris entre 8,8 et 9,4 et en utilisant de l'acide férulique ou le tétrahydrocurcumin. Cependant, outre le fait de se placer à pH basique, cette solution présente l'inconvénient de conduire à une stabilité du glucagon humain assez limitée dans le temps. L'article de Jackson et al (Curr. Diab. Rep., 2012, 12, 705-710) propose de formuler le glucagon humain à pH basique (environ 10) afin de limiter la formation de fibrilles. Cependant cette solution n'empêche pas une dégradation chimique rapide du glucagon humain.

La demande WO2014096440 (NOVOZYME) envisage au contraire de se placer à pH légèrement acide (environ 5,5) en présence d'albumine et de polysorbate, afin d'améliorer la stabilité en réduisant la vitesse de fibrillation. Cependant, cette solution présente une amélioration limitée de la stabilité. La plupart des solutions décrites dans l'art antérieur permettant d'obtenir une solution limpide de glucagon humain et de prévenir l'agrégation, la gélification ou la précipitation du glucagon humain impliquent l'utilisation de tensioactifs, de détergents ou d'agents solubilisant connus.

Par exemple, Matilainen et al (J. Pharm. Sci, 2008, 97, 2720-2729 et Eur. J. Pharm. Sci., 2009, 36, 412-420) a décrit l'utilisation de la cyclodextrine afin de limiter la vitesse de formation des fibrilles de glucagon humain. Cependant, l'amélioration apportée paraît insuffisante pour envisager une utilisation en pompe.

Parmi les solutions proposées figurent les tensioactifs hydrophiles :
- GB1202607 (NOVO NORDISK) décrit l'utilisation de détergents anioniques ou cationiques ;
- US6384016 (NOVO NORDISK) et US2011097386 (BIODEL) utilisent des lysophospholipides (ou lysolécithines) ;
- WO2015095389 (AEGIS) décrit des tensioactifs non-ioniques, comme le dodécyl maltoside, pour améliorer la biodisponibilité d'agents thérapeutiques, dans le cas de délivrance par application sur les muqueuses ou l'épiderme, et en particulier dans le cas de délivrance oculaire, nasale, orale ou nasolacrymale. Ce document décrit que la présence d'alkyles glycosides conduit à une amélioration de l'absorption du glucagon humain au niveau oculaire ;
- la demande WO2012059764 (ARECOR) décrit des tensioactifs cationiques, et plus précisément des chlorures d'ammonium aromatiques.

Les tensioactifs indiqués dans les documents ci-dessus peuvent être trop toxiques ou irritants pour une utilisation chronique par voie sous cutanée. Par exemple les lysophospholipides (ou lysolécithines) sont connus pour lyser les globules rouges du fait de leur propriétés hémolytiques. Lors d'une injection sous cutanée, cela peut provoquer des dommages locaux aux tissus et des douleurs au site d'injection. Dans le cas d'une injection en continue par une pompe, cela peut conduire à des douleurs et/ou à de l'irritation au niveau du site d'insertion de l'aiguille. La demande internationale WO2011138802 (Sun Pharma) décrit une solution prête à l'emploi de glucagon humain en solution aqueuse micellaire à un pH compris entre 5 et 7,5 en présence d'un lipide pegylé (pegylated distearoyl-phosphotidylethanolamine). Cependant, Garay et al. (Expert Opin Drug Deliv (2012) 9, 1319-1323) enseignent que le Poly Ethylène Glycol est à la fois immunogénique et antigénique. Ceci peut être préjudiciable aux patients présentant des anticorps anti-PEG. D'ailleurs, Ganson et al. (J. Allergy Clin. Immunol. (2015) doi:10.1016/j.jaci.2015.10.034) décrivent qu'une étude clinique portant sur de la pegnivacogin couplée à du méthoxypolyéthylène glycol (mPEG) de 40 kDa a conduit à des réponses inflammatoires dès la première dose de pegnivacogin sur 3 des 640 patients. Parmi ces trois patients deux remplissaient les critères d'anaphylaxie et un présentait une réaction dermale isolée, chaque évènement a été estimé sérieux, et l'un a même été estimé mettre la vie du patient en danger. Ces événements adverses ont causé l'arrêt de l'essai clinique et posent le problème des effets indésirables de composés pegylés.

Le document WO2013101749 (LATITUDE) décrit des nano-émulsions de glucagon humain. Cependant il revendique des performances assez modestes en termes de stabilité chimique, c'est-à-dire que la composition comprend au moins 75% de la concentration initiale après 3-7 jours à 37°C.

En outre, il est à noter, qu'à ce jour, à la connaissance de la demanderesse, aucune formulation pharmaceutique comprenant du glucagon humain sous forme de solution aqueuse n'est testée en étude clinique.

Il subsiste donc un besoin pour une formulation aqueuse liquide à un pH proche du pH physiologique compris entre 6,0 et 8,0 permettant de solubiliser et d'obtenir une bonne stabilité du glucagon humain, tant en termes de stabilité physique que de stabilité chimique. Plus particulièrement il existe un besoin pour une telle formulation qui puisse être utilisée dans une pompe bihormonale (insuline/glucagon humain).

Ce besoin est d'autant plus clair que Tan et al. (Diabètes, 2013, 62, 1131-138) montre que combiner le glucagon humain avec un GLP-1 RA est une proposition attractive de traitement de l'obésité et du diabète. Or, pouvoir formuler le glucagon humain de manière stable en solution aqueuse à un pH proche du pH physiologique compris entre 6,0 et 8,0 permet d'être dans des conditions plus favorables pour pouvoir améliorer la stabilité des GLP-1 RA sensibles aux conditions acides ou basiques.

Les co-polyaminoacides porteur de charges carboxylates et de radicaux hydrophobes Hy selon l'invention présentent une excellente résistance à l'hydrolyse. Ceci peut notamment être regardé en conditions accélérées, par exemple par des tests d'hydrolyse à pH basique (pH 12).

En outre des tests d'oxydation forcée, par exemple du type oxydation de fenton, montrent que les co-polyaminoacides porteur de charges carboxylates et de radicaux hydrophobes Hy présentent une bonne résistance à l'oxydation.

L'invention telle que définie dans les revendications concerne ainsi des compositions stables physiquement sous forme d'une solution aqueuse injectable, dont le pH est compris entre 6,0 et 8,0, comprenant au moins :
a) du glucagon humain et
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant choisis parmi les radicaux de formule X telle que définie ci-dessous : 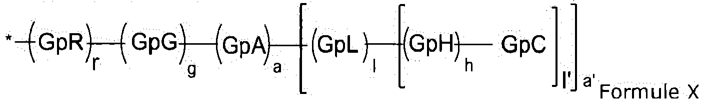 dans laquelle
   - GpR est choisi parmi les radicaux de formules VII, VII' ou VII" : 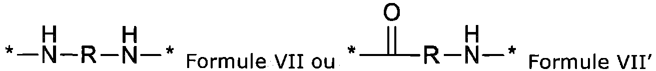 ou 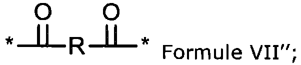
   - GpG et GpH identiques ou différents sont choisis parmi les radicaux de formules XI ou XI': 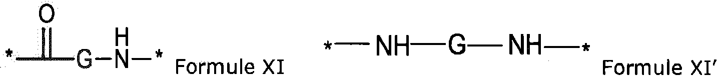
   - GpA est choisi parmi les radicaux de formule VIII 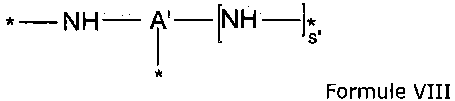 Dans laquelle A' est choisi parmi les radicaux de formule VIII',VIII" ou VIII‴ 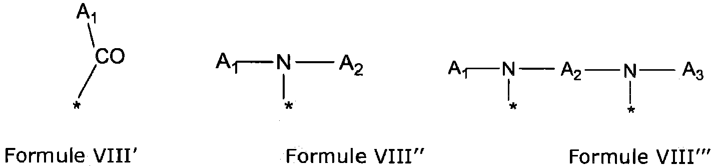
   - -GpL est choisi parmi les radicaux de formule XII 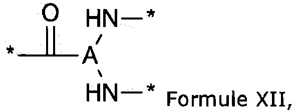
   - GpC est un radical de formule IX : 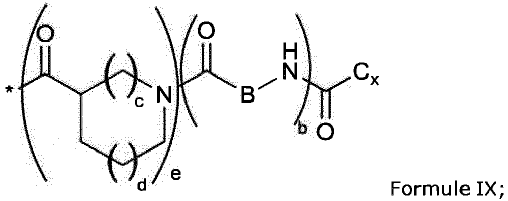
   - les * indiquent les sites de rattachement des différents groupes liés par des fonctions amides ;
   - a est un entier égal à 0 ou à 1 et a' = 1 si a = 0 et a' = 1, 2 ou 3 si a = 1 ;
      - a' est un entier égal à 1, à 2 ou à 3 ;
      - b est un entier égal à 0 ou à 1 ;
      - c est un entier égal à 0 ou à 1, et si c est égal à 0 alors d est égal à 1 ou à 2 ;
      - d est un entier égal à 0, à 1 ou à 2 ;
      - e est un entier égal à 0 ou à 1 ;
      - g est un entier égal à 0, à 1, à 2, à 3, à 4, à 5 ou à 6 ;
      - h est un entier égal à 0, à 1, à 2, à 3, à 4, à 5 ou à 6 ;
      - l est un entier égal à 0 ou 1 et l' = 1 si l = 0 et l' = 2 si l = 1 ;
      - r est un entier égal à 0, 1 ou à 2 ;
      - s' est un entier égal à 0 ou 1 ;
      - et si e est différent de 0 alors au moins un des g, h ou l est différent de 0 ;
      - et si a = 0 alors l = 0 ;
      - A, A₁, A₂ et A₃ identiques ou différents sont des radicaux alkyles linéaires ou ramifiés comprenant de 1 à 8 atomes de carbone et éventuellement substitué par un radical issu d'un cycle saturé, insaturé ou aromatique ;
      - B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone ;
      - Cₓ est un radical alkyl monovalent linéaire ou ramifié, éventuellement comprenant une partie cyclique, dans lequel x indique le nombre d'atomes de carbone et :
         ▪ Lorsque le radical hydrophobe -Hy porte 1 -GpC, alors 9 ≤ x ≤ 25,
         ▪ Lorsque le radical hydrophobe -Hy porte 2 -GpC, alors 9 ≤ x ≤ 15,
         ▪ Lorsque le radical hydrophobe -Hy porte 3 -GpC, alors 7 ≤ x ≤ 13,
         ▪ Lorsque le radical hydrophobe -Hy porte 4 -GpC, alors 7 ≤ x ≤ 11,
         ▪ Lorsque le radical hydrophobe -Hy porte au moins 5 -GpC alors, 6 ≤ x ≤ 11 ;
      - G est un radical alkyle ramifié de 1 à 8 atomes de carbone ledit radical alkyle portant une ou plusieurs fonction(s) acide carboxylique libre ;
      - R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène ;
      - le ou les radicaux hydrophobes -Hy de formule X étant liés au PLG:
         ∘via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur Hy' du radical hydrophobe -Hy, et
         ∘via une liaison covalente entre un atome d'azote du radical hydrophobe
      - Hy et un carbonyle porté par le PLG formant ainsi une fonction amide issue de la réaction d'une fonction amine du précurseur Hy' du radical hydrophobe -Hy et une fonction acide portée par le PLG ;
      - le ratio M entre le nombre de radicaux hydrophobes et le nombre d'unités glutamiques ou aspartiques étant compris entre 0 < M ≤ 0,5 ;
      - lorsque plusieurs radicaux hydrophobes sont portés par un co-polyaminoacide alors ils sont identiques ou différents ;
      - le degré de polymérisation DP en unités glutamiques ou aspartiques pour les chaines PLG est compris entre 5 et 250 ;
      - les fonctions acides carboxyliques libres étant sous forme de sel de cation alcalin choisi dans le groupe constitué par Na⁺ et K⁺.

L'invention concerne en outre en une méthode de préparation de compositions injectables stables.On entend par « soluble », susceptible de permettre de préparer une solution limpide et dépourvue de particules à une concentration inférieure à 60 mg/ml dans de l'eau distillée à 25 °C.

On entend par « solution » une composition liquide dépourvue de particules visibles, en utilisant la procédure conforme aux pharmacopées EP 8.0, au point 2.9.20, et US <790>.

On entend par « composition stable physiquement » des compositions qui après une certaine durée de stockage à une certaine température satisfont aux critères de l'inspection visuelle décrite dans la pharmacopée européenne, américaine et internationale, c'est-à-dire des compositions qui sont limpides et qui ne contiennent pas de particules visibles, mais également incolores.

On entend par « composition stable chimiquement » des compositions qui, après stockage un certain temps et à une certaine température, présentent une recouvrance minimum des principes actifs et sont conformes aux cahiers des charges applicables aux produits pharmaceutiques.

Une méthode classique pour mesurer les stabilités des protéines ou peptides consiste à mesurer la formation de fibrilles à l'aide de Thioflavine T, encore appelée ThT. Cette méthode permet de mesurer dans des conditions de température et d'agitation qui permettent une accélération du phénomène, le temps de latence avant la formation de fibrilles par mesure de l'augmentation de la fluorescence. Les compositions selon l'invention ont un temps de latence avant la formation de fibrilles nettement supérieur à celui du glucagon au pH d'intérêt.

On entend par « solution aqueuse injectable » des solutions à base d'eau qui répondent aux conditions des pharmacopées EP et US, et qui sont suffisamment liquides pour être injectées.

On entend par « co-polyaminoacide étant constitué d'unités glutamiques ou aspartiques » des enchainements linéaires non cycliques d'unités acide glutamique ou acide aspartique liées entre elles par des liaisons peptidiques, lesdits enchainements présentant une partie C-terminale, correspondant à l'acide carboxylique d'une extrémité, et une partie N-terminale, correspondant à l'amine de l'autre extrémité de l'enchainement.

On entend par « radical alkyl » une chaine carbonée, linéaire ou ramifiée, qui ne comprend pas d'hétéroatome.

Le co-polyaminoacide est un co-polyaminoacide statistique ou bloc.

Le co-polyaminoacide est un co-polyaminoacide statistique dans l'enchaînement des unités glutamiques et/ou aspartiques.

Tous les rattachements entre les différents groupes GpR, GpA, GpL, GpG et GpC sont des fonctions amides.

Les radicaux Hy, GpR, GpA, GpL, GpG et GpC, et D sont chacun indépendamment identiques ou différents d'une unité monomérique à l'autre.

Lorsque le co-polyaminoacide comprend une ou plusieurs d'unité(s) aspartique(s), celle(s)-ci peu(ven)t subir des réarrangements structuraux.

On entend par « radical alkyle » une chaine carbonée, linéaire ou ramifiée, qui ne comprend pas d'hétéroatome.

Le co-polyaminoacide est un co-polyaminoacide statistique dans l'enchaînement des unités glutamiques et/ou aspartiques.

Dans les formules les * indiquent les sites de rattachements des différents éléments représentés.

Dans un mode de réalisation, la composition selon l'invention est caractérisée en ce que Hy comprend entre 15 et 100 atomes de carbone.

Dans un mode de réalisation, la composition selon l'invention est caractérisée en ce que Hy comprend entre 30 et 70 atomes de carbone.

Dans un mode de réalisation, la composition selon l'invention est caractérisée en ce que Hy comprend entre 40 et 60 atomes de carbone.

Dans un mode de réalisation, la composition selon l'invention est caractérisée en ce que Hy comprend entre 20 et 30 atomes de carbone

Dans un mode de réalisation ledit au moins un radical hydrophobe -Hy est choisi parmi les radicaux de formule X telle que définie ci-dessous : 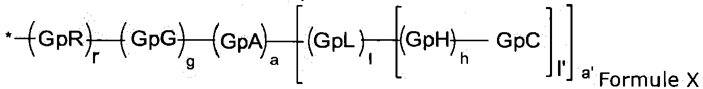 dans laquelle GpC est un radical de formule IX dans laquelle e=0 et GpC est un radical de formule IXa :

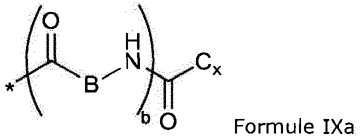

Dans un mode de réalisation ledit au moins un radical hydrophobe -Hy est choisi parmi les radicaux de formule X telle que définie ci-dessous : 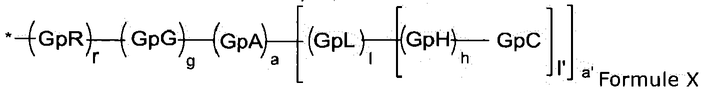 dans laquelle GpC est un radical de formule IX dans laquelle e=1, b = 0 et GpC est un radical de formule IXd :

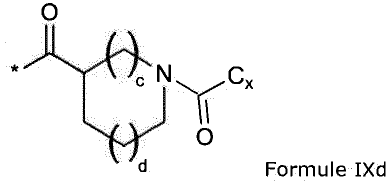

Dans un mode de réalisation ledit au moins un radical hydrophobe -Hy est choisi parmi les radicaux de formule X telle que définie ci-dessous : 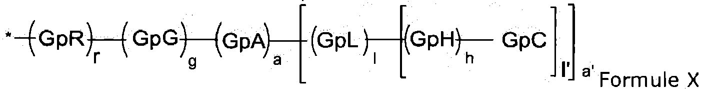 dans laquelle GpC est un radical de formule IX dans laquelle e=1 et GpC est un radical de formule IXb :

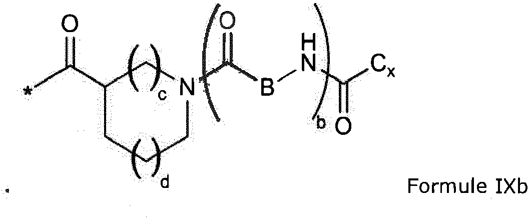

Dans un mode de réalisation, au moins un des g, h ou l est différent de 0.

Dans un mode de réalisation, lorsque r=2 alors le groupe GpR lié au PLG est choisi parmi les GpR de formule VII.

Dans un mode de réalisation, lorsque r=2 alors le groupe GpR lié au PLG est choisi parmi les GpR de formule VII et le deuxième GpR est choisi parmi les GpR de formule VII".

Dans un mode de réalisation, un mode de réalisation, lorsque r=2 alors le groupe GpR lié au PLG est choisi parmi les GpR de formule VII".

Dans un mode de réalisation, un mode de réalisation, lorsque r=2 alors le groupe GpR lié au PLG est choisi parmi les GpR de formule VII" et le deuxième GpR est choisi parmi les GpR de formule VII.

Dans un mode de réalisation, a =0

Dans un mode de réalisation g+h≥2.

Dans un mode de réalisation g est supérieur ou égal à 2 (g≥2).

Dans un mode de réalisation h est supérieur ou égal à 2 (h≥2).

Dans un mode de réalisation, g+h≥2 et a et l sont égaux à 0 (a=l=0).

Dans un mode de réalisation, g+h≥2 et b est égal à 0 (b=0).

Dans un mode de réalisation g ou h est supérieur ou égal à 2 (g≥2) et b est égal à 0.

Dans un mode de réalisation, g+h≥2, b est égal à 0 (b=0) et e est égal à 1 (e=1).

Dans un mode de réalisation g ou h est supérieur ou égal à 2 (g≥2) b est égal à 0 (b=0) et e est égal à 1 (e=1).

Dans un mode de réalisation, au moins un des g, h ou l est différent de 0.

Dans un mode de réalisation, au plus un des g, h ou l est différent de 0.

Dans un mode de réalisation, au moins un des g ou h est égal à 1.

Dans un mode de réalisation, a = 1 et l = 1.

Dans un mode de réalisation, si l = 0, au moins un des g ou h est égal à 0.

Dans un mode de réalisation, si l = 1, au moins un des g ou h est égal à 0.

Dans un mode de réalisation, ledit au moins un radical hydrophobe -Hy est choisi parmi les radicaux de formule X dans laquelle r = 2 de formule Xc', telle que définie ci-dessous : 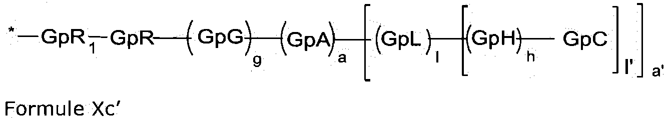 dans laquelle GpR₁ est un radical de formule VII. 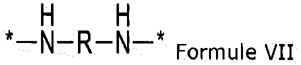 dans laquelle GpR, GpG, GpA, GpL, GpH, GpC, R, a, a', g, h, l et l' ont les définitions données précédemment.

Dans un mode de réalisation, ledit au moins un radical hydrophobe -Hy est choisi parmi les radicaux de formule X dans laquelle r = 2 de formule Xc', telle que définie ci-dessous :
: 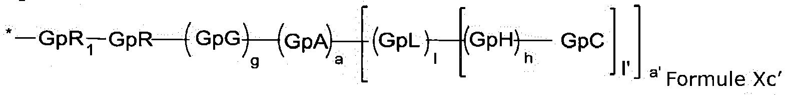 dans laquelle GpR₁ est un radical de formule VII". 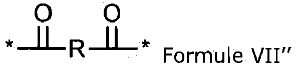 dans laquelle GpR, GpG, GpA, GpL, GpH, GpC, R, a, a', g, h, l et l' ont les définitions données précédemment.

Dans un mode de réalisation, ledit au moins un radical hydrophobe -Hy est choisi parmi les radicaux de formule X dans laquelle
- l = 0,
- de formule Xb' telle que définie ci-dessous 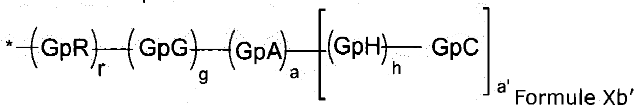 dans laquelle
- GpR est choisi parmi les radicaux de formules VII, VII' ou VII" : 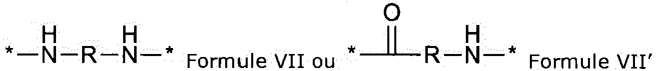 ou 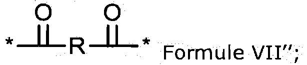
- GpG est choisi parmi les radicaux de formule XI ou XI' : 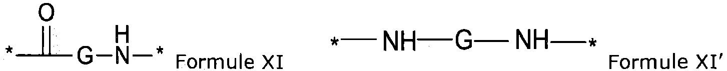
- GpA est choisi parmi les radicaux de formule VIII dans laquelle s' = 1 représentée par la formule VIIIa ou de formule VIII dans laquelle s' = 0 représentée par la formule VIIIb : 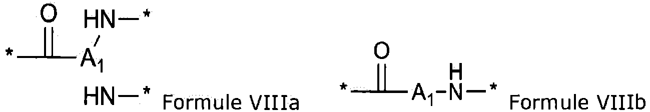
- GpC est un radical de formule IX : 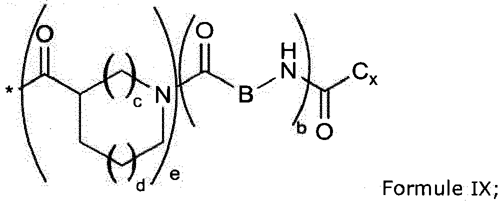
- les * indiquent les sites de rattachement des différents groupes liés par des fonctions amides ;
- a est un entier égal à 0 ou à 1 et a' = 1 si a = 0 et a' = 1 ou a' = 2 si a = 1 ;
   - a' est un entier égal à 1 ou 2 et
      ∘ si a' est égal à 1 alors a est égal à 0 ou à 1 et GpA est un radical de formule VIIIb et,
      ∘ si a' est égal à 2 alors a est égal à 1, et GpA est un radical de formule VIIIa;
- b est un entier égal à 0 ou à 1 ;
- c est un entier égal à 0 ou à 1, et si c est égal à 0 alors d est égal à 1 ou à 2 ;
- d est un entier égal à 0, à 1 ou à 2 ;
- e est un entier égal à 0 ou à 1 ;
   - g est un entier égal à 0, à 1, à 2, à 3 à, 4, à 5 ou à 6 ;
   - h est un entier égal à 0, à 1, à 2, à 3, à 4, à 5 ou à 6, r est un entier égal à 0, 1 ou à 2, et
   - s' est un entier égal à 0 ou 1 ;
   - et si e est différent de 0 alors au moins un des g ou h est différent de 0 ;
   - A₁ est un radical alkyle linéaire ou ramifié comprenant de 1 à 8 atomes de carbone et éventuellement substitué par un radical issu d'un cycle saturé, insaturé ou aromatique;
   - B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone ;
   - Cₓ est un radical alkyl monovalent linéaire ou ramifié, éventuellement comprenant une partie cyclique, dans lequel x indique le nombre d'atomes de carbone et :
      ▪ lorsque le radical hydrophobe -Hy porte 1 -GpC, alors 9 ≤ x ≤ 25 ;
      ▪ lorsque le radical hydrophobe -Hy porte 2 -GpC, alors 9 ≤ x ≤ 15 ;
      ▪ lorsque le radical hydrophobe -Hy porte 3 -GpC, alors 7 ≤ x ≤ 13 ;
      ▪ lorsque le radical hydrophobe -Hy porte 4 -GpC, alors 7 ≤ x ≤ 11 ;
      ▪ lorsque le radical hydrophobe -Hy porte au moins 5 -GpC alors, 6 ≤ x ≤ 11 ;
   - G est un radical alkyle ramifié de 1 à 8 atomes de carbone ledit radical alkyle portant une ou plusieurs fonction(s) acide carboxylique libre ;
   - R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène :
   - le ou les radicaux hydrophobes Hy de formule X étant liés au PLG :
      ▪ via une liaison covalente entre un carbonyle du radical hydrophobe et un atome d'azote porté par le PLG formant ainsi une fonction amide issue de la réaction d'une fonction amine portée par le PLG et une fonction acide portée par le précurseur du radical hydrophobe , et
      ▪ via une liaison covalente entre un atome d'azote du radical hydrophobe et un carbonyle porté par le PLG, formant ainsi une fonction amide issue de la réaction d'une fonction amine du précurseur Hy' du radical hydrophobe et une fonction acide portée par le PLG.

      - le ratio M entre le nombre de radicaux hydrophobes et le nombre d'unités glutamiques ou aspartiques étant compris entre 0 < M ≤ 0,5 ;
      - lorsque plusieurs radicaux hydrophobes sont portés par un co-polyaminoacide alors ils sont identiques ou différents,
      - le degré de polymérisation DP en unités glutamiques ou aspartiques pour les chaînes PLG est compris entre 5 et 250 ;
      - les fonctions acides carboxyliques libres étant sous forme de sel de cation alcalin choisi dans le groupe constitué par Na⁺ et K⁺.

Dans un mode de réalisation ledit au moins un radical hydrophobe -Hy est choisi parmi les radicaux de formule X telle que définie ci-dessous dans laquelle l = 0,
- GpA est choisi parmi les radicaux de formule VIII dans laquelle s' = 1 et A' choisi parmi les radicaux de formule VIII" ou VIII‴,: 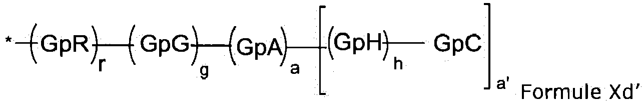 dans laquelle
- GpR est choisi parmi les radicaux de formules VII, VII' ou VII" : 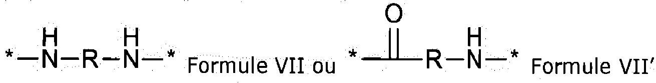 ou 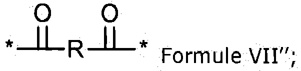
- GpG est choisi parmi les radicaux de formule XI ou XI': 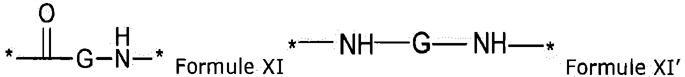
- GpA est choisi parmi les radicaux de formules, VIIIc ou VIIId : 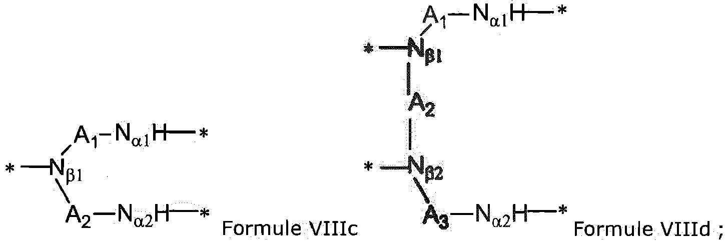
- GpC est un radical de formule IX : 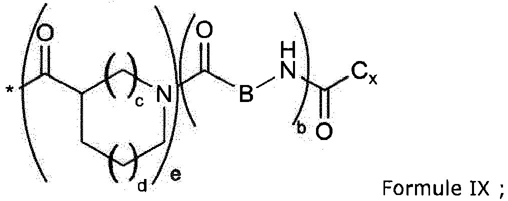
- les * indiquent les sites de rattachement des différents groupes liés par des fonctions amides ;
- a est un entier égal à 0 ou à 1 et a' = 1 si a = 0 et a' = 2 ou 3 si a = 1 ;
- a' est un entier égal à 2 ou à 3 et
   ∘ si a' est égal à 1 alors a est égal à 0 et,
   ∘ si a' est égal à 2 ou 3 alors a est égal à 1, et GpA est un radical de formule VIIIc ou VIIId ;
- b est un entier égal à 0 ou à 1 ;
- c est un entier égal à 0 ou à 1, et si c est égal à 0 alors d est égal à 1 ou à 2 ;
- d est un entier égal à 0, à 1 ou à 2 ;
- e est un entier égal à 0 ou à 1 ;
- g est un entier égal à 0, à1, à 2, à 3 à 4 à 5 ou à 6 ;
- h est un entier égal à 0, à 1, à 2, à 3 à 4 à 5 ou à 6 ;
- r est un entier égal à 0, 1 ou à 2, et
- s' est un entier égal à 1 ;
- et si e est différent de 0 alors au moins un des g ou h est différent de 0 ;
- A₁, A₂, A₃ identiques ou différents sont des radicaux alkyles linéaires ou ramifiés comprenant de 1 à 8 atomes de carbone et éventuellement substitué par un radical issu d'un cycle saturé, insaturé ou aromatique;
- B est un radical éther ou polyéther non substitué comprenant de 4 à 14 atomes de carbone et de 1 à 5 atomes d'oxygène éventuellement comprenant un noyau aromatique, comprenant de 1 à 9 atomes de carbone ;
- Cₓ est un radical alkyl monovalent linéaire ou ramifié, éventuellement comprenant une partie cyclique, dans lequel x indique le nombre d'atomes de carbone et :
   ▪ lorsque le radical hydrophobe -Hy porte 1 -GpC, alors 9 ≤ x ≤ 25 ;
   ▪ lorsque le radical hydrophobe -Hy porte 2 -GpC, alors 9 ≤ x ≤ 15 ;
   ▪ lorsque le radical hydrophobe -Hy porte 3 -GpC, alors 7 ≤ x ≤ 13 ;
   ▪ lorsque le radical hydrophobe -Hy porte 4 -GpC, alors 7 ≤ x ≤ 11 ;
   ▪ lorsque le radical hydrophobe -Hy porte au moins 5 -GpC alors, 6 ≤ x ≤ 11.
- le ou les radicaux hydrophobes Hy de formule X étant liés au PLG :
   ∘ via une liaison covalente entre un carbonyle du radical hydrophobe et un atome d'azote porté par le PLG formant ainsi une fonction amide issue de la réaction d'une fonction amine portée par le PLG et une fonction acide portée par le précurseur Hy' du radical hydrophobe , et
   ∘ via une liaison covalente entre un atome d'azote du radical hydrophobe et un carbonyle porté par le PLG, formant ainsi une fonction amide issue de la réaction d'une fonction amine du précurseur Hy' du radical hydrophobe et une fonction acide portée par le PLG.
- G est un radical alkyle ramifié de 1 à 8 atomes de carbone ledit radical alkyle portant une ou plusieurs fonction(s) acide carboxylique libre,
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions -CONH₂ ou un radical éther ou polyéther non substitué comprenant de 4 à 14 atomes de carbone et de 1 à 5 atomes d'oxygène :
   - le ratio M entre le nombre de radicaux hydrophobes et le nombre d'unités glutamiques ou aspartiques étant compris entre 0 < M≤ 0,5 ;
   - lorsque plusieurs radicaux hydrophobes sont portés par un co-polyaminoacide alors ils sont identiques ou différents,
   - le degré de polymérisation DP en unités glutamiques ou aspartiques pour les chaînes PLG est compris entre 5 et 250 ;
   - les fonctions acides carboxyliques libres étant sous forme de sel de cation alcalin choisi dans le groupe constitué par Na⁺ et K⁺.

Dans un mode de réalisation, lorsque a' = 1, x est compris entre 11 et 25 (11 ≤ x ≤ 25). En particulier, lorsque x est compris entre 15 et 16 (x = 15 ou 16) alors r = 1 et R est un radical éther ou polyéther et lorsque x est supérieur à 17 (x ≥ 17) alors r = 1 et R est un radical éther ou polyéther.

Dans un mode de réalisation, lorsque a' = 2, x est compris entre 9 et 15 (9 ≤ x ≤ 15).

Dans un mode de réalisation, ledit au moins un radical hydrophobe -Hy est choisi parmi les radicaux de formule X dans laquelle a =1 et a' = 1 de formule Xa telle que définie ci-dessous : 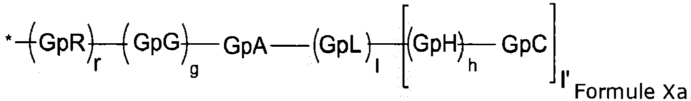 dans laquelle GpA est un radical de formule VIII et A' est choisi parmi les radicaux de formule VIII' avec s'=0 et GpA est un radical de Formule VIIIb 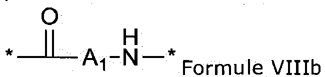 Et GpR, GpG, GpL, GpH, GpC A₁, r, g, h, l et l' ont les définitions données précédemment.

Dans un mode de réalisation, ledit au moins un radical hydrophobe -Hy est choisi parmi les radicaux de formule X dans laquelle a =1 de formule Xb telle que définie ci-dessous : 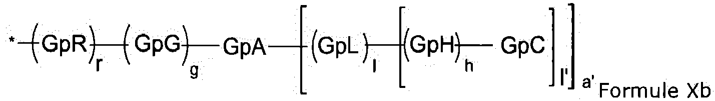 dans laquelle GpA est un radical de formule VIII et A' est choisi parmi les radicaux de formule VIII' avec s' = 1 et GpA est un radical de Formule VIIIa 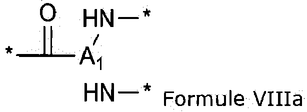 Et GpR, GpG, GpL, GpH, GpC, A₁, a', r, g, h, l et l' ont les définitions données précédemment.

Dans un mode de réalisation, ledit au moins un radical hydrophobe -Hy est choisi parmi les radicaux de formule X dans laquelle a =1 telle que définie ci-dessous : 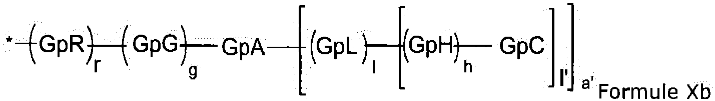 dans laquelle GpA est un radical de formule VIII et A est choisi parmi les radicaux de formule VIII" avec s'=1 et GpA est un radical de Formule VIIIc 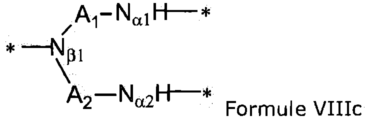 Et GpR, GpG, GpL, GpH, GpC, A₁, A₂, r, g, h, a', l et l' ont les définitions données précédemment.

Dans un mode de réalisation, ledit au moins un radical hydrophobe -Hy est choisi parmi les radicaux de formule X dans laquelle a =1 telle que définie ci-dessous : 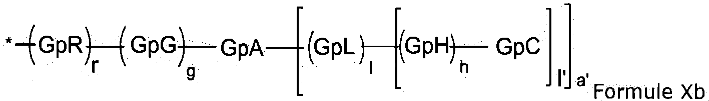 dans laquelle GpA est un radical de formule VIII et A est choisi parmi les radicaux de formule VIII‴ avec s'=1, et GpA est un radical de formule VIIId 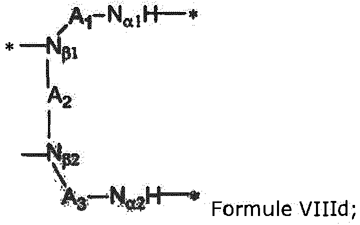 Et GpR, GpG, GpL, GpH, GpC, A₁, A₂, A₃, a', r, g, h, l et l' ont les définitions données précédemment.

Dans un mode de réalisation, ledit au moins un radical hydrophobe -Hy est choisi parmi les radicaux de formule X dans laquelle r=1 de formule Xc, telle que définie ci-dessous : 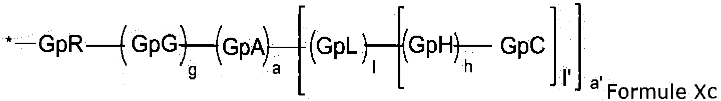 dans laquelle GpR est un radical de formule VII : 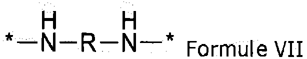 Et GpG, GpA, GpL, GpH, GpC, R, a, g, h, l, a' et l' ont les définitions données précédemment.

Dans un mode de réalisation, ledit au moins un radical hydrophobe -Hy est choisi parmi les radicaux de formule X dans laquelle r=1 de formule Xc telle que définie ci-dessous : 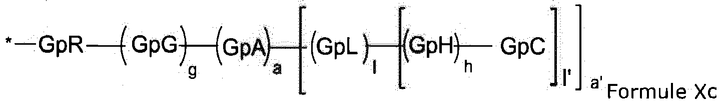 dans laquelle GpR est un radical de formule VII' : 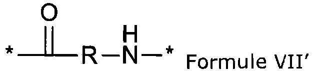 Et GpG, GpA, GpL, GpH, GpC, R, a, g, h, l, a' et l' ont les définitions données précédemment.

Dans un mode de réalisation, ledit au moins un radical hydrophobe -Hy est choisi parmi les radicaux de formule X dans laquelle r=1 de formule Xc telle que définie ci-dessous : 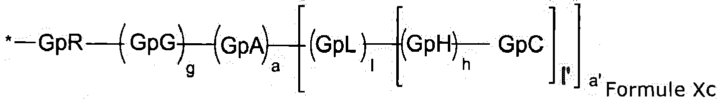 dans laquelle GpR est un radical de formule VII" : 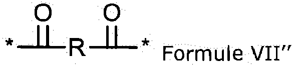 Et GpG, GpA, GpL, GpH, GpC, R, a, g, h, l, a' et l' ont les définitions données précédemment.

Dans un mode de réalisation ledit au moins un radical hydrophobe -Hy est choisi parmi les radicaux de formule X dans laquelle r, g, a, I, h sont égaux à 0, de formule Xd telle que définie ci-dessous :

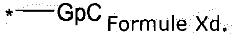

Dans un mode de réalisation ledit au moins un radical hydrophobe -Hy est choisi parmi les radicaux de formule X dans laquelle r, g, a, l, h sont égaux à 0, de formule Xd telle que définie ci-dessous : 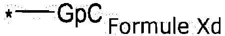 dans laquelle GpC est un radical de formule IX dans laquelle e=0, b= 0 et GpC est un radical de formule IXe :

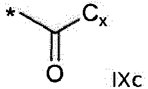

Dans un mode de réalisation, la composition selon l'invention est caractérisée en ce que lesdits radicaux hydrophobes sont choisis parmi les radicaux hydrophobes de formule X dans laquelle GpA est un radical de formule VIIIb, a' = 1 et l = 0 représentée par la formule Xe suivante : 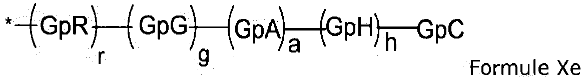 GpR, GpG, GpA, GpH, GpC, r, g, h, et a ont les définitions données précédemment.

Dans un mode de réalisation, la composition selon l'invention est caractérisée en ce que lesdits radicaux hydrophobes sont choisis parmi les radicaux hydrophobes de formule X dans laquelle, a' = 2 et a = 1 et l = 0 représentée par la formule Xf suivante : 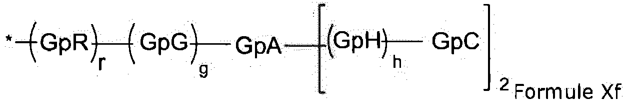 GpR, GpG, GpA, GpH, GpC, r, g et h ont les définitions données précédemment.

Dans un mode de réalisation, la composition selon l'invention est caractérisée en ce que lesdits radicaux hydrophobes sont choisis parmi les radicaux hydrophobes de formule X dans laquelle h = 0, l = 0 et l' = 1 représentée par la formule Xg suivante : 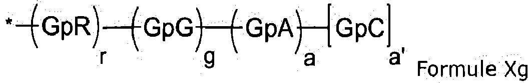 GpR, GpG, GpA, GpC, r, g, a et a' ont les définitions données précédemment.

Dans un mode de réalisation, la composition selon l'invention est caractérisée en ce que lesdits radicaux hydrophobes sont choisis parmi les radicaux hydrophobes de formule X dans laquelle h = 0, a'= 1 représentée par la formule Xh suivante : 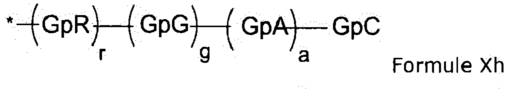 GpR, GpG, GpA, GpC, r, a et g ont les définitions données précédemment.

Dans un mode de réalisation, la composition selon l'invention est caractérisée en ce que lesdits radicaux hydrophobes sont choisis parmi les radicaux hydrophobes de formule X dans laquelle h = 0, a' = 2 et a = 1 représentée par la formule Xi suivante : 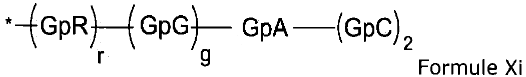 GpR, GpG, GpA, GpC, r et g ont les définitions données précédemment.

Dans un mode de réalisation, a =0,

Dans un mode de réalisation h= 1 et g=0,

Dans un mode de réalisation h= 0 et g=1,

Dans un mode de réalisation, r = 0, g=1 et h=0.

Dans un mode de réalisation, r=1 et GpR est choisi parmi les radicaux de formule VII' ou VII" et h = 0.

Dans un mode de réalisation, r=1, g=0 et GpR est un radical de formule VII' et h = 0.

Dans un mode de réalisation, r=1, g=0 et GpR est un radical de formule VII' et h = 1.

Dans un mode de réalisation, r=1, g=0, GpR est un radical de formule VII', GpA est choisi parmi les radicaux de formule VIIIa ou VIIIb et h = 0.

Dans un mode de réalisation, r=1, g=0, GpR est un radical de formule VII', GpA est choisi parmi les radicaux de formule VIIIa ou VIIIb et h = 1.

Dans un mode de réalisation, r=1, g=0, GpR est un radical de formule VII', GpA est un radical de formule VIIIa et h = 0.

Dans un mode de réalisation, r=1, g=0, GpR est un radical de formule VII', GpA est un radical de formule VIIIa et h = 1.

Dans un mode de réalisation, r=1, g=0, GpR est un radical de formule VII', GpA est un radical de formule VIIIb et h = 0.

Dans un mode de réalisation, r=1, g=0, GpR est un radical de formule VII', GpA est un radical de formule VIIIb et h = 1.

Dans un mode de réalisation, r=0, et GpA est choisi parmi les radicaux de formule VIIIa et VIIIb.

Dans un mode de réalisation, r=0, g=0 et GpA est choisi parmi les radicaux de formule VIIIa et VIIIb

Dans un mode de réalisation, r=0, GpA est choisi parmi les radicaux de formule VIIIa et VIIIb et h=0

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle linéaire divalent comprenant de 2 à 12 atomes de carbone.

Dans un mode de réalisation, la composition est caractérisée en ce que le radical hydrophobe de formules X, Xc', Xa, Xb, Xb', Xc, Xd', Xe Xf, Xg, Xh et Xi est un radical dans lequel R est un radical alkyle divalent comprenant de 2 à 6 atomes de carbone.

Dans un mode de réalisation, la composition est caractérisée en ce que le radical hydrophobe de formules X, Xc', Xa, Xb, Xb', Xc, Xd', Xe Xf, Xg, Xh et Xi est un radical dans lequel R est un radical alkyle linéaire divalent comprenant de 2 à 6 atomes de carbone.

Dans un mode de réalisation, la composition est caractérisée en ce que le radical hydrophobe de formules X, Xc', Xa, Xb, Xb', Xc, Xd', Xe Xf, Xg, Xh et Xi est un radical dans lequel R est un radical alkyle divalent comprenant de 2 à 4 atomes de carbone.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle linéaire divalent comprenant de 2 à 4 atomes de carbone.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle divalent comprenant 2 atomes de carbone.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle linéaire divalent comprenant de 1 à 11 atomes de carbone.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle divalent comprenant de 1 à 6 atomes de carbone.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle divalent, comprenant de 2 à 5 atomes de carbone et portant une ou plusieurs fonctions amide (-CONH₂).

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle linéaire divalent, comprenant de 2 à 5 atomes de carbone et portant une ou plusieurs fonctions amide (-CONH₂).

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical choisi dans le groupe constitué par les radicaux représentés par les formules ci-dessous :

| | |
|---|---|
| 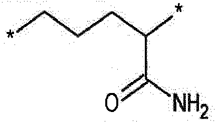 | Formule X1 |
| 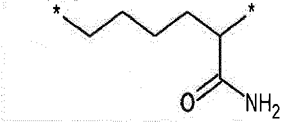 | Formule X2 |

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical de formule X1.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical de formule X2.

Dans un mode de réalisation, la composition est caractérisée en ce que le radical hydrophobe de formule X est un radical dans lequel R est lié au co-polyaminoacide via une fonction amide portée par le carbone en position delta ou epsilon (ou en position 4 ou 5) par rapport à la fonction amide (-CONH2).

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical linéaire éther ou polyéther non substitué comprenant de 4 à 14 atomes de carbone et de 1 à 5 atomes d'oxygène.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical éther.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical éther comprenant de 4 à 6 atomes de carbone.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical alkyle divalent comprenant 6 atomes de carbone.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical éther représenté par la formule

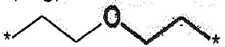

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical polyéther.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical linéaire polyéther comprenant de 6 à 10 atomes de carbone et de 2 à 3 atomes d'oxygène.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical polyéther choisi dans le groupe constitué par les radicaux représentés par les formules ci-dessous :

| | |
|---|---|
| 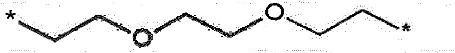 | Formule X3 |
| 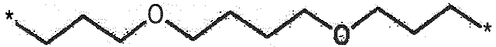 | Formule X4 |
| 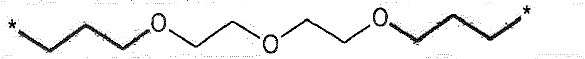 | Formule X5 |
| 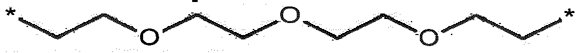 | Formule X6 |

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical de formule X3.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical de formule X4.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical polyéther choisi dans le groupe constitué par les radicaux représentés par les formules X5 et X6 ci-dessous :

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical polyéther de formule X5.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel R est un radical polyéther de formule X6.

| | |
|---|---|
| 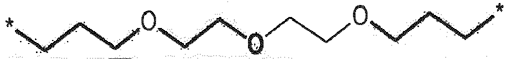 | Formule X5 |
| 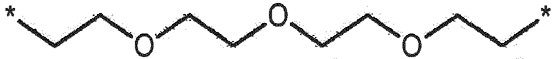 | Formule X6 |

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel le radical GpG et/ou GpH est de formule XI' dans lequel G est un radical alkyle comprenant 6 atomes de carbone représenté par la formule Z ci-dessous :

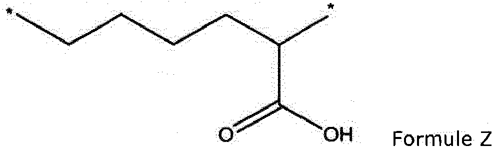

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel le radical GpG et/ou GpH est de formule XI dans lequel G est un radical alkyle comprenant 4 atomes de carbone représenté par la formule Z ci-dessous :

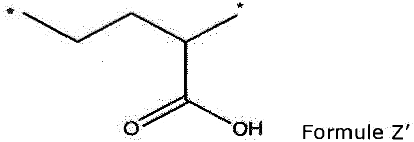

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel le radical GpG et/ou GpH est de formule XI dans lequel G est un radical alkyle comprenant 4 atomes de carbone représenté par -(CH₂)₂-CH(COOH)-.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel le radical GpG et/ou GpH est de formule XI dans lequel G est un radical alkyle comprenant 4 atomes de carbone représenté par -CH((CH₂)₂COOH)-.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel le radical GpG et/ou GpH est de formule XI dans lequel G est un radical alkyle comprenant 3 atomes de carbone représenté par -CH₂-CH-(COOH).

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel le radical GpG et/ou GpH est de formule XI dans lequel G est un radical alkyle comprenant 3 atomes de carbone représenté par -CH(CH₂)COOH)-.

Dans un mode de réalisation, la composition est caractérisée en ce que le radical hydrophobe de formules X, Xc', Xa, Xb, Xb', Xc, Xd', Xe, Xf, Xg, Xh et Xi est un radical dans lequel le radical GpA est de formule VIII et dans laquelle Aiest choisi dans le groupe constitué des radicaux représentés par les formules ci-dessous :

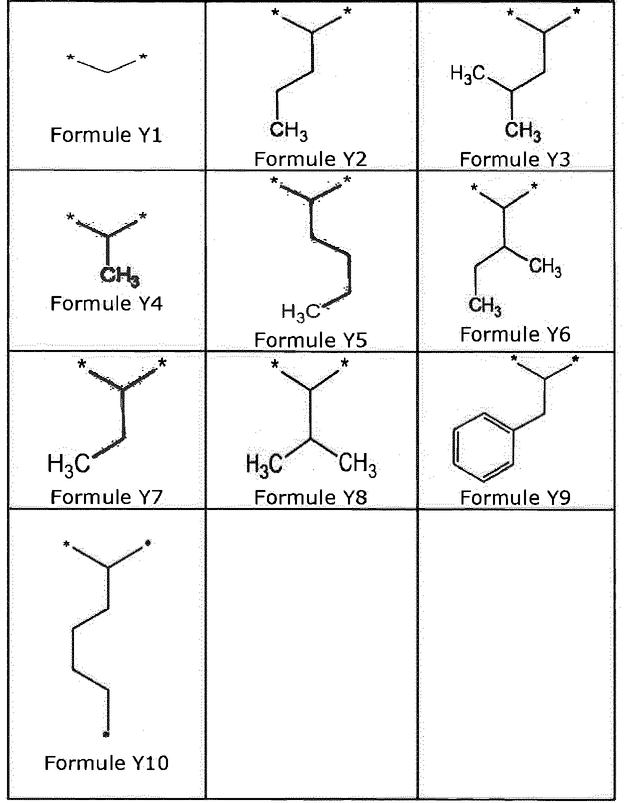

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel le radical GpC de formule IX est choisi dans le groupe constitué des radicaux de formules IXe, IXf ou IXg ci-après représentées :

| | |
|---|---|
| 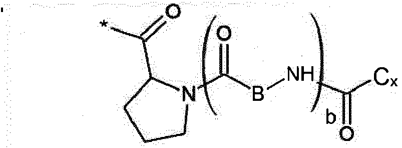 | Formule IXe |
| 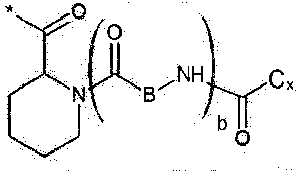 | Formule IXf |
| 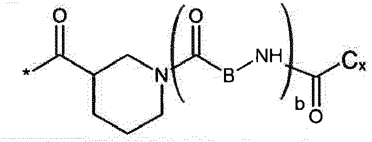 | Formule IXg |

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel le radical GpC de formule IX est choisi dans le groupe constitué des radicaux de formules IXe, IXf ou IXg dans lesquels b est égal à 0, répondant respectivement aux formules IXh, IXi, et IXj ci-après représentées :

| | |
|---|---|
| 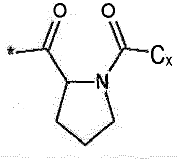 | Formule IXh |
| 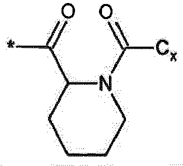 | Formule IXi |
| 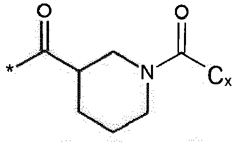 | Formule IXj |

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel le radical GpC répond à la formule IX ou IXe dans lesquelles b = 0, et répond à la formule IXh.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles linéaires.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles ramifiés.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles comprenant entre 19 et 14 atomes de carbone.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux représentés par les formules ci-dessous :

| | |
|---|---|
| 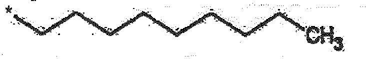 | x = 9 |
| 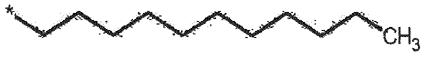 | x = 11 |
| 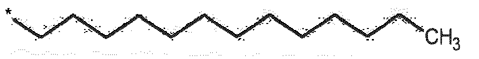 | x = 13 |

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles comprenant entre 15 et 16 atomes de carbone.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux représentés par les formules ci-dessous :

| | |
|---|---|
| 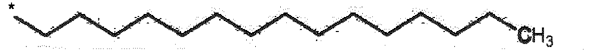 | x = 15 |

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux représentés par les formules ci-dessous :

| | |
|---|---|
| 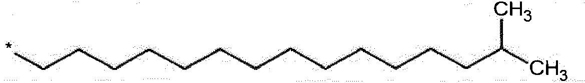 | x = 16 |

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles comprenant entre 17 et 25 atomes de carbone.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles comprenant entre 17 et 18 atomes de carbone.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles représentés par les formules ci-dessous :

| | |
|---|---|
| 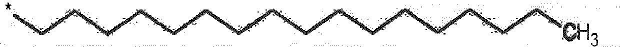 | x = 17 |

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles comprenant entre 18 et 25 atomes de carbone.

Dans un mode de réalisation, la composition est caractérisée en ce que le radical hydrophobe de formules X, Xc', Xd, Xa, Xb, Xb', Xc, Xd', Xe, Xf, Xg, Xh et Xi est un radical dans lequel Cx est choisi dans le groupe constitué par les radicaux alkyles représentés par les formules ci-dessous :

| | |
|---|---|
| 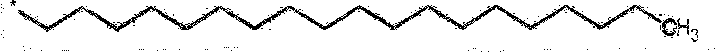 | x = 19 |
| 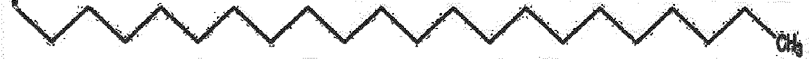 | x = 21 |

Dans un mode de réalisation, la composition est caractérisée en ce que le radical hydrophobe est un radical de formules X, Xc', Xd, Xa, Xb, Xb', Xc, Xd', Xe, Xf, Xg, Xh et Xi dans laquelle le radical GpC de formule IX est choisi dans le groupe constitué des radicaux dans lesquels Cx est choisi dans le groupe constitué par les radicaux alkyles comprenant 14 ou 15 atomes de carbone.

Dans un mode de réalisation, la composition est caractérisée en ce que le radical hydrophobe est un radical de formule X, Xc', Xd, Xa, Xb, Xb', Xc, Xd', Xe, Xf, Xg, Xh et dans laquelle le radical GpC de formule IX est choisi dans le groupe constitué des radicaux dans lesquels Cx est choisi dans le groupe constitué par les radicaux représentés par les formules ci-dessous :

| | |
|---|---|
| 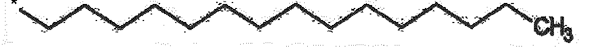 | x = 15 |

Dans un mode de réalisation, r=0 et le radical hydrophobe de formule X est lié au PLG via une liaison covalente entre un carbonyl du radical hydrophobe et un atome d'azote porté par le PLG formant ainsi une fonction amide issue de la réaction d'une fonction amine portée par le précurseur du PLG et une fonction acide portée par le précurseur Hy' du radical hydrophobe.

Dans un mode de réalisation, r = 1 ou 2 et le radical hydrophobe de formule X est lié à au PLG :
▪ via une liaison covalente entre un atome d'azote du radical hydrophobe et un carbonyle porté par le PLG, formant ainsi une fonction amide issue de la réaction d'une fonction amine du précurseur Hy' du radical hydrophobe et une fonction acide portée par le PLG ou,
▪ via une liaison covalente entre un carbonyl du radical hydrophobe et un atome d'azote porté par le PLG, formant ainsi une fonction amide issue de la réaction d'une fonction acide du précurseur Hy' du radical hydrophobe -Hy et une fonction amine du PLG.

Dans un mode de réalisation, si GpA est un radical de formule VIIIc et r = 1 ou 2, alors :
- les GpC sont liés directement ou indirectement à N_{α1} et N_{α2} et le PLG est lié directement ou indirectement via GpR à N_{βl} , ou
- les GpC sont liés directement ou indirectement à N_{α1} et N_{β1}, et le PLG est lié directement ou indirectement via GpR à N_{α2}, ou
- les GpC sont liés directement ou indirectement à N_{α2} et N_{β1}, et le PLG est lié directement ou indirectement via GpR à N_{α1}.

Dans un mode de réalisation, si GpA est un radical de formule VIIIc et r = 0, alors :
- les GpC sont liés directement ou indirectement à N_{α1} et N_{α2} et le PLG est lié directement ou indirectement à N_{β1} ; ou
- les GpC sont liés directement ou indirectement à N_{α1} et N_{β1}, et le PLG est lié directement ou indirectement à N_{α2} ; ou
- les GpC sont liés directement ou indirectement à N_{α2} et N_{β1}, et le PLG est lié directement ou indirectement à N_{α1}.

Dans un mode de réalisation, si GpA est un radical de formule VIIId et r = 1 ou 2, alors
- les GpC sont liés directement ou indirectement à N_{α1}, N_{α2} et N_{β1} et le PLG est lié directement ou indirectement via GpR à N_{β2}; ou
- les GpC sont liés directement ou indirectement à N_{α1}, N_{α2} et N_{β2} et le PLG est lié directement ou indirectement via GpR à N_{β1} ; ou
- les GpC sont liés directement ou indirectement à N_{α1}, N_{β1} et N_{β2} et le PLG est lié directement ou indirectement via GpR à N_{α2} ; ou
- les GpC sont liés directement ou indirectement à N_{α2}, N_{β1} et N_{β2} et le PLG est lié directement ou indirectement via GpR à N_{α1}.

Dans un mode de réalisation, si GpA est un radical de formule VIIId et r = 0, alors
- les GpC sont liés directement ou indirectement à N_{α1}, N_{α2} et N_{β1} et le PLG est lié directement ou indirectement à N_{β2}; ou
- les GpC sont liés directement ou indirectement à N_{α1}, N_{α2} et N_{β2} et le PLG est lié directement ou indirectement à N_{β1} ; ou
- les GpC sont liés directement ou indirectement à N_{α1}, N_{β1} et Npz et le PLG est lié directement ou indirectement à N_{α2} ; ou
- les GpC sont liés directement ou indirectement à N_{α2}, N_{β1} et N_{β2} et le PLG est lié directement ou indirectement à N_{α1}.

Dans les formules, les * indiquent les sites de rattachement des radicaux hydrophobes au PLG ou entre les différents groupes GpR, GpG, GpA, GpL et GpC pour former des fonctions amides.

Les radicaux Hy sont rattachés au PLG via des fonctions amides.

Dans les formules VII, VII' et VII", les * indiquent, de gauche à droite respectivement, les sites de rattachement de GpR :
- au PLG et
- à GpR si r = 2 ou à GpG si g = 1 ou à GpA si g = 0.

Dans les formules VIIIa, VIIIb, VIIIc et VIIId, les * indiquent, de gauche à droite respectivement, les sites de rattachement de GpA :
- à GpG si g = 1 ou à GpR si r = 1 ou 2 et g = 0 ou au PLG si g = r = 0 et
- à GpL si l = 1 ou à GpH si h=1 et l = 0 ou à GpC si l = h = 0

Dans la formule IX, le * indique le site de rattachement de GpC :
- à GpH si h = 1,
- à GpL si l = 1 et h = 0,
- à GpA si a = 1 et h = l = 0,
- à GpG si g = 1 et h = l = a = 0.

Les radicaux Hy, GpR, GpG, GpA, GpH, GpL et GpC sont chacun indépendamment identiques ou différents d'un résidu à l'autre.

Dans un mode de réalisation, la composition selon l'invention est caractérisée en ce que le ratio M entre le nombre de radicaux hydrophobes et le nombre d'unités glutamiques ou aspartiques est compris entre 0,02 et 0,2.

Dans un mode de réalisation, la composition est caractérisée en ce que le pH est compris entre 6,6 et 7,8.

Dans un mode de réalisation, la composition est caractérisée en ce que le pH est compris entre 7,0 et 7,8.

Dans un mode de réalisation, la composition est caractérisée en ce que le pH est compris entre 6,8 et 7,4.

Dans un mode de réalisation, la composition est caractérisée en ce que le co-polyaminoacide porteur de charges carboxylates et de radicaux hydrophobes est choisi parmi les co-polyaminoacides de formule XXX suivante : 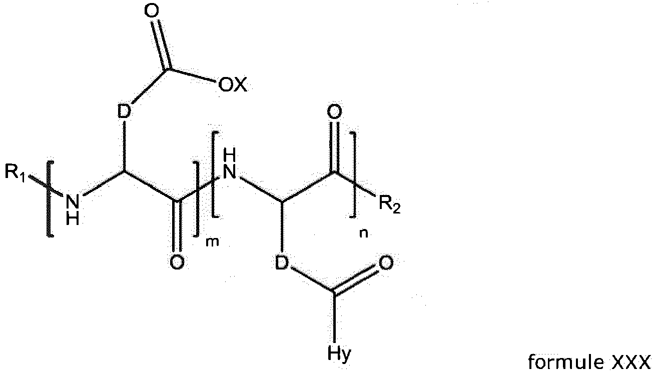 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules X, dans lesquelles r = 1 et GpR est un radical de Formule VII,
- R₁ est un radical hydrophobe choisi parmi les radicaux hydrophobes de formules X dans lesquelles r=0 ou r=1 et GpR est un radical de Formule VII', ou un radical choisi dans le groupe constitué par un H, un groupe acyle linéaire en C2 à C10, un groupe acyle ramifié en C3 à C10, un benzyle, une unité « acide aminé » terminale et un pyroglutamate,
- R₂ est un radical hydrophobe choisi parmi les radicaux hydrophobes de formules X dans lesquelles r = 1 et GpR est un radical de Formule VII, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
- X représente un H ou une entité cationique choisie dans le groupe comprenant les cations métalliques ;
- n + m représente le degré de polymérisation DP du co-polyaminoacide, c'est-à-dire le nombre moyen d'unités monomériques par chaîne de co-polyaminoacide et 5 ≤ n + m ≤ 250 ;

Le co-polyaminoacide porteur de charges carboxylates et d'au moins un radical hydrophobe de formule X peut également être appelé « co-polyaminoacide » dans la présente description.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₁ est un radical hydrophobe de formule X et R₂ est un radical -NR'R", R' et R" étant tels que définis ci-dessus.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₁ est un radical hydrophobe de formule X et R₂ est un radical radical -NR'R", R' et R" étant tels que définis ci-dessus, et Hy est un radical de formule X, dans laquelle r = 1.

Dans un mode de réalisation, la composition selon l'invention est caractérisée en ce que le co-polyaminoacide porteur de charges carboxylates et de radicaux hydrophobes est choisi parmi les co-polyaminoacides de formules XXX, dans laquelle R₁ est un radical hydrophobe de formule X et R₂ est un radical radical -NR'R", R' et R" étant tels que définis ci-dessus, et Hy est un radical de formule X, dans laquelle r = 1, et pour GpC, b = 0.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X et R₁ est un radical radical -NR'R", R' et R" étant tels que définis ci-dessus.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X et R₁ est un radical radical -NR'R", R' et R" étant tels que définis ci-dessus, et Hy est un radical de formule X, dans laquelle r = 1.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X et R₁ est un radical radical -NR'R", R' et R" étant tels que définis ci-dessus, et Hy est un radical de formule X, dans laquelle r = 1, et pour GpC, b = 0.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₁ et R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₁ et R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₁ et R₂ est un radical hydrophobe de formule X, et Hy est un radical de formule X, dans laquelle r = 1.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₁ et R₂ est un radical hydrophobe de formule X, et Hy est un radical de formule X, dans laquelle r = 1, et pour GpC, b = 0.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X dans lesquelles r = 1 et GpR est de Formule VII.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X dans laquelle r = 1 et GpR est de Formule VII.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X dans laquelle r = 1, GpR est de Formule VII et GpC est de formule IX dans laquelle b = 0, c = 0 et d = 1.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X dans laquelle r = 1, GpR est de Formule VII et GpC est de formule IX dans laquelle b = 0, c = 0, d = 1 et x = 13.

On appelle « co-polyaminoacide statistique » un co-polyaminoacide porteur de charges carboxylates et d'au moins un radical hydrophobe, un co-polyaminoacide de formule XXXa.

Dans un mode de réalisation, la composition est caractérisée en ce que le co-polyaminoacide porteur de charges carboxylates et de radicaux hydrophobes est choisi parmi les co-polyaminoacides de formules XXX, dans laquelle R₁ = R'₁ et R₂ = R'₂, de formule XXXa suivante : 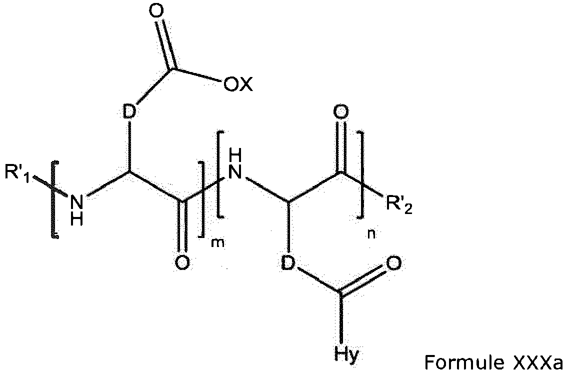 dans laquelle,
- m, n, X, D et Hy ont les définitions données précédemment,
- R'₁ est un radical choisi dans le groupe constitué par un H, un groupe acyle linéaire en C2 à C10, un groupe acyle ramifié en C3 à C10, un benzyle, une unité « acide aminé » terminale et un pyroglutamate,
- R'₂ est un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S.

Dans un mode de réalisation, la composition selon l'invention est caractérisée en ce que le co-polyaminoacide porteur de charges carboxylates et de radicaux hydrophobes est choisi parmi les co-polyaminoacides de formules XXXa, dans laquelle Hy est un radical de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa, dans laquelle Hy est un radical de formule X, dans laquelle r = 1.

Dans un mode de réalisation, la composition selon l'invention est caractérisée en ce que le co-polyaminoacide porteur de charges carboxylates et de radicaux hydrophobes est choisi parmi les co-polyaminoacides de formules XXXa, dans laquelle Hy est un radical de formule X, dans laquelle r = 1, et pour GpC, b = 0.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa, dans laquelle Hy est un radical de formule X et dans laquelle GpC est un radical de formule IX.

Dans un mode de réalisation, la composition selon l'invention est caractérisée en ce que le co-polyaminoacide porteur de charges carboxylates et de radicaux hydrophobes est choisi parmi les co-polyaminoacides de formule XXXa, dans laquelle Hy est un radical de formule X et dans laquelle GpC est un radical de formule IX et r = 1.

On appelle « co-polyaminoacide défini » un co-polyaminoacide porteur de charges carboxylates et d'au moins un radical hydrophobe, un co-polyaminoacide de formule XXXb.

Dans un mode de réalisation, la composition est caractérisée en ce que le co-polyaminoacide porteur de charges carboxylates et de radicaux hydrophobes est choisi parmi les co-polyaminoacides de formule XXX dans laquelle n = 0 de formule XXXb suivante : 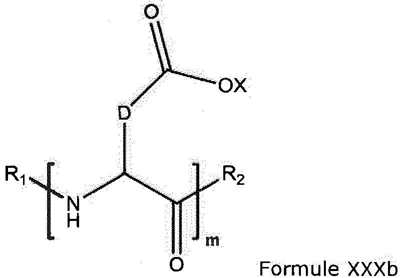 dans laquelle m, X, D, R₁ et R₂ ont les définitions données précédemment et au moins R₁ ou R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb dans laquelle au moins un des R₁ ou R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb dans laquelle R₁ est un radical hydrophobe de formule X.Dans un mode de réalisation, la composition selon l'invention est caractérisée en ce que le co-polyaminoacide porteur de charges carboxylates et de radicaux hydrophobes est choisi parmi les co-polyaminoacides de formule XXXb dans laquelle R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb dans laquelle R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb dans laquelle R₂ est un radical hydrophobe de formule X dans laquelle r = 0 et R₁ est un radical choisi dans le groupe constitué par un H, un groupe acyle linéaire en C2 à C10, un groupe acyle ramifié en C3 à C10, un benzyle, une unité « acide aminé » terminale et un pyroglutamate.

Dans un mode de réalisation, la composition selon l'invention est caractérisée en ce que le co-polyaminoacide porteur de charges carboxylates et de radicaux hydrophobes est choisi parmi les co-polyaminoacides de formule XXXb dans laquelle R₂ et Hy sont des radicaux hydrophobes de formule X dans laquelle r = 0, et pour GpC, b = 0 et R₁ est un radical choisi dans le groupe constitué par un H, un groupe acyle linéaire en C2 à C10, un groupe acyle ramifié en C3 à C10, un benzyle, une unité « acide aminé » terminale et un pyroglutamate.

Dans un mode de réalisation, la composition selon l'invention est caractérisée en ce que lorsque le co-polyaminoacide comprend des unités aspartate, alors le co-polyaminoacide peut en outre comprendre des unités monomériques de formule XXXI et/ou XXXI' :

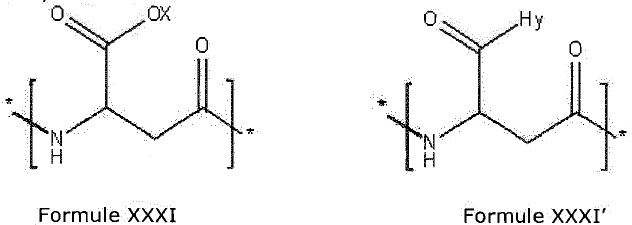

Dans un mode de réalisation, la composition selon l'invention est caractérisée en ce que le co-polyaminoacide porteur de charges carboxylates et de radicaux hydrophobes est choisi parmi les co-polyaminoacides de formule XXX suivante : 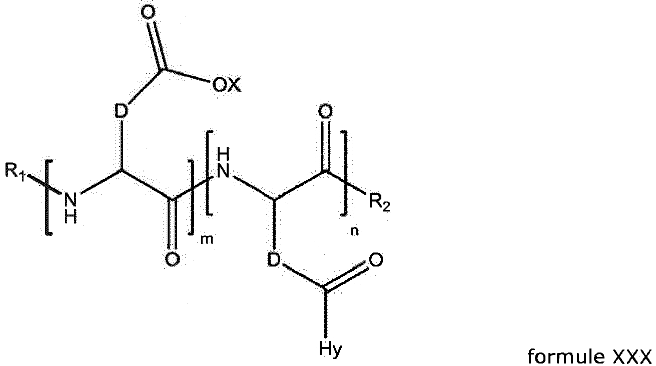 dans laquelle,
- D représente, indépendamment, soit un groupe -CH₂- (unité aspartique) soit un groupe -CH₂-CH₂- (unité glutamique),
- Hy est un radical hydrophobe choisi parmi les radicaux hydrophobes de formules X, dans lesquelles r = 1 et GpR est un radical de Formule VII,
- R₁ est un radical hydrophobe choisi parmi les radicaux hydrophobes de formules X dans lesquelles r = 0 ou r = 1 et GpR est un radical de Formule VII', ou un radical choisi dans le groupe constitué par un H, un groupe acyle linéaire en C2 à C10, un groupe acyle ramifié en C3 à C10, un benzyle, une unité « acide aminé » terminale et un pyroglutamate,
- R₂ est un radical hydrophobe choisi parmi les radicaux hydrophobes de formules X dans lesquelles r = 1 et GpR est un radical de Formule VII, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et 5 ;
- au moins un des R₁ ou R₂ est un radical hydrophobe tel que ci-dessus défini,
- X représente une entité cationique choisie dans le groupe comprenant les cations alcalins ;
- n ≥ 1 et n + m représente le degré de polymérisation DP du co-polyaminoacide, c'est-à-dire le nombre moyen d'unités monomériques par chaîne de co-polyaminoacide et 5 ≤ n + m ≤ 250 ;

Dans un mode de réalisation, le copolyaminoacide est choisi parmi les copolyaminoacides de formule XXXb dans laquelle le radical hydrophobe -Hy est choisi dans le groupe des radicaux hydrophobes de formule X dans laquelle a' = 1 et l'= 1 et GpC est un radical de formule IXe.

Dans un mode de réalisation, le copolyaminoacide est choisi parmi les copolyaminoacides de formule XXXb dans laquelle le radical hydrophobe -Hy est choisi dans le groupe des radicaux hydrophobes de formule X dans laquelle a' = 1 et l'= 1 et GpC est un radical de formule IX dans lequel e=0.

Dans un mode de réalisation, le copolyaminoacide est choisi parmi les copolyaminoacides de formule XXXb dans laquelle le radical hydrophobe -Hy est choisi dans le groupe des radicaux hydrophobes de formule X dans laquelle a' = 2 ou l'= 2 et GpC est un radical de formule IXe.

Dans un mode de réalisation, le copolyaminoacide est choisi parmi les copolyaminoacides de formule XXXb dans laquelle le radical hydrophobe -Hy est choisi dans le groupe des radicaux hydrophobes de formule X dans laquelle a' = 2 et l'= 2 et GpC est un radical de formule IX dans lequel e=0.

Dans un mode de réalisation, le copolyaminoacide est choisi parmi les copolyaminoacides de formule XXXa dans laquelle le radical hydrophobe -Hy est choisi dans le groupe des radicaux hydrophobes de formule X dans laquelle a' = 1 et l'= 1 et GpC est un radical de formule IXe.

Dans un mode de réalisation, le copolyaminoacide est choisi parmi les copolyaminoacides de formule XXXa dans laquelle le radical hydrophobe -Hy est choisi dans le groupe des radicaux hydrophobes de formule X dans laquelle a' = 2 ou l'= 2 et GpC est un radical de formule IXe.

Le co-polyaminoacide porteur de charges carboxylates et d'au moins un radical hydrophobe de formule I peut également être appelé « co-polyaminoacide » dans la présente description.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle n ≥ 1 et au moins un des R₁ ou R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle n ≥ 1 et R₁ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle n ≥ 1 et R₂ est un radical hydrophobe de formule X.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle n ≥ 1, R₁ est un radical hydrophobe de formule X dans laquelle r = 0.

[000142] Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle n ≥ 1, R₂ est un radical hydrophobe de formule X dans laquelle r = 1.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₁ est un radical hydrophobe de formule X dans laquelle r = 1, et pour GpC, b = 0 et R₂ est un radical -NR'R", R' et R" étant tels que définis ci-dessus.

Dans un mode de réalisation, la composition selon l'invention est caractérisée en ce que le co-polyaminoacide porteur de charges carboxylates et de radicaux hydrophobes est choisi parmi les co-polyaminoacides de formule XXX dans laquelle R₂ est un radical hydrophobe de formule X et R₁ est un radical choisi dans le groupe constitué par un H, un groupe acyle linéaire en C2 à C10, un groupe acyle ramifié en C3 à C10, un benzyle, une unité « acide aminé » terminale et un pyroglutamate.

Dans un mode de réalisation, la composition est caractérisée en ce que le co-polyaminoacide porteur de charges carboxylates et de radicaux hydrophobes est choisi parmi les co-polyaminoacides de formule XXX dans laquelle au moins un des R₁ ou R₂ est un radical hydrophobe, notamment avec n ≥ 1, ou XXXb dans lesquelles le groupe D est un groupe -CH₂- (unité aspartique).

Dans un mode de réalisation, la composition est caractérisée en ce que le co-polyaminoacide porteur de charges carboxylates et de radicaux hydrophobes est choisi parmi les co-polyaminoacides de formule XXX dans laquelle au moins un des au moins un des R₁ ou Rzest un radical hydrophobe, notamment avec n ≥ 1, ou XXXb dans lesquelles le groupe D est un groupe -CH₂-CH₂- (unité glutamique).

Dans un mode de réalisation, la composition est caractérisée en ce que le co-polyaminoacide porteur de charges carboxylates et de radicaux hydrophobes est choisi parmi les co-polyaminoacides de formule XXX, XXXa et XXb dans lesquelles le groupe D est un groupe -CH₂- (unité aspartique).

Dans un mode de réalisation, la composition est caractérisée en ce que le co-polyaminoacide porteur de charges carboxylates et de radicaux hydrophobes est choisi parmi les co-polyaminoacides de formule XXX, XXXa et XXb dans lesquelles le groupe D est un groupe -CH₂-CH₂- (unité glutamique).

Dans un mode de réalisation, la composition est caractérisée en ce que le ratio M entre le nombre de radicaux hydrophobes et le nombre d'unités glutamiques ou aspartiques est compris entre 0,007 et 0,3.

Dans un mode de réalisation, la composition est caractérisée en ce que le ratio M entre le nombre de radicaux hydrophobes et le nombre d'unités glutamiques ou aspartiques est compris entre 0,01 et 0,3.

Dans un mode de réalisation, la composition est caractérisée en ce que le ratio M entre le nombre de radicaux hydrophobes et le nombre d'unités glutamiques ou aspartiques est compris entre 0,03 et 0,3.

Dans un mode de réalisation, la composition est caractérisée en ce que le ratio M entre le nombre de radicaux hydrophobes et le nombre d'unités glutamiques ou aspartiques est compris entre 0,02 et 0,2.

Dans un mode de réalisation, la composition selon l'invention est caractérisée en ce que n + m est compris entre 10 et 250.

Dans un mode de réalisation, la composition selon l'invention est caractérisée en ce que n + m est compris entre 10 et 200.

Dans un mode de réalisation, la composition selon l'invention est caractérisée en ce que n + m est compris entre 10 et 100.

Dans un mode de réalisation, la composition selon l'invention est caractérisée en ce que n + m est compris entre 10 et 50.

Dans un mode de réalisation, la composition selon l'invention est caractérisée en ce que n + m est compris entre 15 et 150.

Dans un mode de réalisation, la composition selon l'invention est caractérisée en ce que n + m est compris entre 15 et 100.

Dans un mode de réalisation, la composition selon l'invention est caractérisée en ce que n + m est compris entre 15 et 80.

Dans un mode de réalisation, la composition selon l'invention est caractérisée en ce que n + m est compris entre 15 et 65.

Dans un mode de réalisation, la composition selon l'invention est caractérisée en ce que n + m est compris entre 20 et 60.

Dans un mode de réalisation, la composition selon l'invention est caractérisée en ce que n + m est compris entre 20 et 50.

Dans un mode de réalisation, la composition selon l'invention est caractérisée en ce que n + m est compris entre 20 et 40.

L'invention concerne également lesdits co-polyaminoacides porteurs de charges carboxylates et de radicaux hydrophobes de formule I et les précurseurs desdits radicaux hydrophobes.

Les co-polyaminoacides porteurs de charges carboxylates et de radicaux hydrophobes de formule X sont solubles dans l'eau distillée à un pH compris entre 6 et 8, à une température de 25°C et à une concentration inférieure à 60 mg/ml.

Dans un mode de réalisation, l'invention concerne également les précurseurs desdits radicaux hydrophobes de formule X.

L'invention concerne également le co-polyaminoacide porteur de charges carboxylates et de radicaux hydrophobes Hy, choisi parmi les radicaux de formule X telle que définie ci-dessous : 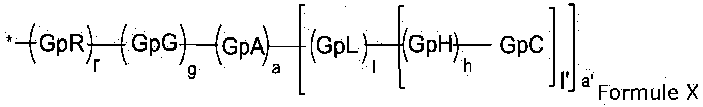 dans laquelle
- GpR est choisi parmi les radicaux de formules VII, VII' ou VII" : 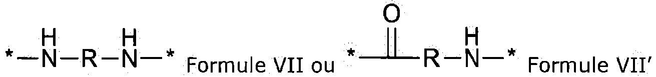 ou 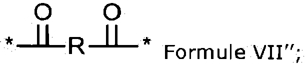
- GpG et GpH identiques ou différents sont choisis parmi les radicaux de formules XI ou XI': 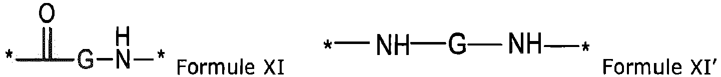
   - GpA est choisi parmi les radicaux de formule VIII 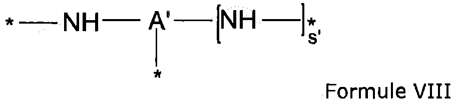 Dans laquelle A' est choisi parmi les radicaux de formule VIII',VIII" ou VIII'" 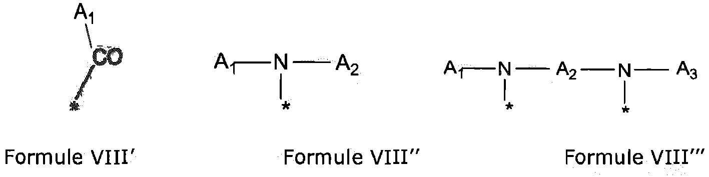
- -GpL est choisi parmi les radicaux de formule XII 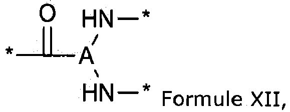
- GpC est un radical de formule IX : 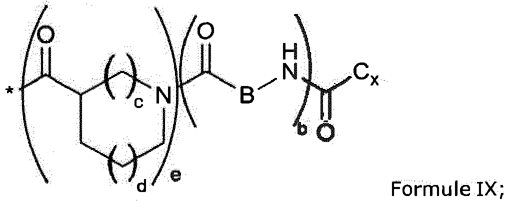
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
   - a' est un entier égal à 1, à 2 ou à 3 ;
   - b est un entier égal à 0 ou à 1 ;
   - c est un entier égal à 0 ou à 1, et si c est égal à 0 alors d est égal à 1 ou à 2 ;
   - d est un entier égal à 0, à 1 ou à 2 ;
   - e est un entier égal à 0 ou à 1 ;
   - g est un entier égal à 0, à 1, à 2, à 3, à 4, à 5 ou à 6 ;
   - h est un entier égal à 0, à 1, à 2, à 3, à 4, à 5 ou à 6 ;
   - l est un entier égal à 0 ou 1 et l' = 1 si l = 0 et l' = 2 si l = 1 ;
   - r est un entier égal à 0, 1 ou à 2 ;
   - s' est un entier égal à 0 ou 1 ;
   - et si e est différent de 0 alors au moins un des g, h ou l est différent de 0 ;
   - et si a = 0 alors l = 0 ;
   - A, A₁, A₂ et A₃ identiques ou différents sont des radicaux alkyles linéaires ou ramifiés comprenant de 1 à 8 atomes de carbone et éventuellement substitué par un radical issu d'un cycle saturé, insaturé ou aromatique ;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone ;
- Cₓ est un radical alkyl monovalent linéaire ou ramifié, éventuellement comprenant une partie cyclique, dans lequel x indique le nombre d'atomes de carbone et :
   ▪ Lorsque le radical hydrophobe -Hy porte 1 -GpC, alors 9 ≤ x ≤ 25,
   ▪ Lorsque le radical hydrophobe -Hy porte 2 -GpC, alors 9 ≤ x ≤ 15,
   ▪ Lorsque le radical hydrophobe -Hy porte 3 -GpC, alors 7 ≤ x ≤ 13,
   ▪ Lorsque le radical hydrophobe -Hy porte 4 -GpC, alors 7 ≤ x ≤ 11,
   ▪ Lorsque le radical hydrophobe -Hy porte au moins 5 -GpC alors, 6 ≤ x ≤ 11 ;
- G est un radical alkyle ramifié de 1 à 8 atomes de carbone ledit radical alkyle portant une ou plusieurs fonction(s) acide carboxylique libre ;
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions
- CONH₂ ou un radical éther ou polyéther non substitué comprenant de 4 à 14 atomes de carbone et de 1 à 5 atomes d'oxygène ;
- le ou les radicaux hydrophobes -Hy de formule X étant liés au PLG:
   ∘ via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur Hy' du radical hydrophobe -Hy, et
   ∘ via une liaison covalente entre un atome d'azote du radical hydrophobe
      - Hy et un carbonyle porté par le PLG formant ainsi une fonction amide issue de la réaction d'une fonction amine du précurseur Hy' du radical hydrophobe -Hy et une fonction acide portée par le PLG ;
      - le ratio M entre le nombre de radicaux hydrophobes et le nombre d'unités glutamiques ou aspartiques étant compris entre 0 < M ≤ 0,5 ;
      - lorsque plusieurs radicaux hydrophobes sont portés par un co-polyaminoacide alors ils sont identiques ou différents ;
      - le degré de polymérisation DP en unités glutamiques ou aspartiques pour les chaines PLG est compris entre 5 et 250 ;
      - les fonctions acides carboxyliques libres étant sous forme de sel de cation alcalin choisi dans le groupe constitué par Na⁺ et K⁺.

L'invention concerne également le précurseur Hy' du radical hydrophobe - Hy de formule X' 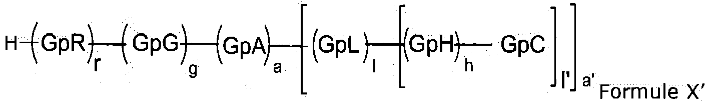 dans laquelle
- GpR est choisi parmi les radicaux de formules VII, VII' ou VII" : 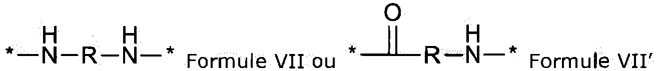 ou 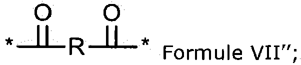
- GpG et GpH identiques ou différents sont choisis parmi les radicaux de formules XI ou XI': 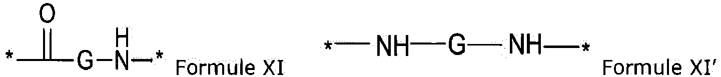
   - GpA est choisi parmi les radicaux de formule VIII 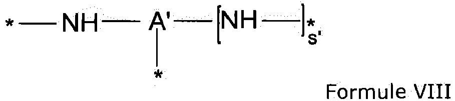 Dans laquelle A' est choisi parmi les radicaux de formule VIII',VIII" ou VIII'" 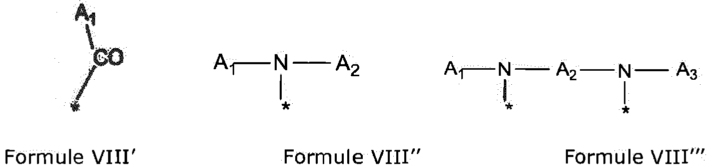
- -GpL est choisi parmi les radicaux de formule XII 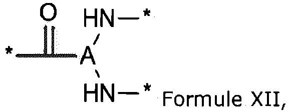
- GpC est un radical de formule IX : 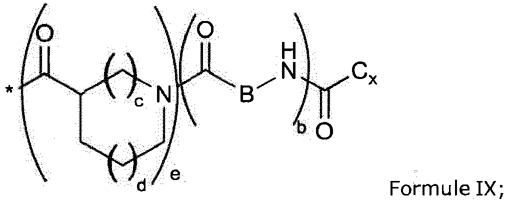
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
   - a' est un entier égal à 1, à 2 ou à 3 ;
   - b est un entier égal à 0 ou à 1 ;
   - c est un entier égal à 0 ou à 1, et si c est égal à 0 alors d est égal à 1 ou à 2 ;
   - d est un entier égal à 0, à 1 ou à 2 ;
   - e est un entier égal à 0 ou à 1 ;
   - g est un entier égal à 0, à 1, à 2, à 3, à 4, à 5 ou à 6 ;
   - h est un entier égal à 0, à 1, à 2, à 3, à 4, à 5 ou à 6 ;
   - l est un entier égal à 0 ou 1 et l' = 1 si l = 0 et l' = 2 si l = 1 ;
   - r est un entier égal à 0, 1 ou à 2 ;
   - s' est un entier égal à 0 ou 1 ;
   - et si e est différent de 0 alors au moins un des g, h ou l est différent de 0 ;
   - et si a = 0 alors l = 0 ;
   - A, A₁, A₂ et A₃ identiques ou différents sont des radicaux alkyles linéaires ou ramifiés comprenant de 1 à 8 atomes de carbone et éventuellement substitué par un radical issu d'un cycle saturé, insaturé ou aromatique ;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone ;
- Cₓ est un radical alkyl monovalent linéaire ou ramifié, éventuellement comprenant une partie cyclique, dans lequel x indique le nombre d'atomes de carbone et :
   ▪ Lorsque le radical hydrophobe -Hy porte 1 -GpC, alors 9 ≤ x ≤ 25,
   ▪ Lorsque le radical hydrophobe -Hy porte 2 -GpC, alors 9 ≤ x ≤ 15,
   ▪ Lorsque le radical hydrophobe -Hy porte 3 -GpC, alors 7 ≤ x ≤ 13,
   ▪ Lorsque le radical hydrophobe -Hy porte 4 -GpC, alors 7 ≤ x ≤ 11,
   ▪ Lorsque le radical hydrophobe -Hy porte au moins 5 -GpC alors, 6 ≤ x ≤ 11 ;
- G est un radical alkyle ramifié de 1 à 8 atomes de carbone ledit radical alkyle portant une ou plusieurs fonction(s) acide carboxylique libre ;
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions
- CONH₂ ou un radical éther ou polyéther non substitué comprenant de 4 à 14 atomes de carbone et de 1 à 5 atomes d'oxygène ;
- le ou les radicaux hydrophobes -Hy de formule X étant liés au PLG:
   ∘ via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur Hy' du radical hydrophobe -Hy, et
   ∘ via une liaison covalente entre un atome d'azote du radical hydrophobe
      - Hy et un carbonyle porté par le PLG formant ainsi une fonction amide issue de la réaction d'une fonction amine du précurseur Hy' du radical hydrophobe -Hy et une fonction acide portée par le PLG ;
      - le ratio M entre le nombre de radicaux hydrophobes et le nombre d'unités glutamiques ou aspartiques étant compris entre 0 < M ≤ 0,5 ;
      - lorsque plusieurs radicaux hydrophobes sont portés par un co-polyaminoacide alors ils sont identiques ou différents ;
      - le degré de polymérisation DP en unités glutamiques ou aspartiques pour les chaines PLG est compris entre 5 et 250 ;
      - les fonctions acides carboxyliques libres étant sous forme de sel de cation alcalin choisi dans le groupe constitué par Na⁺ et K⁺.

Dans un mode de réalisation, la composition selon l'invention est caractérisée en ce que le co-polyaminoacide est issu d'un polyaminoacide obtenu par polymérisation.

Dans un mode de réalisation, la composition selon l'invention est caractérisée en ce que le co-polyaminoacide est issu d'un polyaminoacide obtenu par polymérisation par ouverture de cycle d'un dérivé de N-carboxyanhydride d'acide glutamique ou d'un dérivé de N-carboxyanhydride d'acide aspartique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique comme décrit dans l'article de revue Adv. Polym. Sci. 2006, 202, 1-18 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique choisi dans le groupe constitué par le N-carboxyanhydride glutamate de méthyle (GluOMe-NCA), le N-carboxyanhydride glutamate de benzyle (GluOBzl-NCA) et le N-carboxyanhydride glutamate de t-butyle (GluOtBu-NCA).

Dans un mode de réalisation, le dérivé de N-carboxyanhydride d'acide glutamique est le N-carboxyanhydride L-glutamate de méthyle (L-GluOMe-NCA).

Dans un mode de réalisation, le dérivé de N-carboxyanhydride d'acide glutamique est le N-carboxyanhydride L-glutamate de benzyle (L-GluOBzl-NCA).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur un complexe organométallique d'un métal de transition comme décrit dans la publication Nature 1997, 390, 386-389 (Deming, T.J.).

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'ammoniaque ou une amine primaire comme décrit dans le brevet FR 2,801,226 (Touraud, F. ; et al.) et les références citées par ce brevet.

Dans un mode de réalisation, la composition selon l'invention est caractérisée en ce que le co-polyaminoacide est issu d'un polyaminoacide obtenu par polymérisation d'un dérivé de N-carboxyanhydride d'acide glutamique ou d'un dérivé de N-carboxyanhydride d'acide aspartique en utilisant comme initiateur l'hexaméthyldisilazane comme décrit dands la publication J. Am. Chem. Soc. 2007, 129, 14114-14115 (Lu H. ; et al.) ou une amine silylée comme décrit dans la publication J. Am. Chem. Soc. 2008, 130, 12562-12563 (Lu H. ; et al.).

Dans un mode de réalisation, la composition selon l'invention est caractérisée en ce que le procéde de synthèse du polyaminoacide obtenu par polymérisation d'un dérivé de N-carboxyanhydride d'acide glutamique ou d'un dérivé de N-carboxyanhydride d'acide aspartique dont est issu le co-polyaminoacide comprend une étape d'hydrolyse de fonctions ester.

Dans un mode de réalisation, cette étape d'hydrolyse de fonctions ester peut consister en une hydrolyse en milieu acide ou une hydrolyse en milieu basique ou être effectuée par hydrogénation.

Dans un mode de réalisation, cette étape d'hydrolyse de groupements ester est une hydrolyse en milieu acide.

Dans un mode de réalisation, cette étape d'hydrolyse de groupements ester est effectuée par hydrogénation.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation enzymatique et chimique d'un polyaminoacide de plus haut poids moléculaire.

Dans un mode de réalisation, la composition selon l'invention est caractérisée en ce que le co-polyaminoacide est issu d'un polyaminoacide obtenu par dépolymérisation d'un polyaminoacide de plus haut poids moléculaire choisi dans le groupe constitué par le polyglutamate de sodium et le polyaspartate de sodium.

Dans un mode de réalisation, la composition selon l'invention est caractérisée en ce que le co-polyaminoacide est issu d'un polyaminoacide obtenu par dépolymérisation d'un polyglutamate de sodium de plus haut poids moléculaire.

Dans un mode de réalisation, la composition selon l'invention est caractérisée en ce que le co-polyaminoacide est issu d'un polyaminoacide obtenu par dépolymérisation d'un polyaspartate de sodium de plus haut poids moléculaire.

Dans un mode de réalisation, la composition selon l'invention est caractérisée en ce que le co-polyaminoacide est obtenu par greffage d'un groupe hydrophobe sur un acide poly-L-glutamique ou acide poly-L-aspartique en utilisant les procédés de formation de liaison amide bien connus de l'homme de l'art.

Dans un mode de réalisation, la composition selon l'invention est caractérisée en ce que le co-polyaminoacide est obtenu par greffage d'un groupe hydrophobe sur un acide poly-L-glutamique ou acide poly-L-aspartique en utilisant les procédés de formation de liaison amide utilisés pour la synthèse peptidique.

Dans un mode de réalisation, la composition selon l'invention est caractérisée en ce que le co-polyaminoacide est obtenu par greffage d'un groupe hydrophobe sur un acide poly-L-glutamique ou acide poly-L-aspartique comme décrit dans le brevet FR 2,840,614 (Chan, Y.P. ; et al.).

Dans un mode de réalisation, la concentration en co-polyaminoacide porteur de charges carboxylates et de radicaux hydrophobes est au plus de 40 mg/mL.

Dans un mode de réalisation, la concentration en co-polyaminoacide porteur de charges carboxylates et de radicaux hydrophobes est au plus de 30 mg/mL.

Dans un mode de réalisation, la concentration en co-polyaminoacide porteur de charges carboxylates et de radicaux hydrophobes est au plus de 20 mg/mL.

Dans un mode de réalisation, la concentration en co-polyaminoacide porteur de charges carboxylates et de radicaux hydrophobes est au plus de 10 mg/mL.

Dans un mode de réalisation, la concentration en co-polyaminoacide porteur de charges carboxylates et de radicaux hydrophobes est au plus de 5 mg/mL.

Dans un mode de réalisation, la concentration en co-polyaminoacide porteur de charges carboxylates et de radicaux hydrophobes est au plus de 2,5 mg/mL.

Dans un mode de réalisation, la concentration en co-polyaminoacide porteur de charges carboxylates et de radicaux hydrophobes est au plus de 1 mg/mL.

Dans un mode de réalisation, la concentration en co-polyaminoacide porteur de charges carboxylates et de radicaux hydrophobes est au plus de 0,5 mg/mL.

Le glucagon humain est un polypeptide hautement conservé comprenant une chaîne simple de 29 résidus d'acides aminés présentant la séquence suivante H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-OH.

Il peut être obtenu de différentes manières, par synthèse peptidique ou par recombinaison.

Le glucagon humain est disponible via de nombreuses sources. Par exemple il peut s'agir du glucagon humain produit par Bachem via synthèse peptidique, notamment sous la référence 407473.

Le glucagon humain est utilisé à des posologies qui varient en fonction des applications.

En traitement d'urgence des hypoglycémies, la posologie recommandée est de 1 mg par voie intramusculaire ou intraveineuse (0,5 mg si la masse corporelle est inférieur à 25 kg). Cette administration est effectuée avec une solution de glucagon humain à la concentration de 1 mg/ml.

Dans les pompes, la dose journalière envisagée est d'environ 0,5 mg, les solutions peuvent ainsi comprendre de 0,25 mg/ml à 5 mg/ml de glucagon humain.

Selon un mode de réalisation les solutions peuvent comprendre de 0,5 mg/ml à 3 mg/ml de glucagon humain.

Dans le traitement de l'obésité la dose journalière envisagée est d'environ 0,5 mg, les solutions peuvent ainsi comprendre de 0,25 mg/ml à 5 mg/ml de glucagon humain.

Dans un mode de réalisation, la concentration en glucagon humain est comprise entre 0,25 et 5 mg/mL.

Dans un mode de réalisation, la concentration en glucagon humain est comprise entre 0,5 et 4 mg/mL.

Dans un mode de réalisation, la concentration en glucagon humain est comprise entre 0,75 et 3 mg/mL.

Dans un mode de réalisation, la concentration en glucagon humain est comprise entre 0,75 et 2,5 mg/mL.

Dans un mode de réalisation, la concentration en glucagon humain est comprise entre 0,75 et 2 mg/mL.

Dans un mode de réalisation, la concentration en glucagon humain est comprise entre 1 et 2 mg/mL.

Dans un mode de réalisation, le ratio molaire [radical hydrophobe]/[glucagon humain] est inférieur à 20.

Dans un mode de réalisation, le ratio molaire [radical hydrophobe]/[glucagon humain] est inférieur à 15.

Dans un mode de réalisation, le ratio molaire [radical hydrophobe]/[glucagon humain] est inférieur à 10.

Dans un mode de réalisation, le ratio molaire [radical hydrophobe]/[glucagon humain] est inférieur à 5.

Dans un mode de réalisation, le ratio molaire [radical hydrophobe]/[glucagon humain] est inférieur à 2,5.

Dans un mode de réalisation, le ratio molaire [radical hydrophobe]/[glucagon humain] est inférieur à 1,5.

Dans un mode de réalisation, le ratio molaire [co-polyaminoacide porteur de charges carboxylates et de radicaux hydrophobes Hy]/[glucagon humain] est inférieur à 20.

Dans un mode de réalisation, le ratio molaire [co-polyaminoacide porteur de charges carboxylates et de radicaux hydrophobes Hy]/[glucagon humain] est inférieur à 15.

Dans un mode de réalisation, le ratio molaire [co-polyaminoacide porteur de charges carboxylates et de radicaux hydrophobes Hy]/[glucagon humain] est inférieur à 10.

Dans un mode de réalisation, le ratio molaire [co-polyaminoacide porteur de charges carboxylates et de radicaux hydrophobes Hy]/[glucagon humain] est inférieur à 5.

Dans un mode de réalisation, le ratio molaire [co-polyaminoacide porteur de charges carboxylates et de radicaux hydrophobes Hy]/[glucagon humain] est inférieur à 2,5.

Dans un mode de réalisation, le ratio molaire [co-polyaminoacide porteur de charges carboxylates et de radicaux hydrophobes Hy]/[glucagon humain] est inférieur à 1,5.

Dans un mode de réalisation, le ratio massique co-polyaminoacide porteur de charges carboxylates et de radicaux hydrophobes sur glucagon est compris entre 1,5 et 25.

Dans un mode de réalisation, le ratio massique co-polyaminoacide porteur de charges carboxylates et de radicaux hydrophobes sur glucagon est compris entre 2 et 20.

Dans un mode de réalisation, le ratio massique co-polyaminoacide porteur de charges carboxylates et de radicaux hydrophobes sur glucagon est compris entre 2,5 et 15.

Dans un mode de réalisation, le ratio massique co-polyaminoacide porteur de charges carboxylates et de radicaux hydrophobes sur glucagon est compris entre 2 et 10.

Dans un mode de réalisation, le ratio massique co-polyaminoacide porteur de charges carboxylates et de radicaux hydrophobes sur glucagon est compris entre 2 et 7.

Dans un mode de réalisation, la composition comprend en outre un composé nicotinique ou un de ses dérivés.

Dans un mode de réalisation, la composition comprend de la nicotinamide.

Dans un mode de réalisation, la concentration de nicotinamide est comprise entre 10 et 160 mM.

Dans un mode de réalisation, la concentration de nicotinamide est comprise entre 20 et 150 mM.

Dans un mode de réalisation, la concentration de nicotinamide est comprise entre 40 et 120 mM.

Dans un mode de réalisation, la concentration de nicotinamide est comprise entre 60 et 100 mM.

Dans un mode de réalisation les espèces ioniques comprennent moins de 10 atomes de carbone.

Lesdites espèces ioniques sont choisies dans le groupe des anions, des cations et/ou des zwitterions. Par zwitterion, on entend une espèce portant au moins une charge positive et au moins une charge négative sur deux atomes non adjacents.

Lesdites espèces ioniques sont utilisées seules ou en mélange et de préférence en mélange.

Dans un mode de réalisation, les anions sont choisis parmi les anions organiques.

Dans un mode de réalisation les anions organiques comprennent moins de 10 atomes de carbone.

Dans un mode de réalisation, les anions organiques sont choisis dans le groupe constitué par l'acétate, le citrate et le succinate

Dans un mode de réalisation, les anions sont choisis parmi les anions d'origine minérale.

Dans un mode de réalisation, les anions d'origine minérale sont choisis dans le groupe constitué par les sulfates, les phosphates et les halogénures, notamment les chlorures.

Dans un mode de réalisation, les cations sont choisis parmi les cations organiques.

Dans un mode de réalisation les cations organiques comprennent moins de 10 atomes de carbone.

Dans un mode de réalisation, les cations organiques sont choisis dans le groupe constitué par les ammoniums, par exemple le 2-Amino-2-(hydroxyméthyl)propane-1,3-diol où l'amine est sous forme d'ammonium.

Dans un mode de réalisation, les cations sont choisis parmi les cations d'origine minérale.

Dans un mode de réalisation, les cations d'origine minérale sont choisis dans le groupe constitué par le zinc, en particulier Zn²⁺ et les métaux alcalins, en particulier Na⁺ et K⁺,

Dans un mode de réalisation, les zwitterions sont choisis parmi les zwitterions d'origine organique.

Dans un mode de réalisation, les zwitterions d'origine organique sont choisis parmi les aminoacides.

Dans un mode de réalisation les aminoacides sont choisis parmi les aminoacides aliphatiques dans le groupe constitué par la glycine, l'alanine, la valine, l'isoleucine et la leucine.

Dans un mode de réalisation les aminoacides sont choisis parmi les aminoacides cycliques dans le groupe constitué par la proline.

Dans un mode de réalisation les aminoacides sont choisis parmi les aminoacides hydroxylés ou soufrés dans le groupe constitué par la cystéine, la sérine, la thréonine, et la méthionine.

Dans un mode de réalisation les aminoacides sont choisis parmi les aminoacides aromatiques dans le groupe constitué par la phénylalanine, la tyrosine et le tryptophane.

Dans un mode de réalisation les aminoacides sont choisis parmi les aminoacides dont la fonction carboxyle de la chaine latérale est amidifiée dans le groupe constitué par l'asparagine et la glutamine.

Dans un mode de réalisation, les zwitterions d'origine organique sont choisis dans le groupe constitué par les aminoacides ayant une chaine latérale non chargée.

Dans un mode de réalisation, les zwitterions d'origine organique sont choisis dans le groupe constitué par les aminodiacides ou acides aminés acides.

Dans un mode de réalisation, les aminodiacides sont choisis dans le groupe constitué par l'acide glutamique et l'acide aspartique, éventuellement sous forme de sels.

Dans un mode de réalisation, les zwitterions d'origine organique sont choisis dans le groupe constitué par les acides aminés basiques ou dits « cationiques ».

Dans un mode de réalisation les aminoacides dits « cationiques » sont choisis parmi l'arginine, l'histidine et la lysine, en particulier arginine et lysine.

Tout particulièrement les zwitterions comprennent autant de charges négatives que de charges positives et donc une charge globale nulle au point isoélectrique et/ou à un pH compris entre 6 et 8.

Lesdites espèces ioniques sont introduites dans les compositions sous forme de sels. L'introduction de ceux-ci peut se faire sous forme solide avant mise en solution dans les compositions, ou sous forme de solution, en particulier de solution concentrée.

Par exemple, les cations d'origine minérale sont apportés sous forme de sels choisis parmi le chlorure de sodium, le chlorure de zinc, le phosphate de sodium, le sulfate de sodium, etc.

Par exemples, les anions d'origine organique sont apportés sous forme des sels choisis parmi le citrate de sodium ou de potassium, l'acétate de sodium.

Par exemple, les acides aminés sont ajoutés sous forme de sels choisis parmi le chlorhydrate d'arginine, le chlorhydrate d'histidine ou sous forme non salifiée comme par exemple l'histidine, l'arginine.

Dans un mode de réalisation, la concentration molaire totale en espèces ioniques dans la composition est supérieure ou égale à 10 mM.

Dans un mode de réalisation, la concentration molaire totale en espèces ioniques dans la composition est supérieure ou égale à 20 mM.

Dans un mode de réalisation, la concentration molaire totale en espèces ioniques dans la composition est supérieure ou égale à 30 mM.

Dans un mode de réalisation, la concentration molaire totale en espèces ioniques dans la composition est supérieure ou égale à 50 mM.

Dans un mode de réalisation, la concentration molaire totale en espèces ioniques dans la composition est supérieure ou égale à 75 mM.

Dans un mode de réalisation, la concentration molaire totale en espèces ioniques dans la composition est supérieure ou égale à 100 mM.

Dans un mode de réalisation, la concentration molaire totale en espèces ioniques dans la composition est supérieure ou égale à 200 mM.

Dans un mode de réalisation, la concentration molaire totale en espèces ioniques dans la composition est supérieure ou égale à 300 mM.

Dans un mode de réalisation, la concentration molaire totale en espèces ioniques dans la composition est supérieure ou égale à 500 mM.

Dans un mode de réalisation, la concentration molaire totale en espèces ioniques dans la composition est supérieure ou égale à 600 mM.

Dans un mode de réalisation, la concentration molaire totale en espèces ioniques dans la composition est supérieure ou égale à 700 mM.

Dans un mode de réalisation, la concentration molaire totale en espèces ioniques dans la composition est supérieure ou égale à 800 mM.

Dans un mode de réalisation, la concentration molaire totale en espèces ioniques dans la composition est supérieure ou égale à 900 mM.

Dans un mode de réalisation, la concentration molaire totale en espèces ioniques dans la composition est inférieure ou égale à 1000 mM.

Dans un mode de réalisation, la concentration molaire totale en espèces ioniques dans la composition est inférieure ou égale à 1500 mM.

Dans un mode de réalisation, la concentration molaire totale en espèces ioniques dans la composition est inférieure ou égale à 1200 mM.

Dans un mode de réalisation, la concentration molaire totale en espèces ioniques dans la composition est inférieure ou égale à 1000 mM.

Dans un mode de réalisation, la concentration molaire totale en espèces ioniques dans la composition est inférieure ou égale à 900 mM.

Dans un mode de réalisation, la concentration molaire totale en espèces ioniques dans la composition est inférieure ou égale à 800 mM.

Dans un mode de réalisation, la concentration molaire totale en espèces ioniques dans la composition est inférieure ou égale à 700 mM.

Dans un mode de réalisation, la concentration molaire totale en espèces ioniques dans la composition est inférieure ou égale à 600 mM.

Dans un mode de réalisation, la concentration molaire totale en espèces ioniques dans la composition est inférieure ou égale à 500 mM.

Dans un mode de réalisation, la concentration molaire totale en espèces ioniques dans la composition est inférieure ou égale à 400 mM.

Dans un mode de réalisation, la concentration molaire totale en espèces ioniques dans la composition est inférieure ou égale à 300 mM.

Dans un mode de réalisation, la concentration molaire totale en espèces ioniques dans la composition est inférieure ou égale à 200 mM.

Dans un mode de réalisation, la concentration molaire totale en espèces ioniques dans la composition est inférieure ou égale à 100 mM.

Dans un mode de réalisation, la concentration molaire totale en espèces ioniques dans la composition est comprise entre 10 et 1000 mM.

Dans un mode de réalisation, la concentration molaire totale en espèces ioniques dans la composition est comprise entre 20 et 1000 mM.

Dans un mode de réalisation, la concentration molaire totale en espèces ioniques dans la composition est comprise entre 30 et 1000 mM.

Dans un mode de réalisation, la concentration molaire totale en espèces ioniques dans la composition est comprise entre 50 et 1000 mM.

Dans un mode de réalisation, la concentration molaire totale en espèces ioniques dans la composition est comprise entre 75 et 1000 mM.

Dans un mode de réalisation, la concentration molaire totale en espèces ioniques dans la composition est comprise entre 100 et 1000 mM.

Dans un mode de réalisation, la concentration molaire totale en espèces ioniques dans la composition est comprise entre 200 et 1000 mM.

Dans un mode de réalisation, la concentration molaire totale en espèces ioniques dans la composition est comprise entre 300 et 1000 mM.

Dans un mode de réalisation, la concentration molaire totale en espèces ioniques dans la composition est comprise entre 400 et 1000 mM.

Dans un mode de réalisation, la concentration molaire totale en espèces ioniques dans la composition est comprise entre 500 et 1000 mM.

Dans un mode de réalisation, la concentration molaire totale en espèces ioniques dans la composition est comprise entre 600 et 1000 mM.

Dans un mode de réalisation, la concentration molaire totale en espèces ioniques dans la composition est comprise entre 10 et 900 mM.

Dans un mode de réalisation, la concentration molaire totale en espèces ioniques dans la composition est comprise entre 20 et 900 mM.

Dans un mode de réalisation, la concentration molaire totale en espèces ioniques dans la composition est comprise entre 30 et 900 mM.

Dans un mode de réalisation, la concentration molaire totale en espèces ioniques dans la composition est comprise entre 50 et 900 mM.

Dans un mode de réalisation, la concentration molaire totale en espèces ioniques dans la composition est comprise entre 75 et 900 mM.

Dans un mode de réalisation, la concentration molaire totale en espèces ioniques dans la composition est comprise entre 100 et 900 mM.

Dans un mode de réalisation, la concentration molaire totale en espèces ioniques dans la composition est comprise entre 200 et 900 mM.

Dans un mode de réalisation, la concentration molaire totale en espèces ioniques dans la composition est comprise entre 300 et 900 mM.

Dans un mode de réalisation, la concentration molaire totale en espèces ioniques dans la composition est comprise entre 400 et 900 mM.

Dans un mode de réalisation, la concentration molaire totale en espèces ioniques dans la composition est comprise entre 500 et 900 mM.

Dans un mode de réalisation, la concentration molaire totale en espèces ioniques dans la composition est comprise entre 600 et 900 mM.

Dans un mode de réalisation, la concentration molaire totale en espèces ioniques dans la composition est comprise entre 10 et 800 mM.

Dans un mode de réalisation, la concentration molaire totale en espèces ioniques dans la composition est comprise entre 20 et 800 mM.

Dans un mode de réalisation, la concentration molaire totale en espèces ioniques dans la composition est comprise entre 30 et 800 mM.

Dans un mode de réalisation, la concentration molaire totale en espèces ioniques dans la composition est comprise entre 50 et 800 mM.

Dans un mode de réalisation, la concentration molaire totale en espèces ioniques dans la composition est comprise entre 75 et 800 mM.

Dans un mode de réalisation, la concentration molaire totale en espèces ioniques dans la composition est comprise entre 100 et 800 mM.

Dans un mode de réalisation, la concentration molaire totale en espèces ioniques dans la composition est comprise entre 200 et 800 mM.

Dans un mode de réalisation, la concentration molaire totale en espèces ioniques dans la composition est comprise entre 300 et 800 mM.

Dans un mode de réalisation, la concentration molaire totale en espèces ioniques dans la composition est comprise entre 400 et 800 mM.

Dans un mode de réalisation, la concentration molaire totale en espèces ioniques dans la composition est comprise entre 500 et 800 mM.

Dans un mode de réalisation, la concentration molaire totale en espèces ioniques dans la composition est comprise entre 600 et 800 mM.

Dans un mode de réalisation, la concentration molaire totale en espèces ioniques dans la composition est comprise entre 10 et 700 mM.

Dans un mode de réalisation, la concentration molaire totale en espèces ioniques dans la composition est comprise entre 20 et 700 mM.

Dans un mode de réalisation, la concentration molaire totale en espèces ioniques dans la composition est comprise entre 30 et 700 mM.

Dans un mode de réalisation, la concentration molaire totale en espèces ioniques dans la composition est comprise entre 50 et 700 mM.

Dans un mode de réalisation, la concentration molaire totale en espèces ioniques dans la composition est comprise entre 75 et 700 mM.

Dans un mode de réalisation, la concentration molaire totale en espèces ioniques dans la composition est comprise entre 100 et 700 mM.

Dans un mode de réalisation, la concentration molaire totale en espèces ioniques dans la composition est comprise entre 200 et 700 mM.

Dans un mode de réalisation, la concentration molaire totale en espèces ioniques dans la composition est comprise entre 300 et 700 mM.

Dans un mode de réalisation, la concentration molaire totale en espèces ioniques dans la composition est comprise entre 400 et 700 mM.

Dans un mode de réalisation, la concentration molaire totale en espèces ioniques dans la composition est comprise entre 500 et 700 mM.

Dans un mode de réalisation, la concentration molaire totale en espèces ioniques dans la composition est comprise entre 600 et 700 mM.

Dans un mode de réalisation, la concentration molaire totale en espèces ioniques dans la composition est comprise entre 10 et 600 mM.

Dans un mode de réalisation, la concentration molaire totale en espèces ioniques dans la composition est comprise entre 20 et 600 mM.

Dans un mode de réalisation, la concentration molaire totale en espèces ioniques dans la composition est comprise entre 30 et 600 mM.

Dans un mode de réalisation, la concentration molaire totale en espèces ioniques dans la composition est comprise entre 50 et 600 mM.

Dans un mode de réalisation, la concentration molaire totale en espèces ioniques dans la composition est comprise entre 75 et 600 mM.

Dans un mode de réalisation, la concentration molaire totale en espèces ioniques dans la composition est comprise entre 100 et 600 mM.

Dans un mode de réalisation, la concentration molaire totale en espèces ioniques dans la composition est comprise entre 200 et 600 mM.

Dans un mode de réalisation, la concentration molaire totale en espèces ioniques dans la composition est comprise entre 300 et 600 mM.

Dans un mode de réalisation, la concentration molaire totale en espèces ioniques dans la composition est comprise entre 400 et 600 mM.

Dans un mode de réalisation, la concentration molaire totale en espèces ioniques dans la composition est comprise entre 500 et 600 mM.

Dans un mode de réalisation, la concentration molaire totale en espèces ioniques dans la composition est comprise entre 10 et 500 mM.

Dans un mode de réalisation, la concentration molaire totale en espèces ioniques dans la composition est comprise entre 20 et 500 mM.

Dans un mode de réalisation, la concentration molaire totale en espèces ioniques dans la composition est comprise entre 30 et 500 mM.

Dans un mode de réalisation, la concentration molaire totale en espèces ioniques dans la composition est comprise entre 50 et 500 mM.

Dans un mode de réalisation, la concentration molaire totale en espèces ioniques dans la composition est comprise entre 75 et 500 mM.

Dans un mode de réalisation, la concentration molaire totale en espèces ioniques dans la composition est comprise entre 100 et 500 mM.

Dans un mode de réalisation, la concentration molaire totale en espèces ioniques dans la composition est comprise entre 200 et 500 mM.

Dans un mode de réalisation, la concentration molaire totale en espèces ioniques dans la composition est comprise entre 300 et 500 mM.

Dans un mode de réalisation, la concentration molaire totale en espèces ioniques dans la composition est comprise entre 400 et 500 mM.

Dans un mode de réalisation, la concentration molaire totale en espèces ioniques dans la composition est comprise entre 10 et 400 mM.

Dans un mode de réalisation, la concentration molaire totale en espèces ioniques dans la composition est comprise entre 20 et 400 mM.

Dans un mode de réalisation, la concentration molaire totale en espèces ioniques dans la composition est comprise entre 30 et 400 mM.

Dans un mode de réalisation, la concentration molaire totale en espèces ioniques dans la composition est comprise entre 50 et 400 mM.

Dans un mode de réalisation, la concentration molaire totale en espèces ioniques dans la composition est comprise entre 75 et 400 mM.

Dans un mode de réalisation, la concentration molaire totale en espèces ioniques dans la composition est comprise entre 100 et 400 mM.

Dans un mode de réalisation, la concentration molaire totale en espèces ioniques dans la composition est comprise entre 200 et 400 mM.

Dans un mode de réalisation, la concentration molaire totale en espèces ioniques dans la composition est comprise entre 300 et 400 mM.

Dans un mode de réalisation, la concentration molaire totale en espèces ioniques dans la composition est comprise entre 10 et 300 mM.

Dans un mode de réalisation, la concentration molaire totale en espèces ioniques dans la composition est comprise entre 20 et 300 mM.

Dans un mode de réalisation, la concentration molaire totale en espèces ioniques dans la composition est comprise entre 30 et 300 mM.

Dans un mode de réalisation, la concentration molaire totale en espèces ioniques dans la composition est comprise entre 50 et 300 mM.

Dans un mode de réalisation, la concentration molaire totale en espèces ioniques dans la composition est comprise entre 75 et 300 mM.

Dans un mode de réalisation, la concentration molaire totale en espèces ioniques dans la composition est comprise entre 100 et 300 mM.

Dans un mode de réalisation, la concentration molaire totale en espèces ioniques dans la composition est comprise entre 200 et 300 mM.

Dans un mode de réalisation, la concentration molaire totale en espèces ioniques dans la composition est comprise entre 10 et 200 mM.

Dans un mode de réalisation, la concentration molaire totale en espèces ioniques dans la composition est comprise entre 20 et 200 mM.

Dans un mode de réalisation, la concentration molaire totale en espèces ioniques dans la composition est comprise entre 30 et 200 mM.

Dans un mode de réalisation, la concentration molaire totale en espèces ioniques dans la composition est comprise entre 50 et 200 mM.

Dans un mode de réalisation, la concentration molaire totale en espèces ioniques dans la composition est comprise entre 75 et 200 mM.

Dans un mode de réalisation, la concentration molaire totale en espèces ioniques dans la composition est comprise entre 100 et 200 mM.

Dans un mode de réalisation, la concentration molaire totale en espèces ioniques dans la composition est comprise entre 10 et 100 mM.

Dans un mode de réalisation, la concentration molaire totale en espèces ioniques dans la composition est comprise entre 20 et 100 mM.

Dans un mode de réalisation, la concentration molaire totale en espèces ioniques dans la composition est comprise entre 30 et 100 mM.

Dans un mode de réalisation, la concentration molaire totale en espèces ioniques dans la composition est comprise entre 50 et 100 mM.

Dans un mode de réalisation, la concentration molaire totale en espèces ioniques dans la composition est comprise entre 75 et 100 mM.

Dans un mode de réalisation, la concentration molaire totale en espèces ioniques dans la composition est comprise entre 10 et 75 mM.

Dans un mode de réalisation, la concentration molaire totale en espèces ioniques dans la composition est comprise entre 20 et 75 mM.

Dans un mode de réalisation, la concentration molaire totale en espèces ioniques dans la composition est comprise entre 30 et 75 mM.

Dans un mode de réalisation, la concentration molaire totale en espèces ioniques dans la composition est comprise entre 50 et 75 mM.

Dans un mode de réalisation, la concentration molaire totale en espèces ioniques dans la composition est comprise entre 10 et 50 mM.

Dans un mode de réalisation, la concentration molaire totale en espèces ioniques dans la composition est comprise entre 20 et 50 mM.

Dans un mode de réalisation, la concentration molaire totale en espèces ioniques dans la composition est comprise entre 30 et 50 mM.

Dans un mode de réalisation, lesdites espèces ioniques sont présentes en une concentration allant de 5 à 400 mM.

Dans un mode de réalisation, lesdites espèces ioniques sont présentes en une concentration allant de 5 à 300 mM.

Dans un mode de réalisation, lesdites espèces ioniques sont présentes en une concentration allant de 5 à 200 mM.

Dans un mode de réalisation, lesdites espèces ioniques sont présentes en une concentration allant de 5 à 100 mM.

Dans un mode de réalisation, lesdites espèces ioniques sont présentes en une concentration allant de 5 à 75 mM.

Dans un mode de réalisation, lesdites espèces ioniques sont présentes en une concentration allant de 5 à 50 mM.

Dans un mode de réalisation, lesdites espèces ioniques sont présentes en une concentration allant de 5 à 25 mM.

Dans un mode de réalisation, lesdites espèces ioniques sont présentes en une concentration allant de 5 à 20 mM.

Dans un mode de réalisation, lesdites espèces ioniques sont présentes en une concentration allant de 5 à 10 mM.

Dans un mode de réalisation, lesdites espèces ioniques sont présentes en une concentration allant de 10 à 400 mM.

Dans un mode de réalisation, lesdites espèces ioniques sont présentes en une concentration allant de 10 à 300 mM.

Dans un mode de réalisation, lesdites espèces ioniques sont présentes en une concentration allant de 10 à 200 mM.

Dans un mode de réalisation, lesdites espèces ioniques sont présentes en une concentration allant de 10 à 100 mM.

Dans un mode de réalisation, lesdites espèces ioniques sont présentes en une concentration allant de 10 à 75 mM.

Dans un mode de réalisation, lesdites espèces ioniques sont présentes en une concentration allant de 10 à 50 mM.

Dans un mode de réalisation, lesdites espèces ioniques sont présentes en une concentration allant de 10 à 25 mM.

Dans un mode de réalisation, lesdites espèces ioniques sont présentes en une concentration allant de 10 à 20 mM.

Dans un mode de réalisation, lesdites espèces ioniques sont présentes en une concentration allant de 20 à 300 mM.

Dans un mode de réalisation, lesdites espèces ioniques sont présentes en une concentration allant de 20 à 200 mM.

Dans un mode de réalisation, lesdites espèces ioniques sont présentes en une concentration allant de 20 à 100 mM.

Dans un mode de réalisation, lesdites espèces ioniques sont présentes en une concentration allant de 20 à 75 mM.

Dans un mode de réalisation, lesdites espèces ioniques sont présentes en une concentration allant de 20 à 50 mM.

Dans un mode de réalisation, lesdites espèces ioniques sont présentes en une concentration allant de 20 à 25 mM.

Dans un mode de réalisation, lesdites espèces ioniques sont présentes en une concentration allant de 50 à 300 mM.

Dans un mode de réalisation, lesdites espèces ioniques sont présentes en une concentration allant de 50 à 200 mM.

Dans un mode de réalisation, lesdites espèces ioniques sont présentes en une concentration allant de 50 à 100 mM.

Dans un mode de réalisation, lesdites espèces ioniques sont présentes en une concentration allant de 50 à 75 mM.

S'agissant des cations d'origine minérale et en particulier de Zn²⁺, sa concentration molaire au sein de la composition peut être comprise entre 0,25 et 20 mM, en particulier entre 0,25 et 10 mM ou entre 0,25 et 5 mM.

Dans un mode de réalisation l'espèce ionique présente est le NaCl.

Dans un mode de réalisation, la concentration en NaCl est comprise entre 5 et 250 mM.

Dans un mode de réalisation, la concentration en NaCl est comprise entre 10 et 150 mM Dans un mode de réalisation, la concentration en NaCl est comprise entre 20 et 100 mM

Dans un mode de réalisation l'espèce ionique présente est l'acide citrique et/ou ses sels

Dans un mode de réalisation, la concentration en acide citrique est comprise entre 5 et 40 mM.

Dans un mode de réalisation, la concentration en acide citrique est comprise entre 7 et 30 mM.

Dans un mode de réalisation, la concentration en acide citrique est comprise entre 8 et 20 mM.
Dans un mode de réalisation, la concentration en acide citrique est comprise entre105 et 15 mM

Dans un mode de réalisation, la composition comprend en outre un composé polyanionique.

Dans un mode de réalisation, le composé polyanionique est choisi dans le groupe constitué des polyacides carboxyliques et leurs sels de Na⁺, K⁺, Ca²⁺ ou Mg²⁺.

Dans un mode de réalisation, le polyacide carboxylique est choisi dans le groupe constitué par l'acide citrique, l'acide tartrique, et leurs sels de Na⁺, K⁺, Ca²⁺ ou Mg²⁺.

Dans un mode de réalisation, le composé polyanionique est choisi dans le groupe constitué des polyacides phosphoriques et leurs sels de Na⁺, K⁺, Ca²⁺ ou Mg²⁺.

Dans un mode de réalisation, le polyacide phosphorique est le triphosphate et ses sels de Na⁺, K⁺, Ca²⁺ ou Mg²⁺.

Dans un mode de réalisation, le composé polyanionique est l'acide citrique et ses sels de Na⁺, K⁺, Ca²⁺ ou Mg²⁺.

Dans un mode de réalisation, le composé polyanionique est l'acide tartrique et ses sels de Na⁺, K⁺, Ca²⁺ ou Mg²⁺.

Dans un mode de réalisation, le composé polyanionique est l'acide triphosphorique et ses sels de Na⁺, K⁺, Ca²⁺ ou Mg²⁺.

Dans un mode de réalisation, la concentration en composé polyanionique est comprise entre 1 et 20 mM.

Dans un mode de réalisation, la concentration en composé polyanionique est comprise entre 2 et 15 mM,

Dans un mode de réalisation, la concentration en composé polyanionique est comprise entre 3 et 12 mM.

Dans un mode de réalisation, la concentration en composé polyanionique est de 10 mM.

Dans un mode de réalisation, la concentration en composé polyanionique est de 5 mM.

Dans un mode de réalisation, la concentration en composé polyanionique est de 10 mM pour des concentrations en glucagon comprises entre 0,5 mg/ml et 3 mg/ml.

Dans un mode de réalisation, la concentration en composé polyanionique est de 10 mM pour des concentrations en glucagon comprises entre 0,5 mg/ml et 2 mg/ml.

Dans un mode de réalisation, la concentration en composé polyanionique est de 10 mM pour des concentrations en glucagon comprises entre 1 mg/ml et 2 mg/ml.

Dans un mode de réalisation, la concentration en composé polyanionique est de 5 mM pour des concentrations en glucagon comprises entre 0,5 mg/ml et 3 mg/ml.

Dans un mode de réalisation, la concentration en composé polyanionique est de 5 mM pour des concentrations en glucagon comprises entre 0,5 mg/ml et 2 mg/ml.

Dans un mode de réalisation, la concentration en composé polyanionique est de 5 mM pour des concentrations en glucagon comprises entre 1 mg/ml et 2 mg/ml.

Dans un mode de réalisation, la concentration en acide citrique et ses sels de Na⁺, K⁺, Ca²⁺ ou Mg²⁺ est comprise entre 1 et 20 mM.

Dans un mode de réalisation, la concentration en acide citrique et ses sels de Na⁺, K⁺, Ca²⁺ ou Mg²⁺ est comprise entre 2 et 15 mM.

Dans un mode de réalisation, la concentration en acide citrique et ses sels de Na⁺, K⁺, Ca²⁺ ou Mg²⁺ est comprise entre 3 et 12 mM.

Dans un mode de réalisation, la concentration en acide citrique et ses sels de Na⁺, K⁺, Ca²⁺ ou Mg²⁺ est de 10 mM.

Dans un mode de réalisation, la concentration en acide citrique et ses sels de Na⁺, K⁺, Ca²⁺ ou Mg²⁺ est de 5 mM.

Dans un mode de réalisation, la concentration en acide citrique et ses sels de Na⁺, K⁺, Ca²⁺ ou Mg²⁺ est de 10 mM pour des concentrations en glucagon comprises entre 0,5 mg/ml et 3 mg/ml.

Dans un mode de réalisation, la concentration en acide citrique et ses sels de Na⁺, K⁺, Ca²⁺ ou Mg²⁺ est de 10 mM pour des concentrations en glucagon comprises entre 0,5 mg/ml et 2 mg/ml.

Dans un mode de réalisation, la concentration en acide citrique et ses sels de Na⁺, K⁺, Ca²⁺ ou Mg²⁺ est de 10 mM pour des concentrations en glucagon comprises entre 1 mg/ml et 2 mg/ml.

Dans un mode de réalisation, la concentration en acide citrique et ses sels de Na⁺, K⁺, Ca²⁺ ou Mg²⁺ est de 5 mM pour des concentrations en glucagon comprises entre 0,5 mg/ml et 3 mg/ml.

Dans un mode de réalisation, la concentration en acide citrique et ses sels de Na⁺, K⁺, Ca²⁺ ou Mg²⁺ est de 5 mM pour des concentrations en glucagon comprises entre 0,5 mg/ml et 2 mg/ml.

Dans un mode de réalisation, la concentration en acide citrique et ses sels de Na⁺, K⁺, Ca²⁺ ou Mg²⁺ est de 5 mM pour des concentrations en glucagon comprises entre 1 mg/ml et 2 mg/ml.

Dans un mode de réalisation, la composition pharmaceutique comprend en outre au moins un promoteur d'absorption choisi parmi les promoteurs d'absorption, les promoteurs de diffusion ou les agents vasodilatateurs, seuls ou en mélange.

Les promoteurs d'absorption incluent, sans se limiter, les surfactants, par exemple, les sels biliaires, les sels d'acides gras ou les phospholipides ; les agents nicotiniques, comme les nicotinamides, les acides nicotiniques, la niacine, la niacinamide, la vitamine B3 et leurs sels ; les inhibiteurs de la trypsine pancréatique ; les sels de magnésium ; les acides gras polyinsaturés ; le phosphatidylcholine didécanoyl ; les aminopolycarboxylates ; la tolmétine ; le caprate de sodium ; l'acide salicylique ; l'acide oléique ; l'acide linoléique ; l'acide eicosapentaénoïque (EPA) ; l'acide docosahexaénoïque (DHA) ; l'acide benzylique ; les donneurs de monoxyde d'azote, par exemple, la 3-(2-Hydroxy-1-(1-méthyléthyl)-2-nitrosohydrazino)-1-propanamine, la N-éthyl-2-(1-éthyl-hydroxy-2-1-nitrosohydrazino)-éthanamine, ou la S-nitroso-N-acétylpenicillamine ; les acides biliaires, la glycine sous sa forme conjuguée à un acide biliaire ; l'ascorbate de sodium, l'ascorbate de potassium ; le salicylate de sodium, le salicylate de potassium, l'acide acétyl-salicylique, l'acide salicylosalicylique, l'acétylsalicylate d'aluminum, le salicylate de choline, le salicylamide, l'acétylsalicylate de lysine ; l'exalamide ; le diflunisal ; l'éthenzamide ; l'EDTA ; seul ou en mélange.

Dans un mode de réalisation, la composition pharmaceutique comprend en outre au moins un promoteur de diffusion. Des exemples de promoteur de diffusion incluent, sans se limiter, les glycosaminoglycanases, par exemple la hyaluronidase.

Dans un mode de réalisation, la composition pharmaceutique comprend en outre au moins un agent vasodilatateur.

Dans un mode de réalisation, la composition pharmaceutique comprend en outre au moins un agent vasodilatateur provoquant une hyperpolarisation en bloquant les canaux ioniques de calcium.

Dans un mode de réalisation, l'agent vasodilatateur provoquant une hyperpolarisation en bloquant les canaux ioniques de calcium est l'adénosine, un agent hyperpolarisant dérivé de l'endothélium, un inhibiteur de la phosphodiestérase de type 5 (PDE5), un agent d'ouverture des canaux potassiques ou toute combinaison de ces agents.

Dans un mode de réalisation, la composition pharmaceutique comprend en outre au moins un agent vasodilatateur à médiation par AMPc.

Dans un mode de réalisation, la composition pharmaceutique comprend en outre au moins un agent vasodilatateur à médiation par GMPc.

Dans un mode de réalisation, la composition pharmaceutique comprend en outre au moins un agent vasodilatateur choisi dans le groupe comprenant les agents vasodilatateurs qui agissent en provoquant une hyperpolarisation en bloquant les canaux ioniques de calcium, les agents vasodilatateurs à médiation par AMPc, et les agents vasodilatateurs à médiation par GMPc.

Le au moins un agent vasodilatateur est chosi dans le groupe comprenant, les donneurs de monoxyde d'azote, par exemple, la nitroglycérine, le dinitrate d'isosorbide, le mononitrate d'isosorbide, le nitrate d'amyl, l'érythrityle, le tétranitrate, et le nitroprussiate) ; la prostacycline et ses analogues, par exemple l'époprosténol sodique, l'iloprost, l'époprostenol, le tréprostinil ou le selexipag ; l'histamine, la 2-méthylhistamine, la 4-méthylhistamine; la 2-(2-pyridyl)éthylamine, la 2-(2-thiazolyl)éthylamine ; la papavérine, le chlorhydrate de papavérine ; le minoxidil ; la dipyridamole ; l'hydralazine ; l'adénosine, l'adénosine triphosphate; l'uridine trisphosphate ; le GPLC ; la L-carnitine ; l'arginine ; la prostaglandine D2 ; les sels de potassium ; et dans certains cas, les antagonistes des récepteurs α1 et α2 par exemple, le prazosine, la phénoxybenzamine, la phentolamine, la dibénamine, le chlorhydrate de moxisylyte et la tolazoline), le bétazole, le dimaprit ; les agonistes des récepteurs β2, par exemple, l'isoprotérénol, la dobutamine, l'albutérol, la terbutaline, l'aminophylline, la théophylline, la caféine ; l'alprostadil, l'ambrisentan ; la cabergoline ; la diazoxide ; le mesilate de dihydralazine ; le chlorhydrate de diltiazem ; l'énoximone ; le chlorhydrate de flunarizine ; l'extrait de Ginkgo biloba ; le lévosimendan ; la molsidomine ; l'oxalate acide de naftidrofuryl ; le nicorandil ; la pentoxifylline ; le chlorure de phenoxybenzamine ; le piribédil base ; le mesilate de piribédil ; le regadenoson monohydrate ; le riociguat ; le sildenafil citrate, le tadalafil, le chlorhydrate trihydraté de vardenafil ; le chlorhydrate de trimetazidine ; la trinitrine ; le chlorhydrate de vérapamil ; les antagonistes des récepteur à l'endothéline, par exemple l'avanafil et le bosentran monohydrate ; et les inhibiteurs des canaux calciques,par exemple, l'amlodipine, l'aranidipine, l'azelnidipine, la barnidipine, la benidipine, la cilnidipine, la clévidipine, l'isradipine, l'efonidipine, la felodipine, la lacidipine, la lercanidipine, la manidipine, la nicardipine, la nifedipine, la nilvadipine, la nimodipine, la nisoldipine, la nitrendipine, la prandipine ; seul ou en mélange.

Selon un mode de réalisation l'agent vasodilatateur est le tréprostinil.

Dans un mode de réalisation, la composition comprend en combinaison un composé polyanionique et un promoteur d'absorption.

Dans un mode de réalisation, la composition comprend en combinaison l'acide citrique et/ou ses sels de Na⁺, K⁺, Ca²⁺ ou Mg²⁺ et un promoteur d'absorption.

Dans un mode de réalisation, le composé polyanionique est l'acide citrique et ses sels de Na⁺, K⁺, Ca²⁺ ou Mg²⁺.

Dans un mode de réalisation, la composition comprend en combinaison un composé polyanionique, un promoteur d'absorption et optionnellement du NaCl.

Dans un mode de réalisation, la composition comprend en combinaison de l'acide citrique et/ou ses sels de Na⁺, K⁺, Ca²⁺ ou Mg²⁺, de la nicotinamide ou du tréprostinil et optionnellement du NaCl.

Dans un mode de réalisation, la composition comprend en combinaison de l'acide citrique et/ou ses sels de Na⁺, K⁺, Ca²⁺ ou Mg²⁺, de la nicotinamide ou du tréprostinil et du NaCl, et est destinée à être administrée pas voie intra-musculaire.

Dans un mode de réalisation, la composition comprend en combinaison de l'acide citrique et/ou ses sels de Na⁺, K⁺, Ca²⁺ ou Mg²⁺, de la nicotinamide, optionnellement du NaCl, et est destinée à être administrée pas voie intra-musculaire.

Dans un mode de réalisation, la composition comprend en combinaison de l'acide citrique et/ou ses sels de Na⁺, K⁺, Ca²⁺ ou Mg²⁺, du treprostinil et optionnellement du NaCl, et est destinée à être administrée pas voie intra-musculaire.

Dans un mode de réalisation, la composition comprend en combinaison de l'acide citrique et/ou ses sels de Na⁺, K⁺, Ca²⁺ ou Mg²⁺, de la nicotinamide ou du treprostinil et optionnellement du NaCl, et est destinée à être administrée pas voie sous-cutanée.

Dans un mode de réalisation, la composition comprend en combinaison de l'acide citrique et/ou ses sels de Na⁺, K⁺, Ca²⁺ ou Mg²⁺, de la nicotinamide et optionnellement du NaCl, et est destinée à être administrée pas voie sous-cutanée.

Dans un mode de réalisation, la composition comprend en combinaison de l'acide citrique et/ou ses sels de Na⁺, K⁺, Ca²⁺ ou Mg²⁺, du treprostinil et optionnellement du NaCl et est destinée à être administrée pas voie sous-cutanée.

Dans un mode de réalisation, les compositions selon l'invention comprennent en outre une hormone gastrointestinale.

On entend par « hormones gastrointestinales », les hormones choisies dans le groupe constitué par les GLP-1 RA pour agonistes du récepteur Glucagon humain-Like Peptide-1 (Glucagon like peptide-1 receptor agonist)Glucagon like) et le GIP (Glucose-dependent insulinotropic peptide), l'oxyntomoduline (un dérivé du proglucagon humain), le peptide YY, l'amyline, la cholecystokinine, le polypeptide pancréatique (PP), la ghreline et l'entérostatine, leurs analogues ou dérivés et/ou leurs sels pharmaceutiquement acceptables.

Dans un mode de réalisation, les hormones gastro-intestinales sont des analogues ou dérivés de GLP-1 RA (Glucagon like peptide-1 receptor agonist) choisis dans le groupe constitué par l'exenatide ou Byetta^{®} (ASTRA-ZENECA) , le liraglutide ou Victoza^{®} (NOVO NORDISK), le lixisenatide ou Lyxumia^{®} (SANOFI), l'albiglutide ou Tanzeum^{®} (GSK) ou le dulaglutide ou Trulicity^{®} (ELI LILLY & CO), leurs analogues ou dérivés et leurs sels pharmaceutiquement acceptables.

Dans un mode de réalisation, l'hormone gastrointestinale est le pramlintide ou Symlin^{®}(ASTRA-ZENECA).

Dans un mode de réalisation, l'hormone gastrointestinale est l'exenatide ou Byetta^{®} ses analogues ou dérivés et leurs sels pharmaceutiquement acceptables.

Dans un mode de réalisation, l'hormone gastrointestinale est le liraglutide ou Victoza^{®} ses analogues ou dérivés et leurs sels pharmaceutiquement acceptables.

Dans un mode de réalisation, l'hormone gastrointestinale est le lixisenatide ou Lyxumia^{®} ses analogues ou dérivés et leurs sels pharmaceutiquement acceptables.

Dans un mode de réalisation, l'hormone gastrointestinale est l'albiglutide ou Tanzeum^{®} ses analogues ou dérivés et leurs sels pharmaceutiquement acceptables.

Dans un mode de réalisation, l'hormone gastrointestinale est le dulaglutide ou Trulicity^{®} ses analogues ou dérivés et leurs sels pharmaceutiquement acceptables.

Dans un mode de réalisation, l'hormone gastrointestinale est le pramlintide ou Symlin^{®}, ses analogues ou dérivés et leurs sels pharmaceutiquement acceptables.

On entend par « analogue », lorsqu'il est utilisé par référence à un peptide ou une protéine, un peptide ou une protéine, dans lequel un ou plusieurs résidus d'acides aminés constitutifs ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50%.

On entend par « dérivé », lorsqu'il est utilisé par référence à un peptide ou une protéine, un peptide ou une protéine ou un analogue chimiquement modifié par un substituant qui n'est pas présent dans le peptide ou la protéine ou l'analogue de référence, c'est-à-dire un peptide ou une protéine qui a été modifié par création de liaisons covalentes, pour introduire des substituants.

Dans un mode de réalisation, le substituant est choisi dans le groupe constitué des chaines grasses.

Dans un mode de réalisation, la concentration en hormone gastrointestinale est comprise dans un intervalle de 0,01 à 10 mg/mL.

Dans un mode de réalisation, la concentration en exenatide, ses analogues ou dérivés et leurs sels pharmaceutiquement acceptables est comprise dans un intervalle de 0,04 à 0,5 mg/mL.

Dans un mode de réalisation, la concentration en liraglutide, ses analogues ou dérivés et leurs sels pharmaceutiquement acceptables est comprise dans un intervalle de 1 à 10 mg/mL.

Dans un mode de réalisation, la concentration en lixisenatide, ses analogues ou dérivés et leurs sels pharmaceutiquement acceptables est comprise dans un intervalle de 0,01 à 1 mg/mL.

Dans un mode de réalisation, la concentration en pramlintide, ses analogues ou dérivés et leurs sels pharmaceutiquement acceptables est comprise entre 0,1 à 5 mg/mL.

Dans un mode de réalisation, les compositions selon l'invention sont réalisées par mélange de solutions de glucagon humain obtenues par reconstitution de lyophilisat et de solutions de GLP-1 RA (Glucagon like peptide-1 receptor agonist) GLP-1 RA , d'analogue ou de dérivé de GLP-1 RA lesdites solutions de GLP-1 RA étant commerciales ou reconstituées à partir de lyophilisat.

Dans un mode de réalisation, les compositions selon l'invention comprennent en outre des tampons.

Dans un mode de réalisation, les compositions selon l'invention comprennent des tampons à des concentrations comprises entre 0 et 100 mM.

Dans un mode de réalisation, les compositions selon l'invention comprennent des tampons à des concentrations comprises entre 15 et 50 mM.

Dans un mode de réalisation, les compositions selon l'invention comprennent un tampon choisi dans le groupe constitué par un tampon phosphate, le Tris (trishydroxyméthylaminométhane) ou le citrate de sodium.

Dans un mode de réalisation, le tampon est le phosphate de sodium.

Dans un mode de réalisation, le tampon est le Tris (trishydroxyméthylaminométhane).

Dans un mode de réalisation, le tampon est le citrate de sodium.

Dans un mode de réalisation, la composition comprend en outre un sel de zinc, en particulier du chlorure de zinc.

Dans un mode de réalisation, la concentration en sel de zinc est comprise entre 50 et 5000 µM.

Dans un mode de réalisation, la concentration en sel de zinc est comprise entre 100 et 2000 µM.

Dans un mode de réalisation, la concentration en sel de zinc est comprise entre 200 et 1500 µM.

Dans un mode de réalisation, la concentration en sel de zinc est comprise entre 200 et 1000 µM.

Dans un mode de réalisation, la concentration en zinc est telle que le ratio molaire [zinc]/[glucagon] est compris entre 0,1 et 2,5.

Dans un mode de réalisation, la concentration en zinc est telle que le ratio molaire [zinc]/[glucagon] est compris entre 0,2 et 2.

Dans un mode de réalisation, la concentration en zinc est telle que le ratio molaire [zinc]/[glucagon] est compris entre 0,5 et 1,5.

Dans un mode de réalisation, la concentration en zinc est telle que le ratio molaire [zinc]/[glucagon] est 1.

Dans un mode de réalisation, les compositions selon l'invention comprennent en outre des conservateurs.

Dans un mode de réalisation, les conservateurs sont choisis dans le groupe constitué par le m-crésol et le phénol seuls ou en mélange.

Dans un mode de réalisation, les compositions selon l'invention comprennent en outre des antioxydants.

Dans un mode de réalisation, les antioxydants sont choisis parmi la méthionine.

Dans un mode de réalisation, la concentration des conservateurs est comprise entre 10 et 50 mM.

Dans un mode de réalisation, la concentration des conservateurs est comprise entre 10 et 40 mM.

Dans un mode de réalisation, les compositions selon l'invention comprennent en outre un tensioactif.

Dans un mode de réalisation, le tensioactif est choisi dans le groupe constitué par le propylène glycol ou le polysorbate.

Les compositions selon l'invention peuvent en outre comprendre des additifs tels que des agents de tonicité.

Dans un mode de réalisation, les agents de tonicité sont choisis dans le groupe constitué par le chlorure de sodium, le mannitol, du sucrose, du sorbitol et le glycérol.

Les compositions selon l'invention peuvent comprendre en outre tous les excipients conformes aux pharmacopées et compatibles avec le glucagon humain et les hormones gastro-intestinales, notamment les GLP-1 RA, utilisés aux concentrations d'usage.

L'invention concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

L'invention concerne également des formulations unidoses à pH compris entre 6,6 et 7,8 comprenant du glucagon humain.

L'invention concerne également des formulations unidoses à pH compris entre 6,6 et 7,8 comprenant du glucagon humain et une hormone gastrointestinale, telle que définie précédemment.

Dans un mode de réalisation les formulations unidoses comprennent en outre un co-polyaminoacide substitué tel que défini précédemment.

Dans un mode de réalisation, les formulations sont sous forme d'une solution injectable. Dans un mode de réalisation, le GLP-1 RA, analogue ou dérivé de GLP-1 RA est choisi dans le groupe comprenant exenatide (Byetta^{®}), liraglutide (Victoza^{®}), lixisenatide (Lyxumia^{®}), albiglutide (Tanzeum^{®}), dulaglutide (Trulicity^{®}) ou l'un de leurs dérivés.

Dans un mode de réalisation, l'hormone gastrointestinale est l'exenatide.

Dans un mode de réalisation, l'hormone gastrointestinale est le liraglutide.

Dans un mode de réalisation, l'hormone gastrointestinale est le lixisenatide.

Dans un mode de réalisation, l'hormone gastrointestinale est l'albiglutide.

Dans un mode de réalisation, l'hormone gastrointestinale est le dulaglutide.

Par ailleurs et de façon toute aussi importante, la demanderesse a pu vérifier que le glucagon humain en présence d'un co-polyaminoacide porteur de charges carboxylates et d'au moins un radical hydrophobe selon l'invention conserve son action que ce soit seul ou en combinaison avec une hormone gastrointestinale.

La préparation d'une composition selon l'invention présente l'avantage de pouvoir être réalisée par simple mélange d'une solution de glucagon humain, d'une solution de GLP-1 RA, d'un analogue ou un dérivé de GLP-1 RA, et d'un co-polyaminoacide porteurs de charges carboxylates et d'au moins un radical hydrophobe selon l'invention, en solution aqueuse ou sous forme lyophilisée. Si nécessaire, le pH de la préparation est ajusté à pH 7.

Dans un mode de réalisation le mélange de glucagon humain et de co-polyaminoacide substitué est concentré par ultrafiltration avant le mélange avec de GLP-1 RA, d'un analogue ou un dérivé de GLP-1 RA en solution aqueuse ou sous forme lyophilisée.

Si nécessaire, la composition du mélange est ajustée en excipients tels que glycérol, m-crésol, et polysorbate (Tween^{®}) par ajout de solutions concentrées de ces excipients au sein du mélange. Si nécessaire, le pH de la préparation est ajusté à 7.

Les exemples suivants servent à illustrer l'invention sans toutefois présenter un caractère limitatif.

### Partie A - Synthèse des composés intermédiaires hydrophobes Hy permettant d'obtenir les radicaux -Hy.

| **N°** | **COMPOSES INTERMEDIAIRES HYDROPHOBES** |
|---|---|
| A1 | 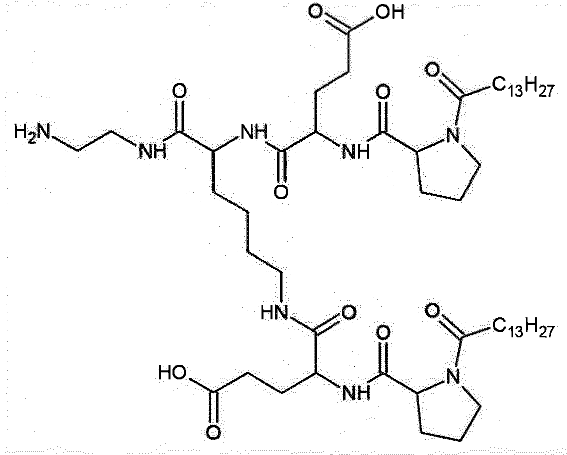 |
| A2 | 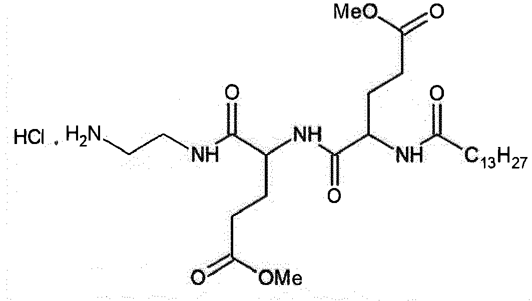 |
| A3 | 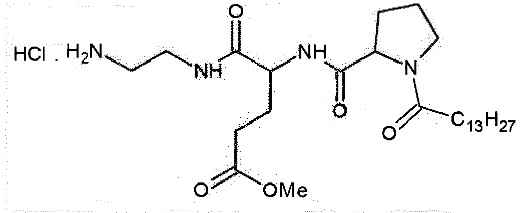 |
| A4 | 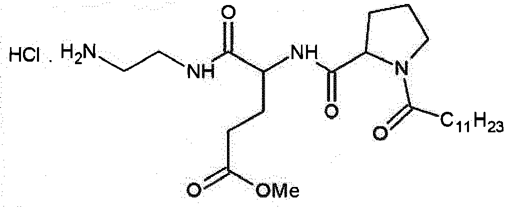 |
| A5 | 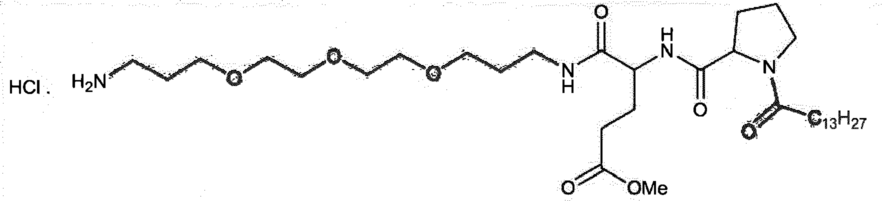 |
| A7 | 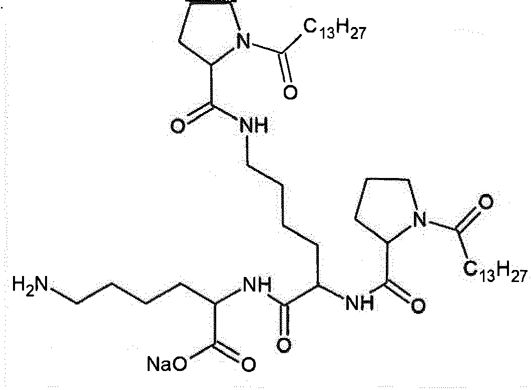 |
| A5a | 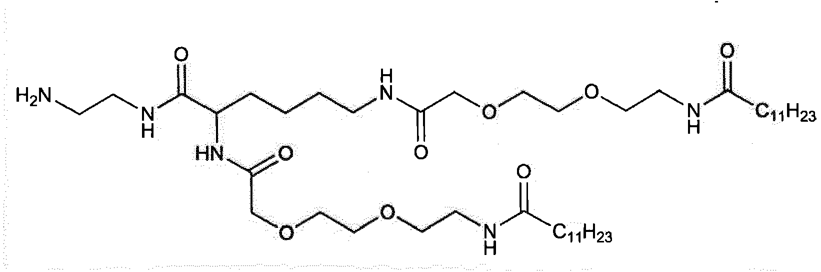 |
| A6a | 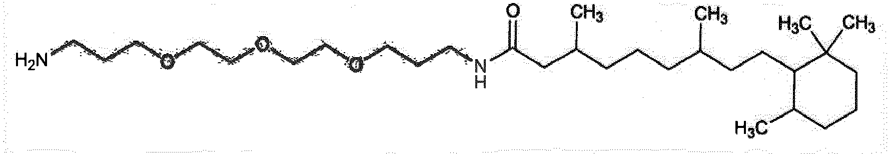 |
| A8 | 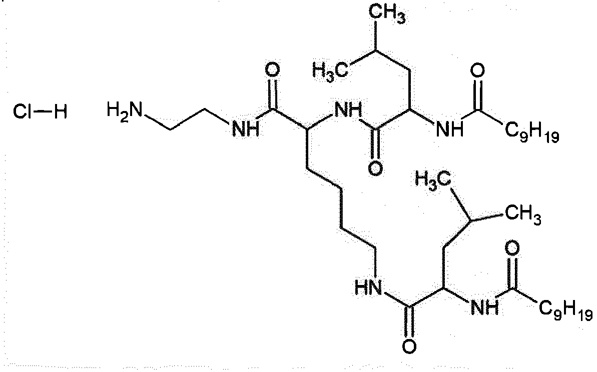 |
| A9 | 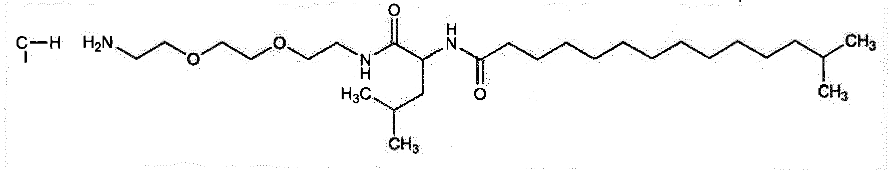 |
| A10 | 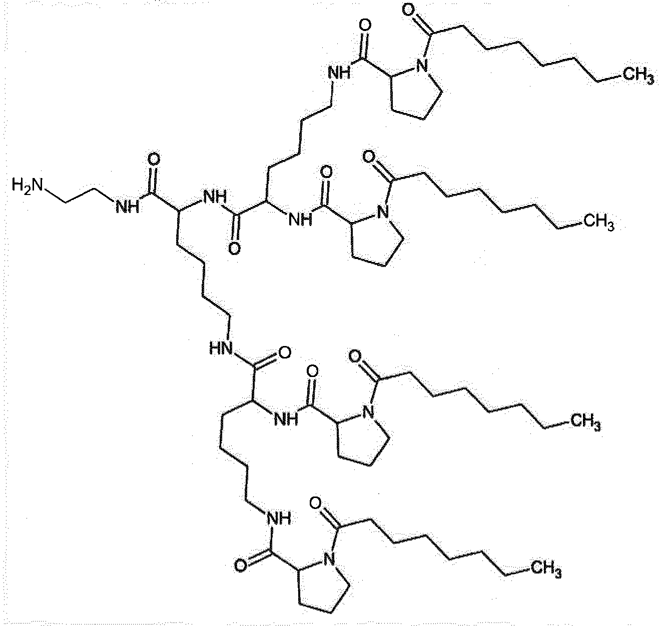 |
| A11 | 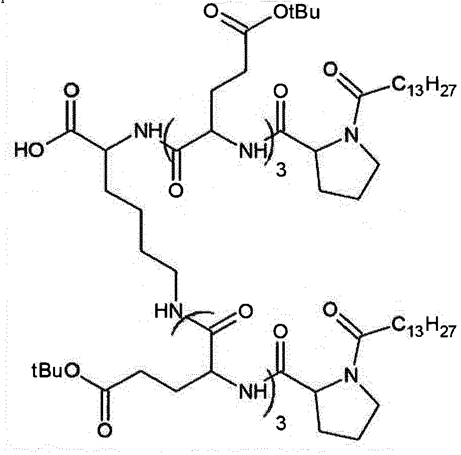 |
| A12 | 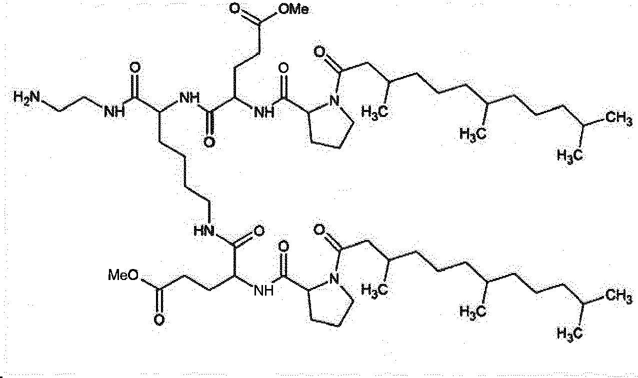 |
| A13 | 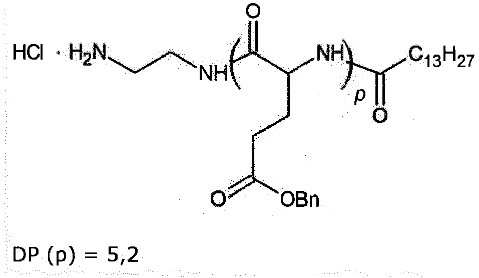 |
| A15 | 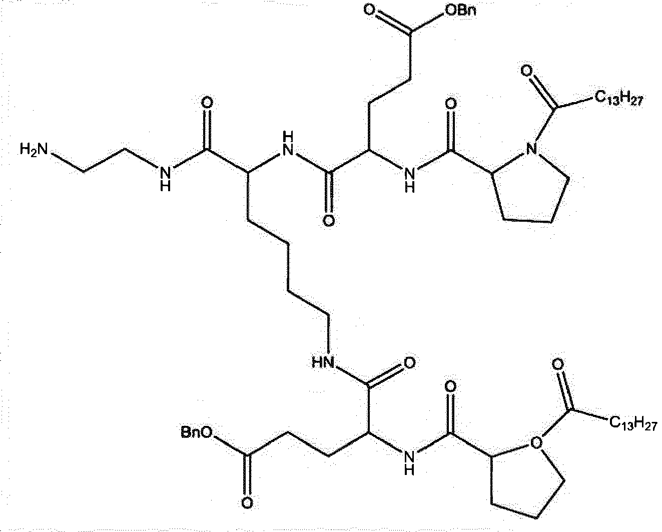 |
| A16 | 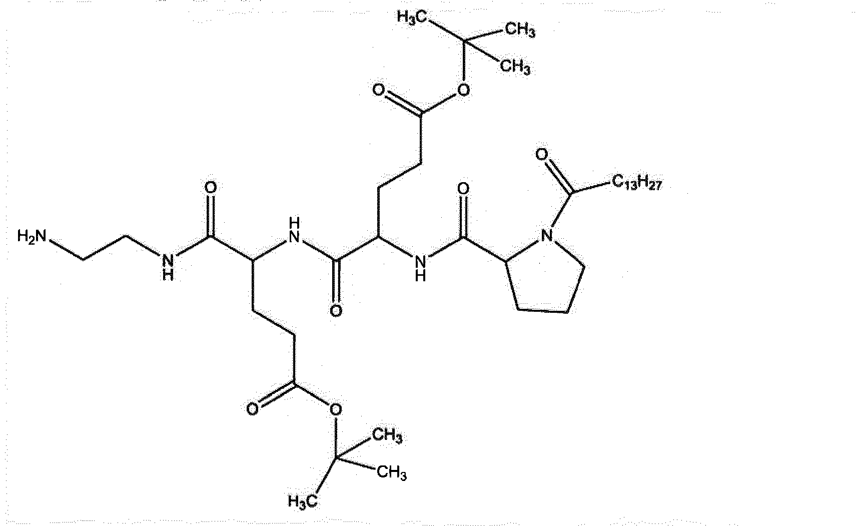 |
| A17 | 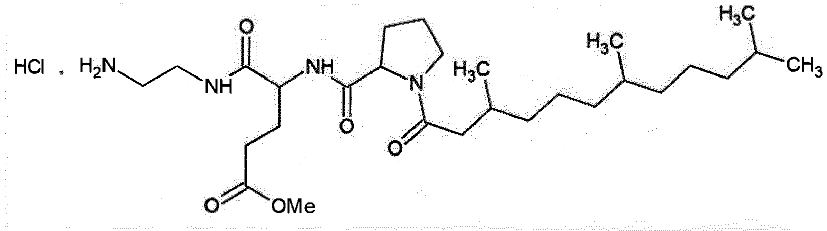 |
| A18 | 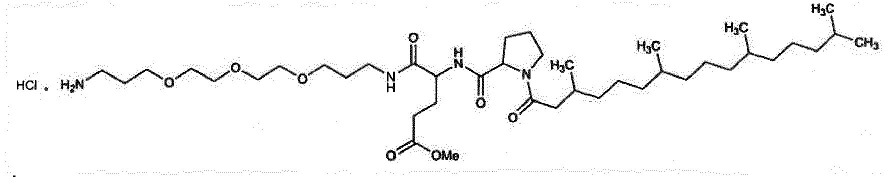 |
| A19 | 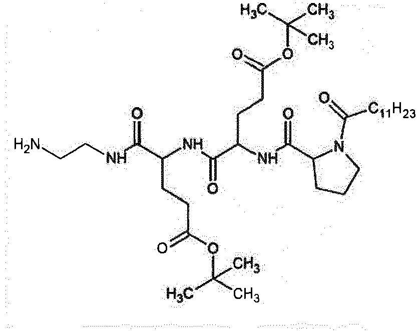 |
| A20 | 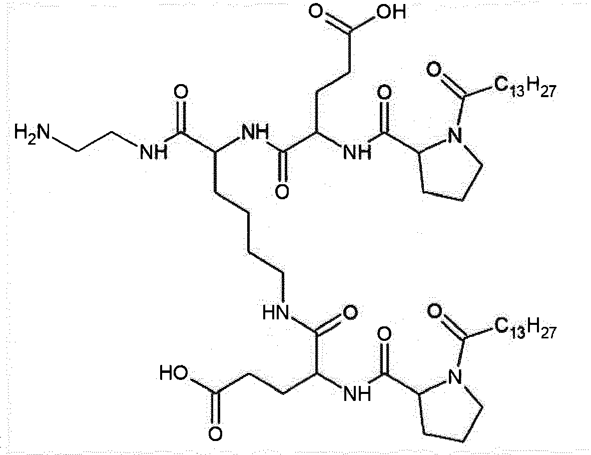 |

### Exemple A1 : molécule A1

### Molécule 1 : Produit obtenu par la réaction entre le Fmoc-Lys(Fmoc)-OH et la résine 2-Cl-trityl chloride.

À une suspension de Fmoc-Lys(Fmoc)-OH (7,32 g, 12,40 mmol) dans du dichlorométhane (60 mL) à température ambiante est ajoutée de la DIPEA (4,32 mL, 24,80 mmol). Après solubilisation complète (10 min), la solution obtenue est versée sur de la résine 2-Cl-trityl chloride préalablement lavée au dichlorométhane (100-200 mesh, 1 % DVB, 1,24 mmol/g) (4,00 g, 4,96 mmol), dans un réacteur adapté à la synthèse peptidique sur support solide. Après 2 h d'agitation à température ambiante, du méthanol grade HPLC (0,8 mL/g résine, 3,2 mL) est ajouté et le milieu est agité à température ambiante pendant 15 min. La résine est filtrée, lavée successivement avec du dichlorométhane (3 x 60 mL), du DMF (2 x 60 mL), du dichlorométhane (2 x 60 mL), de l'isopropanol (1 x 60 mL) et du dichlorométhane (3 x 60 mL).

### Molécule 2 : Produit obtenu par la réaction entre la molécule 1 et un mélange DMF/pipéridine 80:20.

La molécule 1, préalablement lavée au DMF, est traitée avec un mélange DMF/pipéridine 80:20 (60 mL). Après 30 min d'agitation à température ambiante, la résine est filtrée, lavée successivement avec du DMF (3 x 60 mL), de l'isopropanol (1 x 60 mL) et du dichlorométhane (3 x 60 mL).

### Molécule 3 : Produit obtenu par la réaction entre la molécule 2 et le Fmoc-Glu(OtBu)-OH.

À une suspension de Fmoc-Glu(OtBu)-OH (10,55 g, 24,80 mmol) et de 1-[bis(dimethylamino)methyèlene]-1H-1,2,3-triazolo[4,5-b]pyridinium 3-oxide hexafluorophosphate (HATU, 9,43 g, 24,80 mmol) dans un mélange DMF/dichlorométhane 1:1 (60 mL) est ajoutée de la DIPEA (8,64 mL, 49,60 mmol). Après solubilisation complète, la solution obtenue est versée sur la molécule 2. Après 2 h d'agitation à température ambiante, la résine est filtrée, lavée successivement avec du DMF (3 x 60 mL), de l'isopropanol (1 x 60 mL) et du dichlorométhane (3 x 60 mL).

### Molécule 4 : Produit obtenu par la réaction entre la molécule 3 et un mélange DMF/morpholine 50:50.

La molécule 3, préalablement lavée au DMF, est traitée avec un mélange DMF/morpholine 50:50 (60 mL). Après 1 h 15 d'agitation à température ambiante, la résine est filtrée, lavée successivement avec du DMF (3 x 60 mL), de l'isopropanol (1 x 60 mL) et du dichlorométhane (3 x 60 mL).

### Molécule 5 : Produit obtenu par la réaction entre la molécule 4 et la molécule 11.

Par un procédé similaire à celui utilisé pour la molécule 3 appliqué à la molécule 4 et à la molécule 11 (8,07 g, 24,80 mmol) dans du DMF (60 mL), la molécule 5 est obtenue.

### Molécule 6 : Produit obtenu par la réaction entre la molécule 5 et un mélange dichlorométhane/1,1,1,3,3,3-hexafluoro-2-propanol (HFIP) 80:20.

La molécule 5 est traitée avec un mélange dichlorométhane/1,1,1,3,3,3-hexafluoro-2-propanol (HFIP) 80:20 (60 mL). Après 20 min d'agitation à température ambiante, la résine est filtrée et lavée avec du dichlorométhane (2 x 60 mL). Les solvants sont évaporés sous pression réduite. Deux co-évaporations sont ensuite effectuées sur le résidu avec du dichlorométhane (60 mL) puis du diisopropyléther (60 mL). Le produit est purifié par chromatographie sur gel de silice (dichlorométhane, méthanol). Un solide blanc de molécule 6 est obtenu.
Rendement : 2,92 g (52 % sur 6 étapes)
RMN ¹H (CD₃OD, ppm) : 0,90 (6H) ; 1,22-2,47 (88H) ; 3,13-3,25 (2H) ; 3,45-3,76 (4H) ; 4,24-4,55 (5H).
LC/MS (ESI+) : 1131,9 (calculé ([M+H]⁺) : 1131,8).

### Molécule 7: Produit obtenu par la réaction entre la molécule 6 et la N-Boc éthylènediamine.

À une solution de la molécule 6 (2,82 g, 2,49 mmol) dans le Me-THF (20 mL) à température ambiante sont ajoutés successivement du *N*-hydroxybenzotriazole (HOBt, 496 mg, 3,24 mmol) et de la *N*-Boc éthylènediamine (BocEDA, 440 mg, 2,74 mmol). Le mélange est refroidi à 0 °C puis du chlorhydrate de (3-diméthylaminopropyl)-N'-éthylcarbodiimide (EDC, 621 mg, 3,24 mmol) est ajouté. Le milieu réactionnel est agité pendant 15 min à 0 °C puis 18 h à température ambiante. La phase organique est diluée avec du dichlorométhane (30 mL) et lavée par une solution aqueuse saturée en NH₄Cl (2 x 20 mL), une solution aqueuse saturée en NaHCO₃ (2 x 20 mL), et une solution aqueuse saturée en NaCl (2 x 20 mL). La phase organique est séchée sur Na₂SO₄, filtrée et concentrée sous pression réduite. Un solide blanc de la molécule 7 est obtenu après recristallisation dans l'acétonitrile.
Rendement : 2,47 g (78 %)
RMN ¹H (CDCl₃, ppm) : 0,87 (6H) ; 1,09-1,77 (77H) ; 1,84-2,49 (20H) ; 2,99-3,83 (10H) ; 4,16-4,25 (1H) ; 4,27-4,47 (4H) ; 5,68 (0,1H) ; 5,95-6,08 (0,9H) ; 6,91-7,14 (2H) ; 7,43-7,57 (1H) ; 7,68-7,78 (1H) ; 8,22-8,35 (1H).
LC/MS (ESI+) : 1273,9 (calculé ([M+H]⁺) : 1273,9).

### Molécule A1

À une solution de la molécule 7 (2,47 g, 1,94 mmol) dans le dichlorométhane (20 mL) à température ambiante est ajoutée une solution de HCl 4 N dans le dioxane (7,27 mL) puis le milieu est agité pendant 16 h à température ambiante. Après concentration sous pression réduite, co-évaporation et lavage au diisopropyléther, un solide blanc de la molécule A1 sous forme sel de HCl est obtenu. Ce solide est solubilisé dans de l'eau (100 mL) puis le pH est ajusté à 7 par ajout d'une solution aqueuse de NaOH 1 N. La solution est lyophilisée puis le lyophilisat est séché par co-évaporation au toluène. Un solide blanc de molécule A1 est obtenu.
Rendement : 1,64 g (80 %)
RMN ¹H (D₂O, ppm) : 0,90 (6H) ; 1,15-2,59 (70H) ; 3,06-3,86 (10H) ; 4,19-4,43 (5H). LC/MS (ESI+) : 1061,8 (calculé ([M+H]⁺) : 1061,8).

### Exemple A2 : molécule A2

### Molécule 8 : Produit obtenu par le couplage entre l'acide myristique et le L-glutamate de méthyle.

À une solution d'acide myristique (35,0 g, 153,26 mmol) dans le tétrahydrofurane (THF, 315 mL) à 0 °C sont ajoutés successivement du *N-*hydroxysuccinimide (NHS, 17,81 g, 154,79 mmol) et du *N,N-*dicyclohexylcarboxydiimide (DCC, 31,94 g, 154,79 mmol). Le milieu est agité pendant 48 h tout en remontant la température à l'ambiante, filtré sur fritté puis ajouté à une solution de L-glutamate de méthyle (24,95 g, 154,79 mmol) et de *N,N-*diisopropyléthylamine (DIPEA, 99,0 g, 766,28 mmol) dans l'eau (30 mL). Le mélange réactionnel est agité à 20 °C pendant 48 h puis concentré sous pression réduite. De l'eau (200 mL) est ajoutée et le mélange obtenu est traitée par addition successive d'acétate d'éthyle (AcOEt, 100 mL) puis d'une solution aqueuse de Na₂CO₃ à 5 % (50 mL). La phase aqueuse est ensuite lavée une nouvelle fois à l'AcOEt (100 mL), acidifiée par ajout d'une solution aqueuse de HCl 10 % et le produit est extrait au dichlorométhane (DCM, 3 x 150 mL). La phase organique est séchée sur Na₂SO₄, filtrée et concentrée sous pression réduite. Un solide blanc de molécule 8 est obtenu.
Rendement : 47,11 g (84 %)
RMN ¹H (CDCl₃, ppm) : 0,87 (3H) ; 1,07-1,66 (22H) ; 2,02-2,11 (1H) ; 2,18-2,36 (3H) ; 2,39-2,47 (1H) ; 2,50-2,58 (1H) ; 3,69 (3H) ; 4,54-4,59 (1H) ; 6,62 (1H) ; 8,26 (1H). LC/MS (ESI+) : 372,2 (calculé ([M+H]⁺) : 372,3).

### Molécule 9 : Produit obtenu par le couplage entre la molécule 8 et le L-glutamate de méthyle.

Par un procédé similaire à celui utilisé pour la préparation de la molécule 8 et appliqué à la molécule 8 (35,0 g, 94,21 mmol) et au L-glutamate de méthyle (15,33 g, 95,15 mmol), un solide blanc de la molécule 9 est obtenu après recristallisation dans l'acétonitrile.
Rendement : 24,0 g (49 %)
RMN ¹H (DMSO-d6, ppm) : 0,85 (3H) ; 1,06-1,51 (22H) ; 1,70-1,94 (3H) ; 1,96-2,15 (3H) ; 2,29-2,40 (4H) ; 3,58 (3H) ; 3,58 (3H) ; 4,16-4,22 (1H) ; 4,25-4,32 (1H) ; 7,93 (1H) ; 8,16 (1H) ; 12,66 (1H).
LC/MS (ESI+) : 515,3 (calculé ([M+H]⁺) : 515,3).

### Molécule 10 : Produit obtenu par le couplage entre la molécule 9 et la N-Boc éthylènediamine.

À une suspension de la molécule 9 (24,0 g, 46,63 mmol) dans le DCM (285 mL) à 0 °C sont ajoutés successivement du HOBt (714 mg, 46,66 mmol), de la BocEDA (8,97 g, 55,96 mmol) en solution dans le DCM (25 mL) puis du EDC (9,83 g, 51,30 mmol). Le milieu réactionnel est agité pendant 1 h à 0 °C puis 18 h à température ambiante. La phase organique est lavée par une solution aqueuse saturée en NaHCO₃ (2 x 300 mL), une solution aqueuse de HCl 1 N (2 x 300 mL), une solution aqueuse saturée en NaCl (500 mL). Du méthanol (40 mL) est ajouté, la phase organique est séchée sur Na₂SO₄, filtrée et concentrée sous pression réduite. Un solide blanc de la molécule 10 est obtenu après recristallisation dans l'acétonitrile.
Rendement : 27,15 g (89 %)
RMN ¹H (CDCl₃, ppm) : 0,87 (3H) ; 1,07-1,68 (22H) ; 1,42 (9H) ; 1,97-2,18 (4H) ; 2,22-2,31 (2H) ; 2,35-2,55 (4H) ; 3,19-3,29 (2H) ; 3,30-3,38 (2H) ; 3,66 (3H) ; 3,68 (3H) ; 4,34-4,41 (1H) ; 4,42-4,48 (1H) ; 5,54 (1H) ; 6,99-7,18 (2H) ; 7,56 (1H). LC/MS (ESI+) : 657,4 (calculé ([M+H]⁺) : 657,4).

### Molécule A2

À une solution de la molécule 10 (27,15 g, 41,33 mmol) dans un mélange DCM/méthanol (410 mL) à 0 °C est ajoutée une solution de HCl 4 N dans le dioxane (51,7 mL) et le milieu est agité pendant 2 h à 0 °C puis 16 h à température ambiante. Après concentration sous pression réduite et co-évaporation au méthanol (2 x 150 mL), un solide blanc de la molécule A2 sous la forme d'un sel de chlorhydrate est obtenu après recristallisation dans l'acétonitrile.
Rendement : 23,2 g (95 %)
RMN ¹H (DMSO-d6, ppm) : 0,85 (3H) ; 1,05-1,52 (22H) ; 1,71-1,85 (2H) ; 1,87-2,03 (2H) ; 2,07-2,18 (2H) ; 2,24-2,37 (4H) ; 2,84 (2H) ; 3,24-3,38 (2H) ; 3,58 (3H) ; 3,58 (3H) ; 4,17-4,24 (2H) ; 7,95-8,08 (5H) ; 8,14 (1H).
LC/MS (ESI+) : 557,3 (calculé ([M+H]⁺) : 557,4).

### Exemple A3 : molécule A3

### Molécule 11 : Produit obtenu par la réaction entre le chlorure de myristoyle et la L-proline.

A une solution de L-proline (300,40 g, 2,61 mol) dans de la soude aqueuse 2 N (1,63 L) à 0 °C est ajouté lentement sur 1 h du chlorure de myristoyle (322 g, 1,30 mol) en solution dans du dichlorométhane (DCM, 1,63 L). A la fin de l'ajout, le milieu réactionnel est remonté à 20 °C en 3 h, puis agité 2 h supplémentaires. Le mélange est refroidi à 0 °C puis une solution aqueuse de HCl à 37 % (215 mL) est ajoutée en 15 min. Le milieu réactionnel est agité pendant 1 h entre 0 °C et 20 °C. La phase organique est séparée, lavée avec une solution aqueuse de HCl 10 % (3 x 430 mL), une solution aqueuse saturée en NaCl (430 mL), séchée sur Na₂SO₄, filtrée sur coton puis concentrée sous pression réduite. Le résidu est solubilisé dans de l'heptane (1,31 L) à 50 °C, puis la solution est ramenée progressivement à température ambiante. Après amorçage de la cristallisation à l'aide d'une tige en verre, le milieu est à nouveau chauffé à 40 °C pendant 30 min puis ramené à température ambiante pendant 4 h. Un solide blanc est obtenu après filtration sur fritté, lavage à l'heptane (2 x 350 mL) et séchage sous pression réduite.
Rendement : 410 g (97 %)
RMN ¹H (CDCl₃, ppm) : 0,88 (3H) ; 1,28 (20H) ; 1,70 (2H) ; 1,90-2,10 (3H) ; 2,36 (2H) ; 2,51 (1H) ; 3,47 (1H) ; 3,56 (1H) ; 4,61 (1H).
LC/MS (ESI) : 326,4 ; 651,7 ; (calculé ([M+H]⁺) : 326,3 ; ([2M+H]⁺) : 651,6).

### Molécule 12: Produit obtenu par le couplage entre la molécule 11 et le L-glutamate de méthyle.

Par un procédé similaire à celui utilisé pour la préparation de la molécule 8 et appliqué à la molécule 11 (30,0 g, 92,17 mmol) et au L-glutamate de méthyle (15,60 g, 96,78 mmol), un solide blanc de la molécule 12 est obtenu après solubilisation dans l'acétone au reflux, refroidissement à température ambiante et filtration sur fritté. Le filtrat est évaporé et le résidu est précipité dans l'acétone comme précédemment, cette opération étant répétée 3 fois.
Rendement : 15,5 g (36 %)
RMN ¹H (DMSO-d6, ppm) : 0,85 (3H) ; 1,07-1,37 (20H) ; 1,40-1,50 (2H) ; 1,71-2,27 (8H) ; 2,30-2,40 (2H) ; 3,28-3,54 (2H) ; 3,58 (1,3H) ; 3,59 (1,7H); 4,14-4,28 (1H); 4,28-4,37 (1H) ; 8,06 (0,55H) ; 8,33 (0,45H) ; 12,64 (1H).
LC/MS (ESI+) : 469,2 (calculé ([M+H]⁺) : 469,3).

### Molécule 13 : Produit obtenu par le couplage entre la molécule 12 et la N-Boc éthylènediamine.

Par un procédé similaire à celui utilisé pour la préparation de la molécule 10 et appliqué à la molécule 12 (15,5 g, 33,05 mmol) et à la BocEDA (5,83 g, 36,36 mmol), un solide blanc de la molécule 13 est obtenu après recristallisation dans l'acétonitrile.
Rendement : 19,8 g (83 %)
RMN ¹H (DMSO-d6, ppm) : 0,85 (3H) ; 1,07-1,55 (22H) ; 1,37 (9H) ; 1,69-2,19 (7H) ; 2,22-2,36 (3H) ; 2,91-3,17 (4H) ; 3,28-3,60 (5H) ; 4,11-4,18 (0,7H) ; 4,20-4,28 (1H) ; 4,38-4,42 (0,3H) ; 6,74 (1H) ; 7,64 (0,7H) ; 7,87 (0,7H) ; 7,98 (0,3H) ; 8,22 (0,3H). LC/MS (ESI+) : 611,4 (calculé ([M+H]⁺) : 611,4).

### Molécule A3

Par un procédé similaire à celui utilisé pour la préparation de la molécule A2 et appliqué à la molécule 13 (16,8 g, 27,50 mmol), un solide blanc de la molécule A3 sous la forme d'un sel de chlorhydrate est obtenu après recristallisation dans l'acétonitrile.
Rendement : 13,5 g (90 %)
RMN ¹H (DMSO-d6, ppm) : 0,85 (3H) ; 1,08-1,52 (22H) ; 1,70-2,37 (10H) ; 2,80-2,90 (2H) ; 3,22-3,62 (4H) ; 3,57 (3H) ; 4,15-4,28 (1,75H) ; 4,41-4,44 (0,25H) ; 7,81-8,13 (4,5H) ; 8,24-8,29 (0,25H) ; 8,33-8,39 (0,25H).
LC/MS (ESI+) : 511,3 (calculé ([M+H]⁺) : 511,4).

### Exemple A4 : Molécule A4

### Molécule 14 : Produit obtenu par la réaction entre le chlorure de lauroyle et la L-proline

Par un procédé similaire à celui utilisé pour la préparation de la molécule 11 et appliqué à au chlorure de lauroyle (27,42 g, 685,67 mmol) et à la L-proline (60,0 g, 247,27 mmol), un solide blanc de la molécule 14 est obtenu.
Rendement : 78,35 g (96 %)
RMN ¹H (CDCl₃, ppm) : 0,87 (3H) ; 1,26 (16H) ; 1,70 (2H) ; 1,90-2,10 (3H) ; 2,35 (2H) ; 2,49 (1H) ; 3,48 (1H) ; 3,56 (1H) ; 4,60 (1H).
LC/MS (ESI+) : 298,1 (calculé ([M+H]⁺) : 298,2).

### Molécule 15 : Produit obtenu par le couplage entre la molécule 14 et le L-glutamate de méthyle.

Par un procédé similaire à celui utilisé pour la préparation de la molécule 8 et appliqué à la molécule 14 (34,64 g, 116,46 mmol) et au L-glutamate de méthyle (19,14 g, 118,79 mmol), un solide blanc de la molécule 15 est obtenu après recristallisation dans l'acétonitrile.
Rendement : 37,28 g (73 %)
RMN ¹H (CDCl₃, ppm) : 0,85 (3H) ; 1,08-1,42 (16H) ; 1,54-1,06 (2H) ; 1,80-2,47 (10H) ; 3,42-3,80 (2H) ; 3,65 (2,55H) ; 3,67 (0,45H) ; 4,37-4,40 (0,15H) ; 4,51-4,58 (0,85H) ; 4,58-4,67 (1H) ; 7,26 (0,15H) ; 7,65 (0,85H) ; 8,06 (1H).
LC/MS (ESI+) : 441,1 (calculé ([M+H]⁺) : 441,3).

### Molécule 16: Produit obtenu par le couplage entre la molécule 15 et la N-Boc éthylènediamine.

Par un procédé similaire à celui utilisé pour la préparation de la molécule 10 et appliqué à la molécule 15 (37,30 g, 84,66 mmol) et à la BocEDA (14,92 g, 93,13 mmol), un solide blanc de la molécule 16 est obtenu après recristallisation dans l'acétonitrile.
Rendement : 43,10 g (87 %)
RMN ¹H (DMSO-d6, ppm) : 0,85 (3H) ; 1,08-1,53 (18H) ; 1,37 (9H) ; 1,70-2,36 (10H) ; 2,91-3,60 (9H) ; 4,11-4,18 (0,7H) ; 4,21-4,28 (1H) ; 4,38-4,42 (0,3H) ; 6,38 (0,1H) ; 6,74 (0,9H) ; 7,65 (0,7H) ; 7,87 (0,7H) ; 7,99 (0,3H) ; 8,22 (0,3H).
LC/MS (ESI+) : 583,4 (calculé ([M+H]⁺) : 583,4).

### Molécule A4

Par un procédé similaire à celui utilisé pour la préparation de la molécule A2 et appliqué à la molécule 16 (43,10 g, 73,96 mmol), un solide blanc de la molécule A4 sous la forme d'un sel de chlorhydrate est obtenu après recristallisation dans l'acétonitrile.
Rendement : 31,90 g (83 %)
RMN ¹H (DMSO-d6, ppm) : 0,85 (3H) ; 1,05-1,37 (16H) ; 1,39-1,52 (2H) ; 1,70-2,37 (10H) ; 2,29-2,91 (2H) ; 3,20-3,62 (7H) ; 4,16-4,29 (1,7H) ; 4,42-4,46 (0,3H) ; 7,86-8,18 (4,6H) ; 8,32 (0,3H) ; 8,40 (0,3H).
LC/MS (ESI+) : 483,2 (calculé ([M+H]⁺) : 483,3).

### Exemple AS : molécule AS

### Molécule 17: Produit obtenu par la réaction entre la 1-amino-4,7,10-trioxa-13-tridécane amine et le tert-butyl phénylcarbonate.

À une solution de 1-amino-4,7,10-trioxa-13-tridécane amine (112,29 g, 509,71 mmol) dans l'éthanol (510 mL) à 80 °C est ajouté au goutte à goutte du *tert-*butyl phénylcarbonate (49,50 g, 254,86 mmol). Le milieu réactionnel est agité à 80 °C pendant 3 h 30 puis concentré sous pression réduite. Le résidu est solubilisé dans de l'eau (250 mL), le pH est ajusté à 2,3 avec une solution de HCl 37 % et le mélange est extrait au méthyl *tert*-butyléther (MTBE, 2 x 150 mL). La phase aqueuse est basifiée à pH 12,6 par addition d'une solution de NaOH 2 N et extraite au DCM (3 x 250 mL). La phase organique est lavée avec une solution aqueuse de NaOH 1 N (1 x 100 mL), une solution aqueuse saturée en NaCl (100 mL), séchée sur Na₂SO₄, filtrée et concentrée sous pression réduite. Une huile jaune de molécule 17 est obtenue.
Rendement : 54,4 g (67 %)
RMN ¹H (CDCl₃, ppm) : 1,40-1,58 (11H) ; 1,73-1,81 (4H) ; 2,80-2,84 (2H) ; 3,20-3,70 (14H) ; 5,11 (1H).
LC/MS (ESI+) : 321,2 (calculé ([M+H]⁺) : 321,2).

### Molécule 18 : Produit obtenu par le couplage entre la molécule 12 et la molécule 17.

Par un procédé similaire à celui utilisé pour la préparation de la molécule 10 et appliqué à la molécule 12 (20,46 g, 43,66 mmol) et à la molécule 17 (16,79 g, 52,39 mmol), une cire blanche de la molécule 18 est obtenue après purification par chromatographie flash (éluant : DCM, méthanol), solubilisation du résidu dans le DCM (300 mL), lavages de la phase organique avec une solution aqueuse de NaHCO₃ (2 x 150 mL), une solution aqueuse de HCl 10 % (2 x 150 mL), une solution aqueuse saturée en NaCl (2 x 150 mL), séchage sur Na₂SO₄ et concentration sous pression réduite.
Rendement : 30,15 g (90 %)
RMN ¹H (DMSO-d6, ppm) : 0,85 (3H) ; 1,09-1,52 (31H) ; 1,55-1,67 (4H) ; 1,69-2,36 (10H) ; 2,91-2,98 (2H) ; 3,02-3,17 (2H) ; 3,28-3,61 (17H) ; 4,12-4,17 (0,7H) ; 4,20-4,28 (1H) ; 4,39-4,42 (0,3H) ; 6,37 (0,1H) ; 6,71 (0,9H) ; 7,59 (0,7H) ; 7,85 (0,7H) ; 7,94 (0,3H) ; 8,21 (0,3H).
LC/MS (ESI+) : 771,4 (calculé ([M+H]⁺) : 771,5).

### Molécule A5

Par un procédé similaire à celui utilisé pour la préparation de la molécule A2 et appliqué à la molécule 18 (30,0 g, 38,91 mmol), un solide blanc de la molécule A5 sous la forme d'un sel de chlorhydrate est obtenu après solubilisation du résidu dans l'eau (500 mL) et lyophilisation.
Rendement : 25,2 g (91 %)
RMN ¹H (DMSO-d6, ppm) : 0,85 (3H) ; 1,06-1,37 (20H) ; 1,39-1,52 (2H) ; 1,58-1,66 (2H) ; 1,70-2,37 (12H) ; 2,78-2,85 (2H) ; 3,01-3,15 (2H) ; 3,31-3,62 (17H) ; 4,11-4,17 (0,7H) ; 4,19-4,27 (1H) ; 4,41-4,44 (0,3H) ; 7,63-7,71 (0,7H) ; 7,90-8,24 (4H) ; 8,28-8,35 (0,3H).
LC/MS (ESI+) : 671,4 (calculé ([M+H]⁺) : 671,5).

### Exemple A7 : molécule A7

### Molécule 21 : Produit obtenu par couplage entre la molécule 11 et la L-lysine.

Par un procédé similaire à celui utilisé pour la préparation de la molécule 8 appliqué à la molécule 11 (133,00 g, 408,61 mmol) et à la L-lysine (31,36 g, 214,52 mmol), un solide blanc de molécule 21 est obtenu après cristallisation 2 fois dans l'acétone.
Rendement : 106,50 g (68 %)
RMN ¹H (DMSO-d₆, ppm) : 0,85 (6H) ; 1,26 (40H) ; 1,35-1,50 (6H) ; 1,50-2,10 (10H) ; 2,10-2,25 (4H) ; 3,01 (2H) ; 3,31-3,55 (4H) ; 4,10-4,40 (3H) ; 7,68 (0,6H) ; 7,97 (1H) ; 8,27 (0,4H) ; 12,50 (1H).
LC/MS (ESI): 761,8 ; (calculé ([M+H]⁺): 762,1).

### Molécule 22 : Produit obtenu par couplage entre la molécule 21 et la N-Boc-L-lysinate de méthyle.

Par un procédé similaire à celui utilisé pour la préparation de la molécule 10 appliqué à la molécule 21 (43,00 g, 56,50 mmol) en solution dans le THF et au chlorhydrate de *N*-Boc-L-lysinate de méthyle (20,12 g, 67,79 mmol), un solide transparent de molécule 22 est obtenu et utilisé sans purification complémentaire.
Rendement : 55,80 g (98 %)
RMN ¹H (DMSO-d6, ppm) : 0,86 (6H) ; 1,08-2,03 (64H) ; 1,37 (9H) ; 2,07-2,30 (4H) ; 2,84-3,09 (4H) ; 3,29-3,57 (4H) ; 3,58-3,65 (3H) ; 4,14-4,43 (4H) ; 6,40 (0,1H) ; 6,74 (0,9H) ; 7,69 (0,6H) ; 7,82 (0,6H) ; 7,95-8,06 (1H) ; 8,11-8,20 (0,4H) ; 8,26 (0,4H). LC/MS (ESI) : 1003,8 (calculé ([M+H]⁺) : 1003,8).

### Molécule 23 : Produit obtenu par saponification de la molécule 23.

Une solution de molécule 22 (55,80 g, 55,61 mmol) dans un mélange THF/eau 1:1 (370 mL) à 0 °C est traitée par addition lente d'une solution de LiOH (2,00 g, 83,41 mmol) dans l'eau (185 mL). Après 16 h d'agitation à 0 °C, le milieu est concentré sous pression réduite et le résidu est repris dans l'eau (500 mL). Du DCM (500 mL) est ajouté, le mélange hétérogène est refroidi à 10 °C et acidifié par ajout d'une solution aqueuse de HCl 10 % jusqu'à pH 1. La phase aqueuse est extraite au DCM (2 x 300 mL), les phases organiques combinées sont lavées par une solution aqueuse saturée en NaCl (2 x 300 mL), séchées sur Na₂SO₄, filtrées et concentrées sous pression réduite. Un solide blanc de molécule 23 est obtenu après cristallisation dans l'acétone.
Rendement : 46,10 g (84 %)
RMN ¹H (pyridine-d6, ppm) : 0,85 (6H) ; 1,05-2,03 (67H) ; 2,07-2,61 (10H) ; 3,12-3,93 (8H) ; 4,54-4,93 (2H) ; 4,98-5,16 (2H) ; 7,35-7,45 (1H) ; 8,34-8,63 (1H) ; 8,94-9,41 (2H).
LC/MS (ESI) : 989,8 (calculé ([M+H]⁺) : 989,8).

### Molécule A7

À une solution de la molécule 23 (12,00 g, 12,13 mmol) dans le dichlorométhane (40 mL) à 0 °C est ajoutée une solution de HCl 4 N dans le dioxane (15,20 mL) puis le milieu est agité pendant 15 h à 0 °C et 5 h température ambiante. Le mélange réactionnel est concentré sous pression réduite, le résidu est solubilisé dans un mélange de DCM (120 mL) et de NaOH 2 N (60 mL). Après séparation des phases, la phase organique est lavée par une solution de NaOH 2 N (60 mL), séchée sur Na₂SO₄ et concentrée sous pression réduite.
Rendement : 10,90 g (98 %)
RMN ¹H (DMSO-d6, ppm) : 0,86 (6H) ; 1,05-2,27 (70H) ; 2,45-2,52 (2H) ; 2,90-3,58 (6H) ; 3,67-3,76 (1H) ; 4,02-4,10 (0,6H) ; 4,11-4,17 (0,4H) ; 4,20-4,26 (0,6H) ; 4,30-4,39 (1H) ; 4,42-4,46 (0,4H) ; 7,29-7,42 (1H) ; 7,71-7,80 (0,6H) ; 7,97-8,05 (0,6H) ; 8,10-8,24 (0,4H) ; 8,33-8,45 (0,4H).
LC/MS (ESI) : 887,7 (calculé ([M-H]⁻) : 887,7).

### Exemple A5a : molécule A5a

### Molécule 3a : Produit obtenu par la réaction entre le Fmoc-Lys(Fmoc)-OH et la résine 2-Cl-trityl chloride.

À une suspension de Fmoc-Lys(Fmoc)-OH (7,32 g, 12,40 mmol) dans du DCM (60 mL) à température ambiante est ajoutée de la DIPEA (4,32 mL, 24,80 mmol). Après solubilisation complète (10 min), la solution obtenue est versée sur de la résine 2-Cl-trityl chloride (100-200 mesh, 1% DVB, 1,24 mmol/g) (4,00 g, 4,96 mmol) préalablement lavée au DCM, dans un réacteur adapté à la synthèse peptidique sur support solide. Après 2 h d'agitation à température ambiante, du méthanol grade HPLC (0,8 mL/g résine, 3,2 mL) est ajouté et le milieu est agité à température ambiante pendant 15 min. La résine est filtrée, lavée successivement avec du DCM (3 x 60 mL), du DMF (2 x 60 mL), du DCM (2 x 60 mL), de l'isopropanol (1 x 60 mL) et du DCM (3 x 60 mL).

### Molécule 4a : Produit obtenu par réaction entre la molécule 3a et un mélange DMF/pipéridine 80:20.

La molécule 3a, préalablement lavée au DMF, est traitée avec un mélange DMF/pipéridine 80:20 (60 mL). Après 30 min d'agitation à température ambiante, la résine est filtrée, lavée successivement avec du DMF (3 x 60 mL), de l'isopropanol (1 x 60 mL) et du DCM (3 x 60 mL).

### Molécule 5a : Produit obtenu par réaction entre la molécule 4a et l'acide 8-(9-Fluorénylméthyloxycarbonyl-amino)-3,6-dioxaoctanoique (Fmoc-O2Oc-OH).

À une suspension de Fmoc-O2Oc-OH (9,56 g, 24,80 mmol) et de 1-[bis(diméthylamino)methylene]-1H-1,2,3-triazolo[4,5-b]pyridinium 3-oxide hexafluorophosphate (HATU, 9,43 g, 24,80 mmol) dans un mélange DMF/DCM 1:1 (60 mL) est ajoutée de la DIPEA (8,64 mL, 49,60 mmol). Après solubilisation complète, la solution obtenue est versée sur la molécule 4a. Après 2 h d'agitation à température ambiante, la résine est filtrée, lavée successivement avec du DMF (3 x 60 mL), de l'isopropanol (1 x 60 mL) et du dichlorométhane (3 x 60 mL).

### Molécule 6a : Produit obtenu par réaction entre la molécule 5a et un mélange DMF/pipéridine 80:20.

Par un procédé similaire à celui utilisé pour la molécule 4a appliqué à la molécule 5a, la molécule 6a est obtenue.

### Molécule 7a : Produit obtenu par réaction entre la molécule 6a et l'acide laurique.

Par un procédé similaire à celui utilisé pour la molécule 5a appliqué à la molécule 6a et à l'acide laurique (4,97 g, 24,80 mmol) dans du DMF (60 mL), la molécule 7a est obtenue.

### Molécule 8a : Produit obtenu par réaction entre la molécule 7a et un mélange dichlorométhane/1,1,1,3,3,3-hexafluoro-2-propanol (HFIP) 80:20.

La molécule 7a est traitée avec un mélange dichlorométhane/1,1,1,3,3,3-hexafluoro-2-propanol (HFIP) 80:20 (60 mL). Après 20 min d'agitation à température ambiante, la résine est filtrée et lavée avec du dichlorométhane (2 x 60 mL). Les solvants sont évaporés sous pression réduite. Deux co-évaporations sont ensuite effectuées sur le résidu avec du dichlorométhane (60 mL) puis du diisopropyléther (60 mL). Un solide blanc de molécule 8a est obtenu après recristallisation dans l'acétonitrile. Rendement : 2,63 g (66 % sur 6 étapes)
RMN ¹H (CDCl₃, ppm) : 0,87 (6H) ; 1,09-1,66 (40H) ; 1,77-1,98 (2H) ; 2,13-2,29 (4H) ; 3,24-3,75 (18H) ; 3,95-4,07 (4H) ; 4,65-4,70 (1H) ; 6,23-6,37 (1H) ; 6,39-6,62 (1H) ; 6,74-6,91 (1H) ; 7,38-7,54 (1H).
LC/MS (ESI) : 801,6 (calculé ([M+H]⁺) : 801,6).

### Molécule 9a : Produit obtenu par la réaction entre la molécule 8a et la N-Boc éthylènediamine.

À une solution de la molécule 8a (2,63 g, 3,29 mmol) dans le chloroforme (20 mL) à température ambiante sont ajoutés successivement du HOBt (654 mg, 4,27 mmol) et de la BocEDA (580 mg, 3,62 mmol). Le mélange est refroidi à 0 °C puis du EDC (819 mg, 4,27 mmol) est ajouté. Le milieu réactionnel est agité pendant 15 min à 0 °C puis 18 h à température ambiante. La phase organique est lavée par une solution aqueuse saturée en NH₄Cl (2 x 10 mL), une solution aqueuse saturée en NaHCO₃ (2 x 10 mL), et une solution aqueuse saturée en NaCl (2 x 10 mL). La phase organique est séchée sur Na₂SO₄, filtrée et concentrée sous pression réduite. Un solide blanc de la molécule 9a est obtenu après purification par chromatographie sur gel de silice (éluant : dichlorométhane, méthanol).
Rendement : 2,37 g (76 %)
RMN ¹H (CDCl₃, ppm) : 0,87 (6H) ; 1,08-1,47 (34H) ; 1,43 (9H) ; 1,48-1,70 (7H) ; 1,78-1,87 (1H) ; 2,14-2,25 (4H) ; 3,16-3,71 (22H) ; 3,92-4,04 (4H) ; 4,47-4,52 (1H) ; 5,33 (1H) ; 6,10 (1H) ; 6,65-7,01 (1H) ; 7,11-7,30 (2H) ; 7,47-7,63 (1H).

### Molécule A5a

À une solution de la molécule 9a (2,37 g, 2,51 mmol) dans le dichlorométhane (50 mL) à température ambiante est ajoutée une solution de HCl 4 M dans le dioxane (6,3 mL) puis le milieu est agité pendant 2 h à température ambiante. Après concentration sous pression réduite, le résidu est solubilisé dans du dichlorométhane (50 mL) puis lavé avec une solution aqueuse de NaOH 1 N (2 x 12,5 mL) et une solution aqueuse saturée en NaCl (25 mL). La phase organique est séchée sur Na₂SO₄, filtrée et concentrée sous pression réduite. Un solide blanc de la molécule A5a est obtenu après recristallisation dans l'acétonitrile.
Rendement : 1,57 g (74 %)
RMN ¹H (CDCl₃, ppm) : 0,87 (6H) ; 1,08-1,43 (34H) ; 1,48-1,71 (7H) ; 1,74-1,93 (3H) ; 2,14-2,25 (4H) ; 2,79-2,86 (2H) ; 3,17-3,71 (20H) ; 3,93-4,05 (4H) ; 4,47-4,54 (1H) ; 6,08-6,29 (1H) ; 6,84-7,01 (1H) ; 7,15-7,32 (2H) ; 7,50-7,64 (1H).
LC/MS (ESI) : 843,6 (calculé ([M+H]⁺) : 843,7).

### Exemple A6a : molécule A6a

### Molécule 10a : Produit obtenu par hydrogénation de l'acide rétinoïque.

Une solution d'acide rétinoïque (19,0 g, 63,24 mmol) dans du méthanol (450 mL) en présence de palladium sur charbon 10 % (1,9 g) est placée sous atmosphère d'hydrogène (1 atm) à température ambiante. Après une nuit, le milieu réactionnel est filtré sur fritté puis le filtrat est concentré sous pression réduite. Une huile incolore de molécule 10a est obtenue.
Rendement : 19,50 g (99 %)
RMN ¹H (CDCl₃, ppm) : 0,45-2,01 (35 H) ; 2,10-2,17 (1H) ; 2,33-2,38 (1H) ; 11,14 (1H).
LC/MS (ESI) : 309,3 ; (calculé ([M-H]⁻) : 309,3).

### Molécule 11a : Produit obtenu par couplage entre la Boc-1-amino-4,7,10-trioxa-13-tridécane amine (BocTOTA) et la molécule 10a.

Par un procédé similaire à celui utilisé pour la préparation de la molécule 9a appliqué à la molécule 10a (19,3 g, 62,15 mmol) et à la BocTOTA (23,9 g, 74,58 mmol), une huile orange de la molécule 11a est obtenue.
Rendement : 37,05 g (97 %)
RMN ¹H (CDCl₃, ppm) : 0,43-1,71 (49 H) ; 2,13-2,17 (1H) ; 3,17-3,24 (2H); 3,32-3,39 (2H) ; 3,51-3,66 (12H) ; 4,77 (0,1H) ; 4,94 (0,9H) ; 6,13 (0,9H) ; 6,29 (0,1H). LC/MS (ESI) : 613,5 ; (calculé ([M+H]⁺) : 613,5).

### Molécule A6a

Par un procédé similaire à celui utilisé pour la préparation de la molécule A5a appliqué à la molécule 11a (34,9 g, 56,94 mmol), une huile orange de la molécule A6a est obtenue.
Rendement : 28,5 g (97 %)
RMN ¹H (CDCl₃, ppm) : 0,41-1,96 (42 H) ; 2,13 (1H) ; 2,78 (2H) ; 3,31-3,36 (2H) ; 3,53 (4H) ; 3,55-3,58 (4H) ; 3,60-3,63 (4H) ; 6,43 (1H).
LC/MS (ESI) : 513,5 ; (calculé ([M+H]⁺) : 513,5).

### Exemple A8 : Molécule A8

### Molécule 15a : Produit obtenu par la réaction entre l'acide décanoïque et la L-leucine.

Par un procédé similaire à celui utilisé pour la préparation de la molécule 8 appliqué à l'acide décanoïque (8,77 g, 50,94 mmol) et à la L-leucine (7,00 g, 53,36 mmol), un solide blanc de la molécule 15a est obtenu.
Rendement : 9,17 g (66 %)
RMN ¹H (DMSO-d6, ppm) : 0,82-0,89 (9H) ; 1,18-1,65 (17H) ; 2,04-2,14 (2H) ; 4,19-4,23 (1H) ; 7,98 (1H) ; 12,40 (1H).
LC/MS (ESI) : 286,2 (calculé ([M+H]⁺) : 286,2).

### Molécule 16a : Produit obtenu par la réaction entre la molécule 15a et l'ester méthylique de la L-lysine.

À une solution de molécule 15a (9,16 g, 32,11 mmol) dans le THF (160 mL) sont ajoutés successivement de la triéthylamine (8,12 g, 80,27 mmol) et du 2-(1H-benzotriazol-1-yl)-1,1,3,3-tétraméthyluronium tétrafluoroborate (TBTU) et le milieu est agité pendant 30 min à température ambiante. Le dichlorhydrate d'ester méthylique de la L-lysine (3,93 g, 16,86 mmol) est ajouté et le milieu réactionnel est agité pendant 3 h puis concentré sous pression réduite. Le résidu est dilué à l'AcOEt (200 mL), la phase organique est filtrée et lavée par une solution aqueuse de HCl 1 N puis à l'eau, séchée sur Na₂SO₄, filtrée et concentrée sous pression réduite. Un solide blanc de la molécule 16a est obtenu après trituration du résidu dans l'acétonitrile.
Rendement : 7,33 g (66 %)
RMN ¹H (DMSO-d6, ppm) : 0,80-0,91 (18H) ; 1,06-1,72 (38H) ; 2,03-2,16 (4H) ; 2,91-3,07 (2H) ; 3,60 (1,15H) ; 3,61 (1,85H) ; 4,13-4,28 (2H) ; 4,33-4,44 (1H) ; 7,79-7,92 (3H) ; 8,13-8,26 (1H).
LC/MS (ESI) 695,7 (calculé ([M+H]⁺) : 695,6).

### Molécule 17a : Produit obtenu par la saponification de la molécule 16a.

À une solution de molécule 16a (7,33 g, 10,55 mmol) dans un mélange THF/méthanol/eau (105 mL) est ajouté du LiOH (505,13 mg, 21,09 mmol) à 0 °C puis le milieu est agité pendant 20 h à température ambiante et concentré sous pression réduite. La phase aqueuse est acidifiée avec une solution de HCl 1 N jusqu'à pH 1 et le solide formé est filtré, lavé à l'eau et séché sous pression réduite pour conduire à un solide blanc de la molécule 17a.
Rendement : 7,09 g (99 %)
RMN ¹H (DMSO-d6, ppm) : 0,80-0,89 (18H) ; 1,18-1,73 (40H) ; 2,03-2,16 (4H) ; 2,91-3,05 (2H) ; 4,03-4,13 (1H) ; 4,21-4,27 (1H) ; 4,31-4,40 (1H) ; 7,79-8,02 (4H). LC/MS (ESI) : 681,7 (calculé ([M+H]⁺) : 681,6).

### Molécule 18a : Produit obtenu par la réaction entre la molécule 17a et la N-Boc éthylènediamine.

Par un procédé similaire à celui utilisé pour la préparation de la molécule 16a appliqué à la molécule 17a (7,09 g, 10,41 mmol) et à la *N*-Boc éthylènediamine (1,83 g, 11,45 mmol), un solide blanc de molécule 18a est obtenu après trituration dans l'acétonitrile.
Rendement : 6,64 g (77 %)
RMN ¹H (DMSO-d6, ppm) : 0,80-0,91 (18H) ; 1,15-1,73 (49H) ; 2,03-2,18 (4H) ; 2,92-3,13 (6H) ; 4,05-4,30 (3H) ; 6,71-6,83 (1H) ; 7,69-8,23 (5H).
LC/MS (ESI) : 824,0 (calculé ([M+H]⁺) : 823,7).

### Molécule A8

Par un procédé similaire à celui utilisé pour la molécule A5a appliqué à la molécule 18a (3,00 g, 3,64 mmol) sans lavage basique, un solide beige de molécule A8 sous la forme d'un sel de chlorhydrate est obtenu après co-évaporation 4 fois du résidu dans le méthanol.
Rendement : 2,66 g (96 %)
RMN ¹H (DMSO-d6, ppm) : 0,80-0,91 (18H) ; 1,15-1,76 (40H) ; 2,03-2,19 (4H) ; 1,78-2,89 (2H) ; 2,91-3,07 (2H) ; 3,22-3,37 (2H) ; 4,08-4,14 (1H) ; 4,17-4,28 (2H) ; 7,81-8,36 (8H).
LC/MS (ESI) : 723,7 (calculé ([M+H]⁺) : 723,6).

### Exemple A9 : Molécule A9

### Molécule 19a : Acide 13-méthyltétradécanoïque.

Dans un tricol sec sous argon est introduit du magnésium (5,50 g, 226,3 mmol) en copeaux. Le magnésium est recouvert de THF (25 mL) anhydre et quelques gouttes de 1-bromo-2-méthylpropane sont ajoutées à température ambiante pour initier la réaction. Après l'observation d'un exotherme et un léger trouble du milieu, le reste du 1-bromo-2-méthylpropane (28,42 g, 207 mmol) dilué dans du THF (60 mL) est ajouté au goutte-à-goutte en 1 h alors que la température du milieu reste stable entre 65 et 70 °C. Le milieu réactionnel est ensuite chauffé à reflux pendant 2 h.

Dans un tricol sous argon, à une solution de CuCI (280 mg, 2,83 mmol) dissout dans la N-méthylpyrrolidone (NMP) préalablement distillée à 0 °C est ajoutée au goutte-à-goutte une solution d'acide 11-bromoundécanoïque (25 g, 94,27 mmol) dissout dans le THF (60 mL). À cette solution est ensuite ajoutée au goutte-à-goutte la solution de l'organomagnésien légèrement chaude diluée dans le THF (50 mL) de façon à maintenir la température du milieu en dessous de 25 °C. Le mélange est ensuite agité à température ambiante pendant 16 h. Le milieu est refroidi à 0 °C et la réaction est stoppée par addition lente d'une solution aqueuse d'HCl 1 N jusqu'à pH 1 (300 mL) et le milieu est extrait à l'hexane (100 mL) et à l'acétate d'éthyle (2 x 75 mL). Après lavage de la phase organique avec une solution aqueuse d'HCl 1 N (100 mL), de l'eau (100 mL) et séchage sur Na₂SO₄, la solution est filtrée et concentrée sous vide pour donner un solide brun. Après purification par chromatographie flash (cyclohexane, acétate d'éthyle), un solide blanc est obtenu.
Rendement : 18,1 g (79 %)
RMN ¹H (CDCl₃, ppm) : 0,87 (6H) ; 1,11-1,18 (2H) ; 1,20-1,38 (16H) ; 1,51 (1H) ; 1,63 (2H) ; 2,35 (2H).

### Molécule 20 : Produit obtenu par la réaction entre la molécule 19a et la L-leucine.

À une solution de molécule 19a (18,05 g, 74,46 mmol) dans le THF (745 mL) à température ambiante sont ajoutés successivement du DCC (14,63 g, 70,92 mmol) et du NHS (8,16 g, 70,92 mmol). Après 40 h d'agitation à température ambiante, le milieu est refroidi à 0 °C pendant 20 min, filtré sur fritté. De la L-leucine (9,77 g, 74,46 mmol), de la DIPEA (86 mL) et de l'eau (150 mL) sont ajoutés au filtrat. Après 20 h d'agitation à température ambiante, le milieu est dilué avec une solution aqueuse saturée de NaHCO₃ (200 mL). La phase aqueuse est lavée à l'acétate d'éthyle (2 x 200 mL) et acidifiée avec une solution aqueuse d'HCl 2 N jusqu'à pH 1. Le précipité est filtré, rincé abondamment à l'eau et séché sous vide à 50 °C. Par 3 fois, le solide est trituré dans le pentane, soniqué puis filtré pour donner un solide blanc.
Rendement : 18,8 g (75 %)
RMN ¹H (CDCl₃, ppm) : 0,86 (6H) ; 0,96 (6H) ; 1,12-1,18 (2H) ; 1,20-1,78 (22H) ; 2,24 (2H) ; 4,58-4,63 (1H) ; 5,89 (1H).
LC/MS (ESI): 356,2 ; (calculé ([M+H]⁺): 356,6).

### Molécule 21a : Produit obtenu par la réaction entre la molécule 20 et la Boc-tri(éthylèneglycol)diamine.

À une solution de molécule 20 (16,7 g, 46,97 mmol) dans le THF (235 mL) sont ajoutés de la DIPEA (20,3 mL) et du TBTU à température ambiante. Après 20 min d'agitation, de la Boc-tri(éthylèneglycol)diamine (14 g, 56,36 mmol) est ajoutée. Après agitation à température ambiante pendant 5 h, le mélange est concentré sous vide. Le résidu est repris dans l'acétate d'éthyle (500 mL), lavé avec une solution aqueuse saturée de NaHCO₃ (3 x 200 mL), une solution aqueuse de HCl 1 N (3 x 200 mL) et une solution aqueuse saturée en NaCl (3 x 200 mL). Après séchage sur Na₂SO₄, filtration et concentration sous vide, le résidu est purifié par chromatographie flash (cyclohexane, acétate d'éthyle, méthanol) pour donner une huile incolore.
Rendement : 23,5 g (85 %)
RMN ¹H (CDCl₃, ppm) : 0,86 (6H) ; 0,93 (6H) ; 1,10-1,17 (2H) ; 1,19-1,08 (31H) ; 2,18 (2H) ; 3,23-3,65 (12H) ; 4,41-4,56 (1H) ; 5,12-5,47 (1H) ; 5,99-6,11 (0,75H) ; 6,48-6,65 (1H) ; 7,30-7,40 (0,25H).

### Molécule A9

Par un procédé similaire à celui utilisé pour la préparation de la molécule A5a appliqué à la molécule 21a (23,46 g, 40,04 mmol) sans lavage basique, le résidu obtenu après concentration sous vide est trituré dans un mélange acétonitrile/acétone. Le surnageant est retiré et le résidu pâteux est séché sous vide. Le résidu est ensuite trituré dans de l'acétone (150 mL) et le solide blanc de molécule A9 sous forme de sel de chlorhydrate est filtré, rincé à l'acétone puis séché sous vide.
Rendement : 13,0 g (64 %)
RMN ¹H (DMSO-d6, ppm) : 0,79-0,90 (12H) ; 1,09-1,61 (24H) ; 2,03-2,17 (2H) ; 2,92-2,98 (2H) ; 3,15-3,23 (2H) ; 3,40 (2H) ; 3,50-3,58 (4H) ; 3,61 (2H) ; 4,30-4,23 (1H) ; 7,88-8,14 (5H).
LC/MS (ESI): 486,4 ; (calculé ([M-Cl]⁺): 486,8).

### Exemple A10 : Molécule A10

### Molécule 22a : Produit obtenu par la réaction entre le chlorure d'octanoyle et la L-proline.

Par un procédé similaire à celui utilisé pour la préparation de la molécule 11 et appliqué au chlorure d'octanoyle (150,0 g, 0,922 mol) et à la L-proline (212,3 g, 1,844 mol), une huile incolore de molécule 22a est obtenue après lavages de la phase organique avec une solution aqueuse de HCl 10 % (3 x 300 mL), une solution aqueuse saturée en NaCl (300 mL), séchage sur Na₂SO₄, filtration sur coton, concentration sous pression réduite, puis le résidu est purifié par chromatographie flash (éluant : DCM, MeOH)
Rendement : 134 g (60 %)
RMN ¹H (CDCl₃, ppm) : 0,87 (3H) ; 1,10-1,52 (8H) ; 1,57-1,74 (2H) ; 1,79-2,52 (6H) ; 3,37-3,67 (2H) ; 4,37-4,42 (0,07H) ; 4,53-5,63 (0,93H) ; 9,83 (1H).
LC/MS (ESI) : 242,1 ; (calculé ([M+H]⁺) : 242,2).

### Molécule 23a : Produit obtenu par couplage entre la molécule 22a et la L-lysine.

À une solution de la molécule 22a (132 g, 0,547 mol) dans le THF (924 mL) refroidie à une température inférieure à 5 °C sont ajoutés successivement du NHS (66,1 g, 0,574 mol) et du DCC (118,5 g, 0,574 mol). Après 21 h d'agitation, le précipité est éliminé par précipitation et le filtrat est additionné en 30 min sur une solution de L-lysine (41,98 g, 0,287 mol) dans un mélange d'eau déionisé (82 mL) et de DIPEA (476 mL, 2,735 mol) à 15 °C. Après 23 h d'agitation à température ambiante, le milieu réactionnel est concentré sous pression réduite pour donner un résidu huileux qui est dilué dans de l'eau (1,3 L). La phase aqueuse est lavée deux fois avec de l'AcOEt (2 x 0,5 L), refroidie à une température inférieure à 10 °C, acidifiée par ajout d'une solution de HCl 6 N (120 mL) pour atteindre un pH de 1 puis extraite trois fois avec du DCM (3 x 0,6 L). Les phases organiques sont réunies, lavées avec une solution saturée de NaCl (0,6 L), séchées sur Na₂SO₄ puis concentrées sous pression réduite. La mousse obtenue est reprise dans de l'acétone (240 mL) au reflux pendant 2 h. Après une nuit à 10 °C, du pentane (240 mL) est ajouté goutte-à-goutte. Après 1 h d'agitation, le précipité est récupéré par filtration sous vide, lavé avec un mélange 1:1 de pentane et d'acétone (150mL) puis séché sous vide.
Rendement : 83,9 g (52 %)
RMN ¹H (CDCl₃, ppm) : 0,87 (6H) ; 1,06-1,78 (25H) ; 1,80-2,41 (13H) ; 2,80-3,72 (6H) ; 4,30-4,39 (0,15H) ; 4,46-4,70 (2,85H) ; 7,84 (1H) ; 7,93 (1H).
LC/MS (ESI) : 593,5 ; (calculé ([M+H]⁺) : 593,4).

### Molécule 24 : Produit obtenu par couplage entre la molécule 23a et l'ester méthylique de la L-lysine.

Sur la molécule 23a (76,26 g, 0,129 mol) sont successivement ajoutés du HOPO (3,57 g, 32,1 mmol), de la LysOMe dihydrochloride (15,0 g, 64,3 mmol) et du EDC (34,53 g, 0,18 mol) puis du DMF (600 mL) préalablement refroidie à 5 °C est ajouté. Après dissolution, de la triéthylamine (43,9 mL, 0,315 mol) est ajouté goutte-à-goutte en maintenant la température inférieure à 5 °C pendant encore 2 h après la fin de l'addition. Après une nuit à température ambiante, le milieu réactionnel est versé sur un mélange eau/glace (2 kg) et DCM (0,5 L). Après 15 min d'agitation, les phases sont séparées. La phase aqueuse est extraite deux fois avec du DCM (2 x 0,4 L). Les phases organiques sont réunies, lavées avec une solution de HCl 1 N (0,5 L) puis avec une solution saturée de NaCl (0,5 L), séchées sur Na₂SO₄, concentrées sous pression réduite, puis le résidu est purifié par chromatographie flash (éluant : DCM, MeOH).
Rendement : 56,7 g (67 %)
RMN ¹H (CDCl₃, ppm) : 0,87 (12H) ; 1,10-2,40 (82H) ; 2,86-3,72 (17H) ; 4,16-4,60 (7H) ; 6,83-8,01 (6H).

### Molécule A10

Une solution de molécule 24 (4,0 g, 3,05 mmol) dans de l'éthylènediamine (30 mL) est chauffée à 50 °C pendant une nuit. Le milieu réactionnel est alors dilué avec du méthyl-tétrahydrofurane puis la phase organique est lavée 4 fois avec une solution saturée de NaCl (4 x 30 mL) puis 2 fois avec de l'eau (2 x 50 mL) avant d'être séchée sur Na₂SO₄ puis concentrée sous pression réduite. Le résidu est solubilisé dans de l'acétonitrile au reflux pendant 30 min puis la solution est refroidie à température ambiante sous agitation pendant une nuit. Le précipité blanc est alors récupéré par filtration sous vide, lavé avec de l'acétonitrile froid (2 x 20 mL) puis séché sous vide.
Rendement : 3,0 g (74 %)
RMN ¹H (CDCl₃, ppm) : 0,87 (12H) ; 1,09-2,37 (84H) ; 2,74-4,56 (25H) ; 6,85-8,00 (7H).
LC/MS (ESI) : 1338,0 (calculé ([M+H]⁺) : 1338,0).

### Exemple A11 : Molécule A11

La molécule A11 est obtenue par la méthode conventionnelle de synthèse peptidique en phase solide (SPPS) sur résine 2-chlorotrityle chloride (CTC) (40,0 g, 1,16 mmol/g).
Le greffage du premier acide aminé Fmoc-Lys(Fmoc)-OH (1,5 équivalents) est effectué dans le DCM (10V), en présence de DIPEA (3,0 équivalents). Les sites n'ayant pas réagi sont cappés au méthanol (0,8 mL/g résine) en fin de réaction.
Les couplages des acides aminés protégés Fmoc-Glu(OtBu)-OH (2,5 équivalents), Fmoc-Pro-OH (2,5 équivalents) et de l'acide myristique (2,5 équivalents) sont effectués dans le DMF (10V), en présence de HATU (2,5 équivalents) et de DIPEA (3,7 équivalents). Les groupements protecteurs Fmoc sont retirés à l'aide d'une solution de DMF/pipéridine 80:20 (10 V).
Le produit est clivé de la résine à l'aide d'une solution de DCM/HFIP 80:20 (10 V). Après concentration sous pression réduite, le résidu est purifié par chromatographie sur gel de silice (dichlorométhane, méthanol).
Rendement : 56,5 g (65 %)
RMN ¹H (CD₃OD, ppm) : 0,90 (6H) ; 1,22-2,53 (140H) ; 3,12-3,25 (2H) ; 3,43-3,80 (4H) ; 4,17-4,54 (9H).
LC/MS (ESI+) : 1894,5 (calculé ([M+Na]⁺) : 1894,2).

### Exemple A12 : Molécule A12

### Molécule 25 : Produit obtenu par hydrogénation du farnésol.

À une solution de farnésol (60,00 g, 269,82 mmol) dans le THF (1200 mL) sous argon est ajouté de l'oxyde de platine (PtO₂, 613 mg, 2,70 mmol) et le milieu est placé sous 1 atm de dihydrogène puis agité pendant 6 jours à température ambiante. Après filtration sur célite en rinçant au THF, une huile noire de molécule 25 est obtenue après concentration sous pression réduite. Ce composé est utilisé sans purification supplémentaire.
Rendement : 61,60 g (100%)
RMN ¹H (CDCl₃, ppm) : 0,85 (3H) ; 0,87 (6H) ; 0,90 (3H) ; 1,01-1,43 (15H) ; 1,47-1,66 (3H) ; 3,62-3,76 (2H).

### Molécule 26 : Produit obtenu par oxydation de la molécule 25

À une solution de molécule 25 (61,60 g, 269,68 mmol) dans un mélange dichloroéthane/eau (1350 mL/1080 mL) sont ajoutés successivement du bromure de tétrabutylammonium (46,95 g, 145,63 mmol), de l'acide acétique (416 mL, 7,28 mol) puis du KMnO₄ (127,85 g, 809,04 mmol) par petites fractions en maintenant la température entre 11 et 13 °C. Le milieu réactionnel est ensuite agité pendant 4 h 30 au reflux, refroidi à 0 °C puis acidifié jusqu'à pH 1 avec une solution de HCl 37 % (50 mL). Du Na₂SO₃ (186,94 g) est ajouté progressivement en maintenant la température entre 0 et 10 °C et le milieu est agité jusqu'à décoloration complète. Le milieu est acidifié jusqu'à pH 1 avec une solution de HCl 37 % puis de l'eau (500 mL) et du DCM (500 mL) sont ajoutés. Les phases sont séparées et la phase aqueuse est extraite au DCM (2 x 500 mL). Les phases organiques combinées sont lavées par une solution aqueuse de HCl à 10 % (400 mL), de l'eau (2 x 400 mL), une solution aqueuse saturée en NaCl (400 mL), séchées sur Na₂SO₄, filtrées et concentrées sous pression réduite. Une huile jaune de molécule 26 est obtenue après purification par chromatographie flash (éluant : cyclohexane, AcOEt).
Rendement : 54,79 g (84 %)
RMN ¹H (CDCl₃, ppm) : 0,85 (3H) ; 0,87 (6H) ; 0,97 (3H) ; 1,03-1,43 (13H) ; 1,52 (1H) ; 1,91-2,01 (1H) ; 2,11-2,18 (1H) ; 2,32-2,39 (1H).
LC/MS (ESI-) : 241,3 (calculé ([M-H]⁻) : 241,2).

### Molécule 27 : Produit obtenu par couplage entre la molécule 26 et la L-prolinate de méthyle.

À une solution de molécule 26 (54,70 g, 225,66 mmol) dans du DCM (1500 mL) à 0 °C sont ajoutés successivement du HOBt (3,46 g, 22,57 mmol), de la DIPEA (117,92 mL, 676,97 mmol), le chlorhydrate de L-prolinate de méthyle (56,06 g, 338,49 mmol) puis du EDC (64,89 g, 338,49 mmol). Le mélange réactionnel est agité à 0 °C pendant 1 h puis à température ambiante pendant 18 h. Le milieu est ensuite dilué avec du DCM (1000 mL) puis lavé par une solution aqueuse saturée en NaHCO₃ (2 × 1 L), une solution aqueuse de HCl 1 N (2 x 1000 mL) et une solution aqueuse saturée en NaCl (2 x 1000 mL). La phase organique est séchée sur Na₂SO₄, filtrée et concentrée sous pression réduite pour donner une huile jaune de molécule 27 qui est utilisée sans purification supplémentaire.
Rendement : 77,15 g (97 %)
RMN ¹H (DMSO-d₆, ppm) : 0,79-0,89 (12H) ; 0,98-1,43 (13H) ; 1,51 (1H) ; 1,70-2,32 (7H) ; 3,33-3,42 (0,4H) ; 3,46-3,57 (1,6H) ; 3,59 (2,4H) ; 3,67 (0,6H) ; 4,23-4,32 (0,8H) ; 4,53-4,62 (0,2H).
LC/MS (ESI+) : 354,2 (calculé ([M+H]⁺) : 354,3).

### Molécule 28 : Produit obtenu par la saponification de la molécule 27.

À une solution de molécule 27 (77,15 g, 218,22 mmol) dans un mélange THF/MeOH 1:1 (1454 mL) à 0 °C est ajoutée goutte-à-goutte une solution de LiOH (7,84 g, 327,33 mmol) dans de l'eau (727 mL). Le mélange réactionnel est agité à 0 °C pendant 18 h puis à température ambiante pendant 5 h. Les solvants organiques sont évaporés sous pression réduite. De l'eau (500 mL), une solution aqueuse de HCl à 10 % (200 mL) et du DCM (800 mL) sont ajoutés et les phases sont séparées. La phase aqueuse est extraite par du DCM (2 × 1 L). Les phases organiques réunies sont lavées par de l'eau (500 mL), une solution aqueuse saturée en NaCl (500 mL), séchées sur Na₂SO₄, filtrées et concentrées sous pression réduite pour donner une huile jaune de molécule 28 qui est utilisée sans purification supplémentaire.
Rendement : 71,72 g (97 %)
RMN ¹H (DMSO-d₆, ppm) : 0,73-0,95 (12H) ; 0,95-1,42 (13H) ; 1,51 (1H) ; 1,65-2,32 (7H) ; 3,24-3,64 (2H) ; 4,13-4,28 (0,8H) ; 4,37-4,50 (0,2H) ; 12,44 (1H).
LC/MS (ESI+) : 340,2 (calculé ([M+H]⁺) : 340,3).

### Molécule A12

La molécule A12 est obtenue par la méthode conventionnelle de synthèse peptidique en phase solide (SPPS) sur résine 2-chlorotrityle chloride (CTC) (34,5 g, 1,16 mmol/g).

Le greffage de l'éthylène diamine (10,0 équivalents) est effectué dans le DCM (10V), en présence de DIPEA (10,0 équivalents). Les sites n'ayant pas réagi sont cappés au méthanol (0,8 mL/g résine) en fin de réaction.
Les couplages des acides aminés protégés Fmoc-Lys(Fmoc)-OH (1,5 équivalents), Fmoc-Glu(OMe)-OH (3,0 équivalents) et de la molécule 28 (3,0 équivalents) sont effectués dans un mélange DCM/DMF 1:1 (10V), en présence de HATU (1,0 équivalent par rapport à l'acide) et de DIPEA (2,0 équivalents par rapport à l'acide).
Les groupements protecteurs Fmoc sont retirés à l'aide d'une solution de DMF/pipéridine 80:20 (10 V) (après couplage de la lysine) ou une solution de morpholine à 50 % dans le DMF (après couplage des acides glutamiques).
Le produit est clivé de la résine à l'aide d'une solution de DCM/TFA 50:50 (10 V). Après évaporation, le résidu est solubilisé dans du MeTHF (450 mL) et la phase organique est lavée par une solution aqueuse saturée en NaHCO₃ (3 x 450 mL) puis une solution aqueuse saturée en NaCl (200 mL). Après séchage sur Na₂SO₄, la phase organique est filtrée, concentrée sous pression réduite et le résidu est purifié par chromatographie sur gel de silice (dichlorométhane, méthanol, NH₄OH).
Rendement : 13,95 g (31 % global sur 7 étapes).
RMN ¹H (DMSO-d₆, ppm) : 0,73-0,91 (24H) ; 0,96-2,41 (56H) ; 2,72 (2H) ; 2,89-3,10 (2H) ; 3,15-3,26 (2H) ; 3,26-3,51 (4H) ; 3,57 (3H) ; 3,58 (3H) ; 3,99-4,50 (5H) ; 6,07 (2H) ; 7,59-8,39 (5H).
LC/MS (ESI+) : 1118,2 (calculé ([M+H]⁺) : 1117,8).

### Exemple A13 : Molécule A13

### Molécule 29 : Produit obtenu par polymérisation du γ-benzyl-L-glutamate N-carboxyanhydride initiée par la N-Boc-ethylènediamine.

Dans un réacteur, du γ-benzyl-L-glutamate N-carboxyanhydride (39,44 g, 149,82 mmol) est solubilisé dans du DMF (81 mL) à 25 °C. Le mélange est alors agité jusqu'à complète dissolution, refroidi à -10 °C, puis une solution de BocEDA (6,00 g, 37,45 mmol) dans le DMF (7 mL) est introduite rapidement. Le milieu réactionnel est agité à 0 °C pendant 3 h puis une solution de HCl dans le 1,4-dioxane (3,33 M, 11,8 mL, 39,29 mmol) est ajoutée. Le milieu réactionnel est agité à température ambiante puis coulé sur une solution MeOH/IPE (125 mL/495 mL) refroidie par un bain de glace. Après 65 h d'agitation à température ambiante, le précipité est filtré sur fritté, lavé par de l'IPE (2 x 90 mL) et séché à 30 °C sous pression réduite.
Rendement : 21,71 g (54 %)
DP (estimé d'après la RMN ¹H) : 4,9
La masse molaire moyenne calculée de la molécule 29 sous forme de sel de chlorhydrate est de 1270,9 g/mol.
RMN ¹H (DMSO-d6, ppm) : 1,35 (9H) ; 1,72-2,09 (9,8H) ; 2,23-2,60 (9,8H) ; 2,86-3,19 (4H) ; 3,85 (1H) ; 4,14-4,52 (3,9H) ; 4,86-5,23 (9,8H) ; 6,33-6,85 (1H) ; 7,09-7,55 (24,5H) ; 7,88-8,42 (6,9H) ; 8,67 (1H).

### Molécule 30 : Produit obtenu entre le couplage du chlorure de myristoyle et la molécule 29.

Après solubilisation de la molécule 29 sous forme de sel de chlorhydrate (12,46 g, 9,80 mmol) dans du DCM (115 mL), la solution est refroidie à 0 °C. Puis sont ajoutés successivement de la triéthylamine (2,35 g, 23,24 mmol) et une solution de chlorure de myristoyle (3,16 g, 12,79 mmol) dans le DCM (16 mL). Le milieu réactionnel est agité à 0 °C pendant 4 h puis à température pendant 2 h avant d'être coulé sur de l'IPE (920 mL). Après 14 h d'agitation à température ambiante, le précipité est filtré, lavé par de l'EtOH (2 x 145 mL puis 100 mL) et séché à 30 °C sous pression réduite.
Rendement : 9,77 g (69 %)
DP (estimé d'après la RMN ¹H) : 5,1
La masse molaire moyenne calculée de la molécule 30 est de 1488,7 g/mol.
RMN ¹H (CDCl₃, ppm) : 0,87 (3H) ; 1,07-1,51 (29H) ; 1,51-1,64 (2H) ; 1,80-2,75 (22,4H) ; 2,98-3,73 (4H) ; 3,84-4,50 (5,1H) ; 4,86-5,32 (10,2H) ; 5,71-6,47 (1H) ; 6,72-8,38 (31,6H).

### Molécule A13

À une solution de la molécule 30 (4,70 g, 3,16 mmol) dans du DCM (31 mL) à 0 °C est ajouté du TFA (31 mL). Le milieu réactionnel est agité à 0 °C pendant 2 h puis concentré sous pression réduite et à température ambiante. Le résidu est repris dans du DCM (100 mL) puis concentré à sec sous pression réduite et à température ambiante. Le résidu est solubilisé dans du DCM (100 mL) et lavé par une solution aqueuse de tampon carbonate à pH = 10,4 (326 mL puis 2 × 200 mL) puis par une solution aqueuse de HCl (0,1 N, 2 × 200 mL). La solution organique est séchée sur Na₂SO₄, filtrée puis concentrée à sec à 40 °C sous pression réduite.
Rendement : 3,96 g (88 %)
DP (estimé d'après la RMN ¹H) : 5,2
La masse molaire moyenne calculée de la molécule A13 sous forme de sel de chlorhydrate est de 1446,9 g/mol.
RMN ¹H (TFA-d, ppm) : 0,91 (3H) ; 1,17-1,47 (20H) ; 1,60-1,74 (2H) ; 1,99-2,78 (22,8H) ; 3,41-4,05 (4H) ; 4,62-4,83 (5,2H) ; 5,05-5,35 (10,4H) ; 6,99-8,02 (26H).

### Exemple A15 : Molécule A15

La molécule A15 est obtenue par la méthode conventionnelle de synthèse peptidique en phase solide (SPPS) sur résine 2-chlorotrityle chloride (CTC) (16,0 g, 1,16 mmol/g).

Le greffage de l'éthylène diamine (20,0 équivalents) est effectué dans le DCM (10V). Les sites n'ayant pas réagi sont cappés au méthanol (0,8 mL/g résine) en fin de réaction.

Les couplages des acides aminés protégés Fmoc-Lys(Fmoc)-OH (3,0 équivalents), Fmoc-Glu(OBn)-OH (4,0 équivalents) et de la molécule 11 (3,0 équivalents) sont effectués dans le DMF (10V) (couplages Lys et molécule 11) ou un mélange DCM/DMF 1:1 (10V) (couplage Glu), en présence de HATU (1,0 équivalent par rapport à l'acide) et de DIPEA (1,5 équivalents par rapport à l'acide).

Les groupements protecteurs Fmoc sont retirés à l'aide d'une solution de DMF/pipéridine 80:20 (10 V) (après couplage de la lysine) ou une solution de DBU à 1 % dans le DMF (après couplage des acides glutamiques)

Le produit est clivé de la résine à l'aide d'une solution de DCM/TFA 50:50 (10 V). Après évaporation, le résidu est solubilisé dans de l'acétate d'éthyle (400 mL) et la phase organique est lavée par une solution aqueuse de tampon carbonate à pH 10 (1 M) (2 × 400 mL) puis une solution aqueuse saturée en NaCl (400 mL). Après séchage sur Na₂SO₄, la phase organique est filtrée, concentrée sous pression réduite et le résidu est purifié par chromatographie sur gel de silice (dichlorométhane, méthanol, NH₄OH), puis par une recristallisation dans l'acétonitrile.
Rendement : 16,20 g (70 % global sur 7 étapes).
RMN ¹H (DMSO-d₆, ppm) : 0,85 (6H) ; 1,11-2,57 (72H) ; 2,50-5,57 (2H) ; 2,90-3,08 (4H) ; 3,36-3,61 (4H) ; 4,06-4,43 (5H) ; 5,08 (4H) ; 7,27-7,40 (10H) ; 7,51-8,31 (5H). LC/MS (ESI+) : 1242,0 (calculé ([M+H]⁺) : 1241,9).

### Exemple A16 : Molécule A16

### Molécule 32 : Produit obtenu par SPPS

La molécule 32 est obtenue par la méthode conventionnelle de synthèse peptidique en phase solide (SPPS) sur résine 2-chlorotrityle chloride (CTC) (50,0 g, 1,14 mmol/g).

Le greffage du premier acide aminé Fmoc-Glu(OtBu)-OH (1,3 équivalents) est effectué dans le DCM (10V), en présence de DIPEA (2,6 équivalents). Les sites n'ayant pas réagi sont cappés au méthanol (0,8 mL/g résine) en fin de réaction. Les couplages de l'acide aminé protégé Fmoc-Glu(OtBu)-OH (1,3 équivalents) et de la molécule 11 (3,0 équivalents) sont effectués dans le DMF (10V), en présence de HATU (1,0 équivalent par rapport à l'acide) et de DIPEA (1,5 équivalents par rapport à l'acide). Les groupements protecteurs Fmoc sont retirés à l'aide d'une solution de DMF/pipéridine 80:20 (10 V).

Le produit est clivé de la résine à l'aide d'une solution de DCM/HFIP 80:20 (10 V).

Après concentration sous pression réduite, le résidu est purifié par trituration dans le diisopropyléther.
Rendement : 35,78 g (90 %)
RMN ¹H (CDCl₃, ppm) : 0,88 (3H) ; 1,19-1,35 (20H) ; 1,43 (9H) ; 1,44 (9H) ; 1,55-1,67 (2H) ; 1,90-2,46 (14H) ; 3,46-3,54 (1H) ; 3,63-3,71 (1H) ; 4,33-4,40 (1H) ; 4,43-4,52 (2H) ; 7,35 (0,05H) ; 7,40 (0,05H) ; 7,63 (0,95H) ; 7,94 (0,95H).
LC/MS (ESI+) : 696,4 (calculé ([M+H]⁺) : 696,5).

### Molécule 33 : Produit obtenu par la réaction entre la molécule 32 et la N-CBz éthylènediamine.

Par un procédé similaire à celui utilisé pour la préparation de la molécule 7 et appliqué à la molécule 32 (30,0 g, 43,11 mmol) et au chlorhydrate de N-CBz éthylènediamine (CBzEDA•HCl, 11,93 g, 51,73 mmol), et en présence de DIPEA (15,0 mL, 86,22 mmol), un solide beige de la molécule 33 est obtenu. Il est utilisé sans purification supplémentaire.
Rendement : 37,6 g (100 %)
RMN ¹H (CDCl₃, ppm) : 0,88 (3H) ; 1,19-1,34 (20H) ; 1,42 (9H) ; 1,44 (9H) ; 1,52-2,54 (16H) ; 3,16-3,70 (6H) ; 4,08-4,15 (1H) ; 4,19-4,25 (1H) ; 4,43-4,53 (1H) ; 5,00 (1H) ; 5,08 (1H) ; 6,56 (1H) ; 7,00 (1H) ; 7,24-7,37 (5H) ; 7,59 (1H) ; 8,41 (1H).
LC/MS (ESI+) : 872,5 (calculé ([M+H]⁺) : 872,6).

### Molécule A16

À une solution de molécule 33 (37,6 g, 43,11 mmol) dans du méthanol (376 mL) est ajouté du Pd/Al₂O₃ (3,76 g) sous atmosphère d'argon. Le mélange est placé sous atmosphère d'hydrogène (7 bar) et agité à température ambiante pendant 72 h. Après filtration du catalyseur sur fritté P4 puis sur une membrane Omnipore 0,2 µm PTFE hydrophile, le filtrat est évaporé sous pression réduite pour donner la molécule A16 sous forme d'une huile collante.
Rendement : 31,06 g (98 %)
RMN ¹H (CDCl₃, ppm) : 0,88 (3H) ; 1,19-1,35 (20H) ; 1,43 (9H) ; 1,46 (9H) ; 1,56-1,67 (2H) ; 1,92-2,12 (6H) ; 2,24-2,54 (8H) ; 2,71 (2H) ; 2,90 (2H) ; 3,22-3,32 (1H) ; 3,42-3,51 (1H) ; 3,55-3,64 (1H) ; 3,73-3,81 (1H) ; 4,13-4,21 (1H) ; 4,26-4,33 (1H) ; 4,39-4,48 (1H) ; 7,10 (1H) ; 7,71 (1H) ; 8,45 (1H).
LC/MS (ESI+) : 738,5 (calculé ([M+H]⁺) : 738,5).

### Molécule A17

La molécule A17 est obtenue par la méthode conventionnelle de synthèse peptidique en phase solide (SPPS) sur résine 2-chlorotrityle chloride (CTC) (64,66 g, 1,16 mmol/g).
Le greffage de l'éthylène diamine (10,0 équivalents) est effectué dans le DCM (10V), en présence de DIPEA (10,0 équivalents). Les sites n'ayant pas réagi sont cappés au méthanol (0,8 mL/g résine) en fin de réaction.

Les couplages de l'acide aminé protégé Fmoc-Glu(OMe)-OH (1,5 équivalents) et de la molécule 28 (1,5 équivalents) sont effectués dans un mélange DCM/DMF 1:1 (10V) pour le couplage de l'acide glutamique ou dans du DMF (10V) pour le couplage de la molécule 28, en présence de HATU (1,0 équivalent par rapport à l'acide) et de DIPEA (2,0 équivalents par rapport à l'acide).
Les groupements protecteurs Fmoc sont retirés à l'aide d'une solution de DMF/morpholine 50:50 (10 V).

Le produit est clivé de la résine à l'aide d'une solution de DCM/TFA 50:50 (10 V). Après évaporation, le résidu est solubilisé dans du MeTHF (500 mL) et la phase organique est lavée par une solution aqueuse de Na₂CO₃ à 5 % (3 × 250 mL) puis les phases aqueuses sont extraites au MeTHF (1 × 150 mL). Les phases organiques réunies sont séchées sur Na₂SO₄ et filtrées. Une solution de HCl dans MeOH (1,25 M) est ajoutée puis le milieu est concentré sous pression réduite. Le résidu est purifié par chromatographie sur gel de silice (dichlorométhane, méthanol) pour donner le sel de chlorhydrate de la molécule A17 sous la forme d'un solide brun clair.
Rendement : 12,48 g (30 % global sur 5 étapes).
RMN ¹H (DMSO-d₆, ppm) : 0,76-0,90 (12H) ; 0,97-1,41 (13H) ; 1,45-1,55 (1H) ; 1,68-2,40 (11H) ; 2,77-2,92 (2H) ; 3,20-3,64 (4H) ; 3,57 (3H) ; 4,15-4,49 (2H) ; 7,90-8,48 (5H).
LC/MS (ESI+) : 525,5 (calculé ([M+H]⁺) : 525,4).

### Exemple A18 : Molécule A18

### Molécule 34 : Produit obtenu par hydrogénation du phytol.

À une solution de phytol (260,00 g, 878,78 mmol) dans l'éthanol (1,25 L) sous argon est ajouté du Nickel de Raney à 50 % dans l'eau (30,75 g, 175,36 mmol). Le milieu est placé sous 1 bar de dihydrogène puis agité pendant 8 jours à température ambiante. Après filtration sur un pad de célite/silice/célite en rinçant à l'éthanol, une huile incolore de molécule 34 est obtenue après concentration sous pression réduite.
Rendement : 261,40 g (quant.)
RMN ¹H (CDCl₃, ppm) : 0,84 (6H) ; 0,86 (6H) ; 0,89 (3H) ; 1,00-1,46 (22H) ; 1,46-1,68 (3H) ; 3,61-3,73 (2H).

### Molécule 35 : Produit obtenu par oxydation de la molécule 34.

Par un procédé similaire à celui utilisé pour la préparation de la molécule 26 appliqué à la molécule 34 (29,00 g, 97,13 mmol), une huile jaune de la molécule 35 est obtenue.
Rendement : 28,70 g (94 %)
RMN ¹H (CDCl₃, ppm) : 0,84 (6H) ; 0,86 (6H) ; 0,97 (3H) ; 1,00-1,41 (20H) ; 1,52 (1H) ; 1,96 (1H) ; 2,14 (1H) ; 2,35 (1H) ; 11,31 (1H).
LC/MS (ESI) : 311,1 (calculé ([M-H]⁻) : 311,3).

### Molécule 36 : Produit obtenu par couplage entre la molécule 35 et la L-prolinate de méthyle.

Par un procédé similaire à celui utilisé pour la préparation de la molécule 27 appliqué à la molécule 35 (18,00 g, 57,59 mmol) et au chlorhydrate de L-prolinate de méthyle (14,31 g, 86,39 mmol), une huile jaune de la molécule 36 est obtenue.
Rendement : 23,20 g (95 %)
RMN ¹H (DMSO-d₆, ppm) : 0,78-0,89 (15H) ; 0,97-1,43 (20H) ; 1,43-1,56 (1H) ; 1,70-1,96 (4H) ; 1,96-2,32 (3H) ; 3,33-3,56 (2H) ; 3,59 (0,6H) ; 3,67 (2,4H) ; 4,27 (0,8H) ; 4,57 (0,2H).
LC/MS (ESI) : 424,4 (calculé ([M+H]⁺) : 424,4).

### Molécule 37 : Produit obtenu par la saponification de la molécule 36.

Par un procédé similaire à celui utilisé pour la préparation de la molécule 28 appliqué à la molécule 36 (21,05 g, 49,68 mmol), une huile jaune de la molécule 37 est obtenue.
Rendement : 20,40 g (99 %)
RMN ¹H (DMSO-d₆, ppm) : 0,77-0,91 (15H) ; 0,97-1,43 (20H) ; 1,43-1,56 (1H) ; 1,67-1,96 (4H) ; 1,96-2,29 (3H) ; 3,26-3,56 (2H) ; 4,20 (0,8H) ; 4,41 (0,2H).
LC/MS (ESI) : 410,3 (calculé ([M+H]⁺): 410,4).

### Molécule A18

La molécule A18 est obtenue par la méthode conventionnelle de synthèse peptidique en phase solide (SPPS) sur résine 2-chlorotrityle chloride (CTC) (26,72 g, 1,16 mmol/g).

Par un procédé similaire à celui utilisé pour la préparation de la molécule A17 appliqué au 4,7,10-trioxa-1,13-tridecanediamine (TOTA, 68,30 g, 310,0 mmol), au Fmoc-Glu(OMe)-OH (23,77 mmol, 62,00 mmol) et à la molécule 37 (19,04 g, 46,50 mmol), une huile jaune de molécule A18 sous forme de chlorhydrate est obtenue.
Rendement : 5,53 g (23 % global sur 5 étapes).
RMN ¹H (DMSO-d₆, ppm) : 0,76-0,89 (15H) ; 0,97-2,38 (36H) ; 2,77-2,87 (2H) ; 3,00-3,17 (3H) ; 3,32-3,54 (13H) ; 3,57 (3H) ; 4,09-4,18 (0,75H) ; 4,20-4,29 (1H) ; 4,39-4,47 (0,25H) ; 7,63-8,36 (5H).
LC/MS (ESI+) : 755,7 (calculé ([M+H]⁺) : 755,6).

### Molécule A19

La molécule A19 est synthétisée de la même manière que la molécule A16 en utilisant la molécule 14 à la place de la molécule 11 lors de l'étape de SPPS.
Rendement global (3 étapes) : 32,6 g (81 %)
RMN ¹H (CDCl₃, ppm) : 0,88 (3H) ; 1,20-1,35 (16H) ; 1,43 (9H) ; 1,46 (9H) ; 1,56-1,68 (2H) ; 1,93-2,11 (6H) ; 2,24-2,55 (10H) ; 2,85 (2H) ; 3,19-3,29 (1H) ; 3,38-3,48 (1H) ; 3,55-3,64 (1H) ; 3,74-3,82 (1H) ; 4,14-4,21 (1H) ; 4,25-4,32 (1H) ; 4,41-4,50 (1H) ; 7,03 (1H) ; 7,69 (1H) ; 8,42 (1H).
LC/MS (ESI) : 710,4 (calculé ([M+H]⁺): 710,5).

### Exemple A20 : Molécule A20

La molécule A20 est obtenue par la méthode conventionnelle de synthèse peptidique en phase solide (SPPS) sur résine 2-chlorotrityle chloride (CTC) (40,00 g, 1,16 mmol/g).
Le greffage de l'éthylène diamine (20,0 équivalents) est effectué dans le DCM (10V). Les sites n'ayant pas réagi sont cappés au méthanol (0,8 mL/g résine) en fin de réaction. Les couplages des acides aminés protégés Fmoc-Lys(Fmoc)-OH (1,5 équivalents), Fmoc-Glu(OtBu)-OH (2,5 équivalents) et de la molécule 11 (2,5 équivalents) sont effectués dans le DMF (10 V), en présence de HATU (1,0 équivalent par rapport à l'acide) et de DIPEA (1,5 équivalents par rapport à l'acide).

Les groupements protecteurs Fmoc sont retirés à l'aide d'une solution de DMF/piperidine 80:20 (10 V).

Le produit est clivé de la résine à l'aide d'une solution de DCM/TFA 50:50 (10 V). Après évaporation, le résidu est solubilisé dans de l'eau (600 mL), le pH de la solution est ajusté à 7 par ajout d'une solution de NaOH 5 N, puis le produit est lyophilisé. Le lyophilisat est purifié par colonne de chromatographie sur gel de silice (dichlorométhane, méthanol, NH₄OH) pour donner la molécule A20 sous la forme d'un solide blanc.
Rendement : 24,6 g (50 % global sur 7 étapes).
RMN ¹H (MeOD-d4, ppm) : 0,90 (6H) ; 1,18-2,45 (68H) ; 2,45-2,60 (2H) ; 3,05-3,11 (2H) ; 3,11-3,19 (1H) ; 3,23-3,33 (1H) ; 3,43-3,66 (4H) ; 3,82-3,94 (2H) ; 4,10-4,51 (5H).
LC/MS (ESI+) : 1061,9 (calculé ([M+H]⁺) : 1061,8).

### Partie B - Synthèse des co-polyaminoacides hydrophobes

### i) Co-polyaminoacides de formule XXX, XXXb et XXXa

| **N°** | **CO-POLYAMINOACIDES PORTEUR DE CHARGES CARBOXYLATES ET DE RADICAUX HYDROPHOBE** |
|---|---|
| B1 | 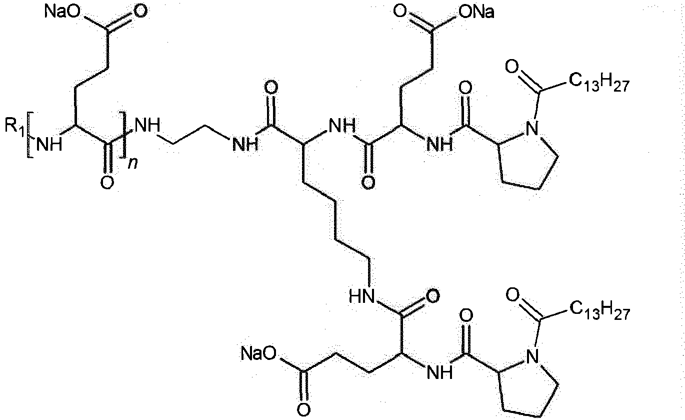 i = 0,038, DP = 26 R₁ = H ou pyroglutamate |
| B2 | 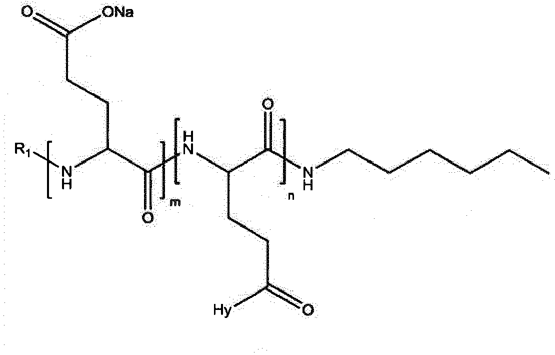 |
| | i= 0,15 , DP (m + n) = 40 Hy = |
| | 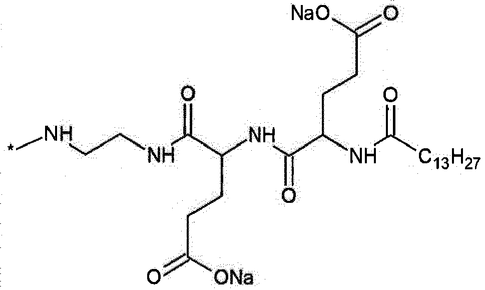 R₁ = H ou pyroglutamate |
| B3 | 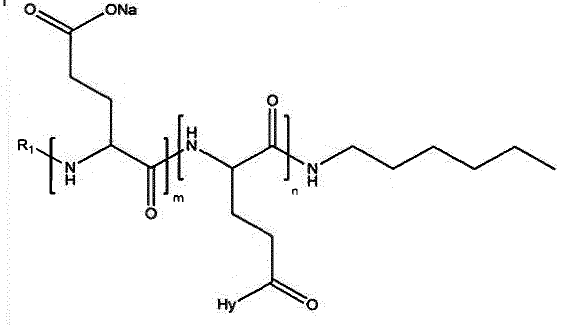 |
| | i= 0,15, DP (m + n) = 40 Hy = |
| | 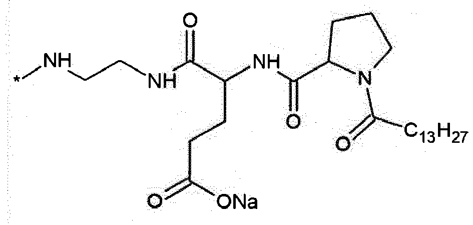 R₁ = H ou pyroglutamate |
| B4 | 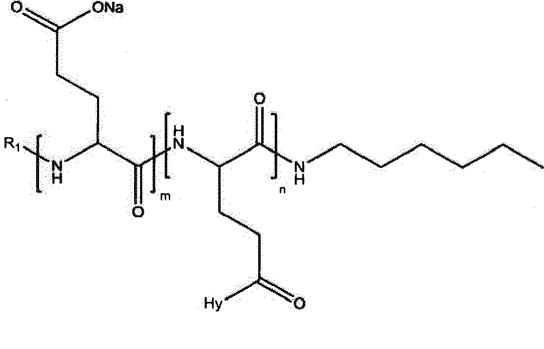 |
| | i= 0,15, DP (m + n) = 40 Hy = |
| | 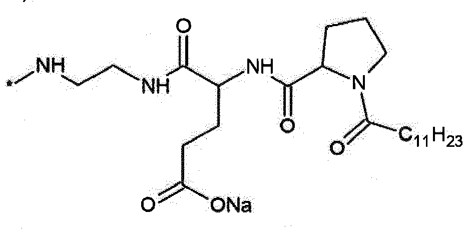 R₁ = H ou pyroglutamate |
| B5 | 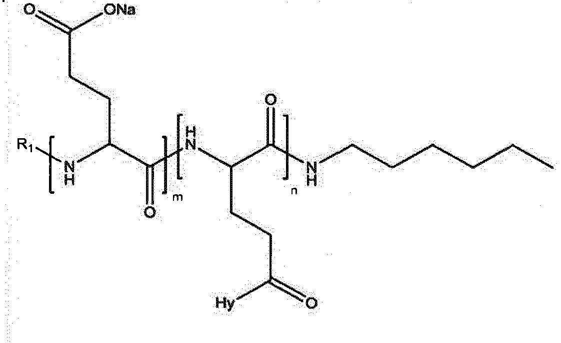 |
| | i= 0,15, DP (m + n) = 40 Hy = |
| | 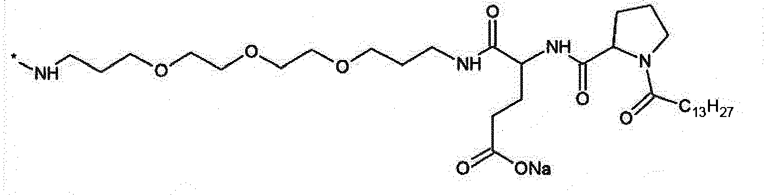 R₁ = H ou pyroglutamate |
| B7 | 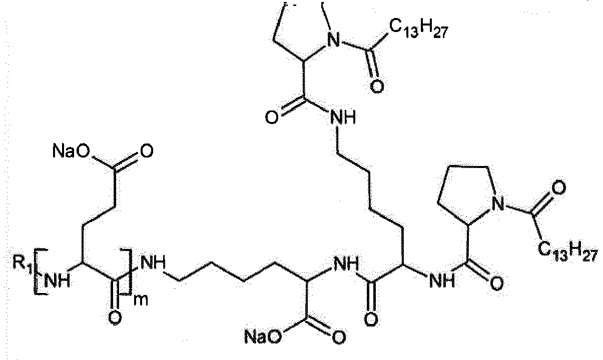 i = 0,038, DP = 26 R₁ = H ou pyroglutamate |
| B13 | 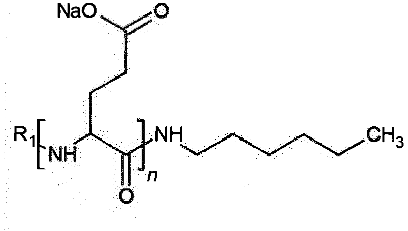 |
| | i = 0,042, DP = 24 R1 = |
| | 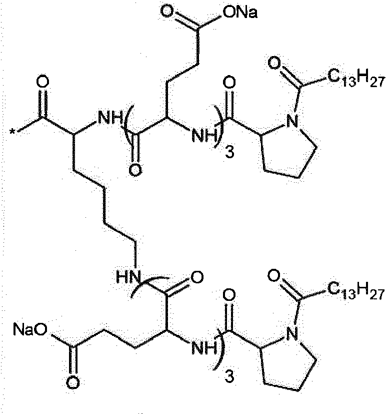 |
| B14 | 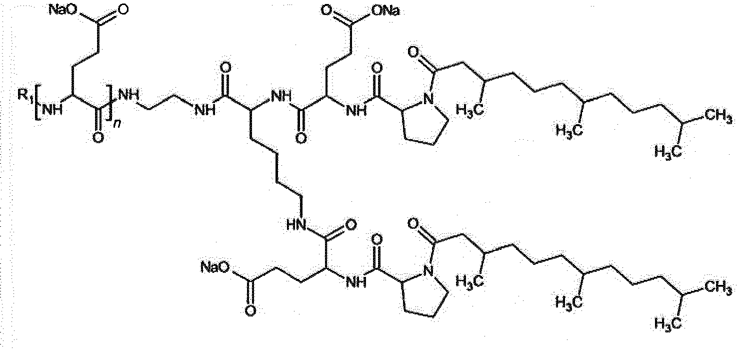 i = 0,042, DP = 24 R₁ = H ou pyroglutamate |
| B15 | 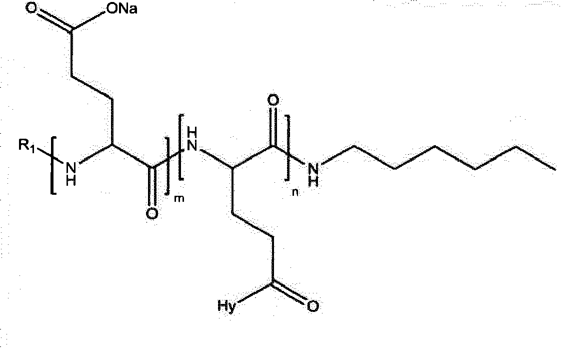 |
| | i= 0,15, DP (m + n) = 40 Hy = |
| | 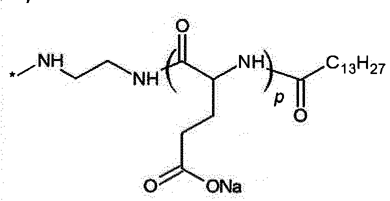 DP (p) = 5,2 R₁ = H ou pyroglutamate |
| B17 | 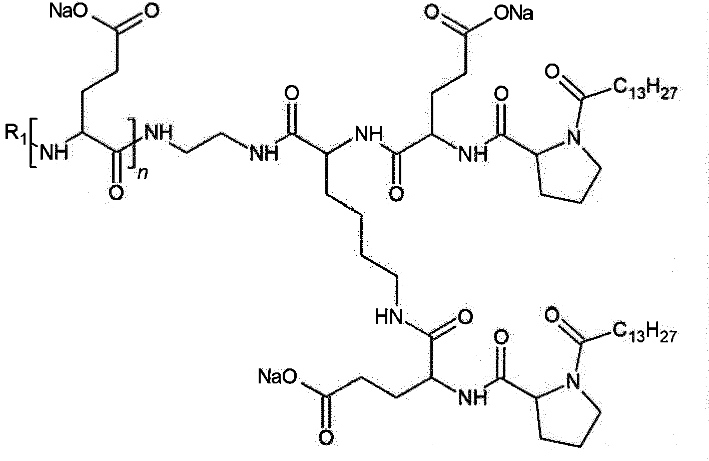 i = 0,1, DP = 10 R₁ = H ou pyroglutamate |
| B18 | 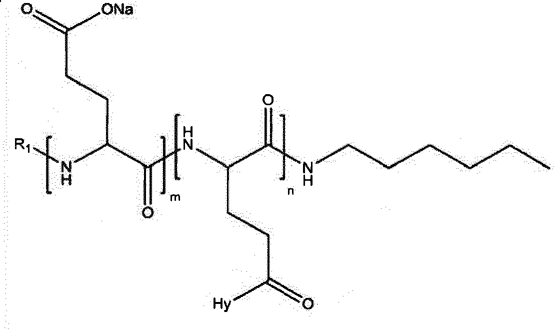 |
| | i= 0,15, DP (m + n) = 40 Hy = |
| | 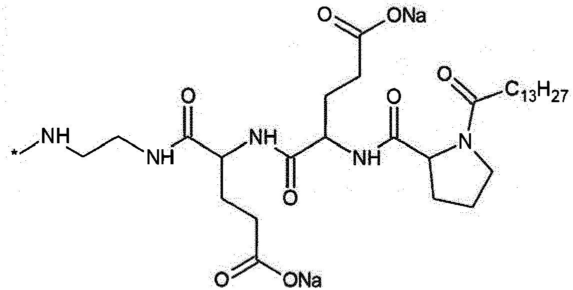 R₁ = H ou pyroglutamate |
| B19 | 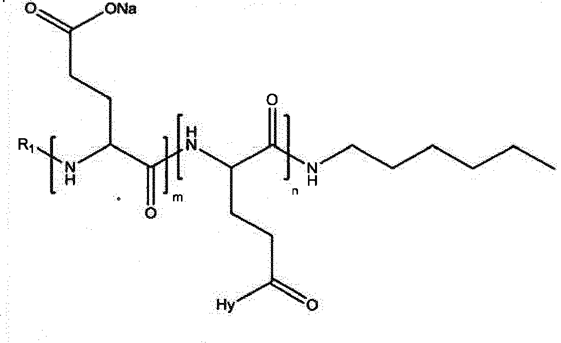 |
| | i= 0,15, DP (m + n) = 40 Hy = |
| | 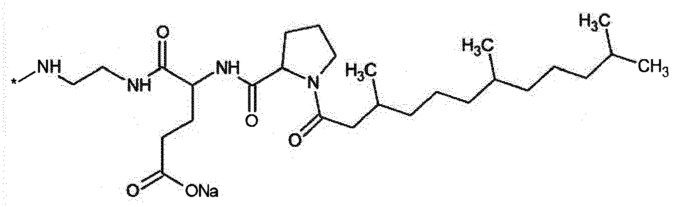 R₁ = H ou pyroglutamate |
| B20 | 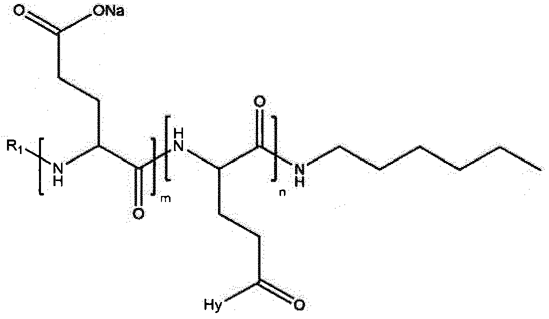 |
| | i= 0,15, DP (m + n) = 40 Hy = |
| | 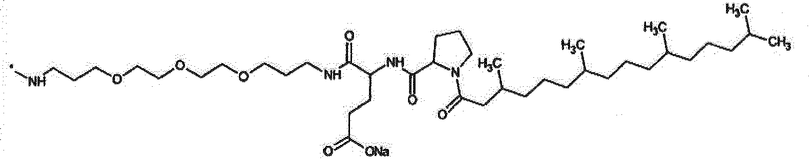 R₁ = H ou pyroglutamate |
| B21 | 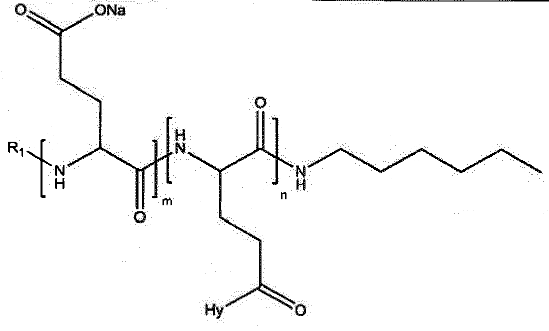 |
| | i= 0,15, DP (m + n) = 40 Hy = |
| | 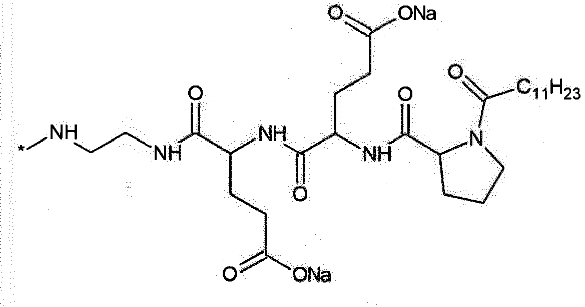 R₁ = H ou pyroglutamate |
| B22 | 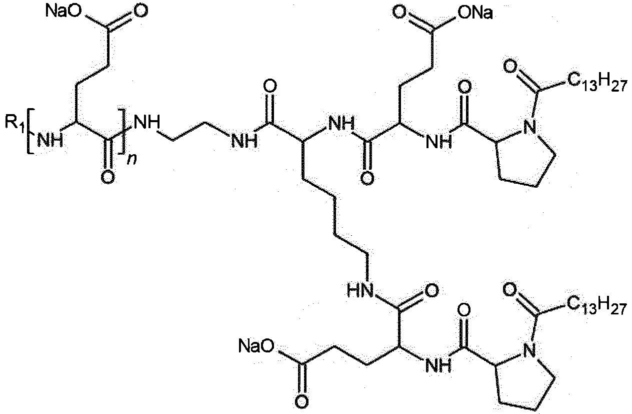 i = 0,05, DP = 20 R₁ = H ou pyroglutamate |

### Co-polyaminoacide B1 : poly-L-glutamate de sodium modifié à une de ses extrémités par la molécule A1 et ayant une masse molaire moyenne en nombre (Mn) de 2800 g/mol

Dans un ballon préalablement séché à l'étuve, du γ-benzyl-L-glutamate *N*-carboxyanhydride (8,95 g, 34 mmol) est solubilisé dans du DMF anhydre (34 mL). Le mélange est refroidi à 4 °C, puis une solution de molécule A1 (1,64 g, 1,55 mmol) dans le chloroforme (6,6 mL) est introduite rapidement. Le mélange est agité entre 4 °C et température ambiante pendant 68 h, puis chauffé à 65 °C pendant 2 h. La moitié du solvant est distillé sous pression réduite puis le milieu réactionnel est refroidi à température ambiante et versé goutte à goutte dans du diisopropyléther (300 mL) sous agitation. Le précipité blanc est récupéré par filtration, lavé avec du diisopropyléther (5 × 50 mL) puis séché sous pression réduite à 30 °C pour obtenir un solide blanc. Le solide (7,9 g) est dilué dans du TFA (30 mL), et une solution d'acide bromhydrique (HBr) à 33 % dans de l'acide acétique (21 mL, 120 mmol) est alors ajoutée au goutte à goutte à 0 °C. La solution est agitée pendant 2 h à température ambiante puis est coulée goutte à goutte sur un mélange 1:1 (v/v) de diisopropyléther/eau sous agitation (360 mL). Après 2 h d'agitation, le mélange hétérogène est laissé au repos pendant une nuit. Le précipité blanc est récupéré par filtration, lavé successivement avec de l'IPE (2 × 30 mL) puis avec de l'eau (2 × 30 mL). Le solide obtenu est solubilisé dans de l'eau (200 mL) en ajustant le pH à 7 par ajout d'une solution aqueuse de soude 1 N. De l'eau (65 mL) est ajoutée. Le mélange est filtré sur filtre 0,45 µm puis purifié par ultrafiltration contre une solution de NaCl 0,9 % puis de l'eau jusqu'à ce que la conductimétrie du perméat soit inférieure à 50 µS/cm. La solution de co-polyaminoacide est ensuite concentrée à environ 25 g/L théorique, le pH est ajusté à 7 et la solution aqueuse est filtrée sur 0,2 µm. Cette solution est diluée avec de l'eau et de l'acétone afin d'obtenir une solution à 12 g/L contenant 30 % massique d'acétone, puis elle est filtrée sur filtre de charbon actif (3M R53SLP). L'acétone est distillée (40 °C, 100 mbar) et la solution est purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 17,8 mg/g
DP (estimé d'après la RMN ¹H) : 26
D'après la RMN ¹H : i = 0,038
La masse molaire moyenne calculée du co-polyaminoacide B1 est de 4994 g/mol. HPLC-SEC organique (calibrant PEG) : Mn = 2800 g/mol

### Co-polyaminoacide B2 : poly-L-glutamate de sodium modifié par la molécule A2 dont les esters sont saponifiés et ayant une masse molaire moyenne en nombre (Mn) de 5200 g/mol

### Co-polyaminoacide B2-1 : acide poly-L-glutamique issu de la polymérisation du γ-benzyl-L-glutamate N-carboxyanhydride initiée par l'hexylamine

Dans un réacteur à double enveloppe, du γ-benzyl-L-glutamate N-carboxyanhydride (500 g, 1,90 mol) est solubilisé dans du DMF anhydre (1100 mL). Le mélange est alors agité jusqu'à complète dissolution, refroidi à 0 °C, puis de l'hexylamine (6,27 mL, 47,5 mmol) est introduite rapidement. Le mélange est agité à 0 °C pendant 5 h, entre 0 °C et 20 °C pendant 7 h, puis à 20 °C pendant 7 h. Le milieu réactionnel est ensuite chauffé à 65 °C pendant 2 h, refroidi à 55 °C et du méthanol (3300 mL) est introduit en 1 h 30. Le mélange réactionnel est alors refroidi à 0 °C et laissé sous agitation pendant 18 h. Le précipité blanc est récupéré par filtration, lavé au diisopropyléther (2 × 800 mL) puis séché sous pression réduite à 30 °C pour donner un acide poly(gamma-benzyl-L-glutamique) (PBLG).

A une solution de PBLG (180 g) dans du *N*,*N*-diméthylacétamide (DMAc, 450 mL) est ajouté du Pd/Al₂O₃ (36 g) sous atmosphère d'argon. Le mélange est placé sous atmosphère d'hydrogène (10 bar) et agité à 60 °C pendant 24 h. Après refroidissement à température ambiante et filtration du catalyseur sur fritté P4 puis sur membrane Omnipore 0,2 µm PTFE hydrophile, une solution d'eau à pH 2 (2700 mL) est coulée goutte à goutte sur la solution de DMAc, sur une période de 45 min et sous agitation. Après 18 h sous agitation, le précipité blanc est récupéré par filtration, lavé avec de l'eau (4 × 225 mL) puis séché sous pression réduite à 30 °C

### Co-polyaminoacide B2

Le co-polyaminoacide B2-1 (15,0 g) est solubilisé dans du DMF (230 mL) à 40 °C puis de la N-méthylmorpholine (NMM, 11,57 g, 114,4 mmol) est ajoutée. En parallèle, la molécule A2 sous forme de sel de chlorhydrate (10,17 g, 17,2 mmol) est mise en suspension dans du DMF (250 mL) et de la triéthylamine (2,39 mL, 17,2 mmol) est ajoutée, puis le mélange est légèrement chauffé sous agitation jusqu'à complète dissolution. À la solution de co-polyaminoacide, refroidie à 25 °C, sont successivement ajoutés la solution de molécule A2, de la *N*-oxyde de 2-hydroxypyridine (HOPO, 3,81 g, 34,3 mmol) puis du EDC (6,58 g, 34,3 mmol). Le milieu réactionnel est agité à 25 °C pendant 2 h, filtré sur filtre tissé 0,2 mm et coulé au goutte-à-goutte sur 2,6 L d'eau contenant du NaCl à 15 % massique et du HCl (pH 2) sous agitation. A la fin de l'ajout, le pH est réajusté à 2 avec une solution de HCl 1 N, et la suspension est laissée reposer une nuit. Le précipité est collecté par filtration, puis rincé par 2 × 100 mL d'eau. Le solide blanc obtenu est solubilisé dans 1,2 L d'eau par ajout lent d'une solution aqueuse de NaOH 1 N jusqu'à pH 7 sous agitation, puis la solution est filtrée sur filtre 0,45 µm. De l'éthanol (30 % massique) est ajouté puis la solution est filtrée sur filtre de charbon actif (3M R53SLP). Une solution de NaOH 10 N est lentement ajoutée sous agitation jusqu'à pH 13 puis le mélange est laissé sous agitation pendant 2 h. Après neutralisation à pH 7 par ajout d'une solution de HCl 37 %, la solution limpide obtenue est purifiée par ultrafiltration contre une solution de NaCl 0,9 % puis de l'eau, jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 22,6 mg/g
DP (estimé d'après la RMN ¹H) : 40
D'après la RMN ¹H : i = 0,15
La masse molaire moyenne calculée du co-polyaminoacide B2 est de 9301 g/mol. HPLC-SEC organique (calibrant PEG) : Mn = 5200 g/mol.

### Co-polyaminoacide B3 : poly-L-glutamate de sodium modifié par la molécule A3 dont l'ester est saponifié et ayant une masse molaire moyenne en nombre (Mn) de 4900 g/mol

Le co-polyaminoacide B2-1 (12,0 g) est solubilisé dans du DMF (92 mL) à 40 °C puis de la N-méthylmorpholine (NMM, 9,25 g, 91,5 mmol) est ajoutée. En parallèle, une solution de la molécule A3 sous forme de sel de chlorhydrate (7,51 g, 13,7 mmol) et de *N*,*N*-diisopropylethylamine (DIPEA, 2,39 mL, 13,7 mmol) dans du DMF (27 mL) est préparée. À la solution de co-polyaminoacide refroidie à 25 °C, sont successivement ajoutés la solution de molécule A3 et de la *N*-oxyde de 2-hydroxypyridine (HOPO, 3,05 g, 27,4 mmol). Le mélange est refroidi à 0 °C puis du EDC (5,26 g, 27,4 mmol) est ajouté. Après 5 min à 0 °C, le milieu réactionnel est agité à 25 °C pendant 2 h, filtré sur filtre tissé 0,2 mm et coulé au goutte-à-goutte sur 950 mL d'eau contenant du NaCl à 15 % massique et du HCl (pH 2) sous agitation. A la fin de l'ajout, le pH est réajusté à 2 avec une solution de HCl 1 N, et la suspension est laissée reposer une nuit. Le précipité est collecté par filtration, puis rincé par 3 × 100 mL d'eau. Le solide obtenu est solubilisé dans 1 L d'eau par ajout lent d'une solution aqueuse de NaOH 1 N jusqu'à pH 7 sous agitation. Une fois la solubilisation complète, le pH est ajusté à pH 12 pendant 2 h puis à pH 13 pendant 1 h par ajout d'une solution de NaOH 10 N. Après neutralisation à pH 7 par ajout d'une solution de HCl 37 %, cette solution est diluée avec de l'eau et de l'éthanol afin d'obtenir une solution à 12 g/L contenant 30 % massique d'éthanol, puis elle est filtrée sur filtre de charbon actif (3M R53SLP). La solution obtenue est filtrée sur 0,45 µm et purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 20,6 mg/g
DP (estimé d'après la RMN ¹H) : 40
D'après la RMN ¹H : i = 0,15
La masse molaire moyenne calculée du co-polyaminoacide B3 est de 8977 g/mol. HPLC-SEC organique (calibrant PEG) : Mn = 4900 g/mol.

### Co-polyaminoacide B4 : poly-L-glutamate de sodium modifié par la molécule A4 dont l'ester est saponifié et ayant une masse molaire moyenne en nombre (Mn) de 4700 g/mol

Par un procédé similaire à celui utilisé pour la préparation du co-polyaminoacide B3 appliqué au sel de chlorhydrate de la molécule A4 (7,12 g, 13,7 mmol) et au co-polyaminoacide B2-1 (12,0 g), un poly-L-glutamate de sodium modifié par la molécule A4 dont l'ester est saponifié est obtenu.
Extrait sec : 19,4 mg/g
DP (estimé d'après la RMN ¹H) : 40
D'après la RMN ¹H : i = 0,15
La masse molaire moyenne calculée du co-polyaminoacide B4 est de 8809 g/mol. HPLC-SEC organique (calibrant PEG) : Mn = 4700 g/mol.

### Co-polyaminoacide B5 : poly-L-glutamate de sodium modifié par la molécule A5 dont l'ester est saponifié et ayant une masse molaire moyenne en nombre (Mn) de 5400 g/mol

Par un procédé similaire à celui utilisé pour la préparation du co-polyaminoacide B3 appliqué au sel de chlorhydrate de la molécule A5 (9,71 g, 13,7 mmol) et au co-polyaminoacide B2-1 (12,0 g), un poly-L-glutamate de sodium modifié par la molécule A5 dont l'ester est saponifié est obtenu.
Extrait sec : 20,8 mg/g
DP (estimé d'après la RMN ¹H) : 40
D'après la RMN ¹H : i = 0,15
La masse molaire moyenne calculée du co-polyaminoacide B5 est de 9939 g/mol. HPLC-SEC organique (calibrant PEG) : Mn = 5400 g/mol.

### Co-polyaminoacide B7 : poly-L-glutamate de sodium modifié à l'une de ses extrémités par la molécule A7 et ayant une masse molaire moyenne en nombre (Mn) de 2500 g/mol

Par un procédé similaire à celui utilisé pour la préparation du co-polyaminoacide B1 appliqué à la molécule A7 (2,50 g, 2,74 mmol) et à du γ-benzyl-L-glutamate *N*-carboxyanhydride (15,89 g, 60,4 mmol), un poly-L-glutamate de sodium modifié à l'une de ses extrémités par la molécule A7 est obtenu.
Extrait sec : 20,3 mg/g
DP (estimé d'après la RMN ¹H) : 26
D'après la RMN ¹H : i = 0,038
La masse molaire moyenne calculée du co-polyaminoacide B7 est de 3893 g/mol. HPLC-SEC organique (calibrant PEG) : Mn = 2500 g/mol

### Co-polyaminoacide B13 : poly-L-glutamate de sodium modifié à l'une de ses extrémités par la molécule A11 dont les esters sont déprotégés et ayant une masse molaire en nombre (Mn) de 3000 g/mol

Dans un réacteur à double enveloppe, du γ-benzyl-L-glutamate N-carboxyanhydride (24,50 g, 93,05 mmol) est solubilisé dans du DMF anhydre (55 mL). Le mélange est alors agité jusqu'à complète dissolution, refroidi à 0 °C, puis de l'hexylamine (0,56 mL, 4,23 mmol) est introduite rapidement. Le mélange est agité à 0 °C pendant 48 h puis sont successivement ajoutés une solution de molécule A11 (9,51 g, 5,08 mmol) dans le DMF (50 mL), HOPO (564 mg, 5,08 mmol) et EDC (973 mg, 5,08 mmol). Le milieu réactionnel est agité à 0 °C pendant 1 h, entre 0 °C et 20 °C pendant 2 h, puis à 20 °C pendant 16 h. Cette solution est ensuite coulée dans un mélange H₂O/MeOH 1:1 (10 V) à température ambiante et sous agitation. Après 4 h sous agitation, le précipité blanc est récupéré par filtration, lavé avec du diisopropyl éther (2 × 100 mL), de l'eau (2 × 100 mL) et un mélange H₂O/MeOH 1:1 (2 × 100 mL) puis séché sous pression réduite.

Le solide obtenu est solubilisé dans du TFA (220 mL) et agité à température ambiante pendant 2 h 30. Cette solution est ensuite coulée dans de l'eau (10 V) à température ambiante et sous agitation. Après 2 h 30 sous agitation, le précipité blanc est récupéré par filtration, lavé avec de l'eau (2 × 200 mL) puis séché sous pression réduite.

Le solide obtenu est solubilisé dans du *N*,*N*-diméthylacétamide (DMAc, 210 mL) puis du Pd/Al₂O₃ (2,1 g) est ajouté sous atmosphère d'argon. Le mélange est placé sous atmosphère d'hydrogène (6 bar) et agité à 60 °C pendant 24 h. Après refroidissement à température ambiante et filtration du catalyseur sur fritté P4 puis sur membrane Omnipore 0,2 µm PTFE hydrophile, une solution d'eau à pH 2 contenant 15 % de NaCl (6 V) est coulée goutte-à-goutte sur la solution de DMAc, sur une période de 45 min et sous agitation. Après 18 h sous agitation, le précipité blanc est récupéré par filtration, lavé avec de l'eau puis séché sous pression réduite. Le solide obtenu est solubilisé dans de l'eau (600 mL) en ajustant le pH à 7 par ajout d'une solution aqueuse de soude 1 N. Le pH est ensuite ajusté à pH 12 et la solution est maintenue sous agitation pendant 1 h. Après neutralisation à pH 7, la solution est filtrée sur 0,2 µm, diluée avec de l'éthanol afin d'obtenir une solution contenant 30 % massique d'éthanol, puis filtrée sur filtre de charbon actif (3M R53SLP). La solution obtenue est filtrée sur 0,45 µm et purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 23,5 mg/g
DP (estimé par RMN ¹H) = 24 donc i = 0,042
La masse molaire moyenne calculée du co-polyaminoacide B13 est de 5377 g/mol. HPLC-SEC organique (calibrant PEG) : Mn = 3000 g/mol.

### Co-polyaminoacide B14 : poly-L-glutamate de sodium modifié à l'une de ses extrémités par la molécule A12 dont les esters sont déprotégés et ayant une masse molaire en nombre (Mn) de 3300 g/mol

### Co-polyaminoacide B14-1 : poly-L-benzylglutamate modifié à l'une de ses extrémités par la molécule A12.

Dans un ballon préalablement séché à l'étuve, du γ-benzyl-L-glutamate *N*-carboxyanhydride (50,00 g, 189,39 mmol) est solubilisé dans du DMF anhydre (65 mL). Le mélange est alors agité jusqu'à complète dissolution, refroidi à 0 °C, puis une solution de la molécule A12 (9,65 g, 8,63 mmol) dans le DMF (50 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (1,8 L) sous agitation. Le précipité blanc est récupéré par filtration, lavé deux fois avec du diisopropyléther puis séché sous vide à 30 °C pour obtenir un solide blanc.

### Co-polyaminoacide B14

Le co-polyaminoacide B14-1 est solubilisé dans du DMAc (250 mL) puis du Pd/Al₂O₃ (5,0 g) est ajouté sous atmosphère d'argon. Le mélange est placé sous atmosphère d'hydrogène (10 bar) et agité à 60 °C pendant 24 h. Après refroidissement à température ambiante et filtration du catalyseur sur fritté P4 puis sur membrane Omnipore 0,2 µm PTFE hydrophile, une solution d'eau à pH 2 (6 V) est coulée goutte-à-goutte sur la solution de DMAc, sur une période de 45 min et sous agitation. Après 18 h sous agitation, le précipité blanc est récupéré par filtration, lavé avec de l'eau puis séché sous pression réduite. Le solide obtenu est solubilisé dans de l'eau (1,25 L) en ajustant le pH à 7 par ajout d'une solution aqueuse de soude 1 N. Le pH est ensuite ajusté à pH 13 et la solution est maintenue sous agitation pendant 3 h. Après neutralisation à pH 7, la solution est filtrée sur 0,2 µm, diluée avec de l'éthanol afin d'obtenir une solution contenant 30 % massique d'éthanol, puis filtrée sur filtre de charbon actif (3M R53SLP). La solution obtenue est filtrée sur 0,45 µm et purifiée par ultrafiltration contre une solution de NaCl 0,9 % puis de l'eau jusqu'à ce que la conductimétrie du perméat soit inférieure à 50 µS/cm. La solution de co-polyaminoacide est ensuite concentrée et le pH est ajusté à 7. La solution aqueuse est filtrée sur 0,2 µm et conservée à 4 °C.
Extrait sec : 25,7 mg/g
DP (estimé par RMN ¹H) = 24 donc i = 0,042
La masse molaire moyenne calculée du co-polyaminoacide B14 est de 4720 g/mol. HPLC-SEC organique (calibrant PEG) : Mn = 3300 g/mol.

### Co-polyaminoacide B15 : poly-L-glutamate de sodium modifié par la molécule A13 dont les esters sont déprotégés et ayant une masse molaire en nombre (Mn) de 4400 g/mol

Par un procédé similaire à celui utilisé pour la préparation du co-polyaminoacide B3 appliqué au sel de chlorhydrate de la molécule A13 (3,39 g, 2,34 mmol) et au co-polyaminoacide B2-1 (2,04 g), avec une étape de saponification à pH 13 pendant 5 h dans un mélange d'eau contenant 30 % massique d'éthanol, un poly-L-glutamate de sodium modifié par la molécule A13 dont les esters sont déprotégés est obtenu.
Extrait sec : 15,7 mg/g
DP (estimé d'après la RMN ¹H) : 40
D'après la RMN ¹H : i = 0,15
La masse molaire moyenne calculée du co-polyaminoacide B15 est de 12207 g/mol. HPLC-SEC organique (calibrant PEG) : Mn = 4400 g/mol.

### Co-polyaminoacide B17 : poly-L-glutamate de sodium modifié à l'une de ses extrémités par la molécule A15 dont les esters sont déprotégés et ayant une masse molaire moyenne en nombre (Mn) de 1000 g/mol

Par un procédé similaire à celui utilisé pour la préparation du co-polyaminoacide B14 appliqué à la molécule A15 (10,85 g, 8,74 mmol) et au γ-benzyl-L-glutamate N-carboxyanhydride (23,00 g, 87,37 mmol), avec une étape de saponification à pH 12 pendant 2 h, un poly-L-glutamate de sodium modifié à l'une de ses extrémités par la molécule A15 dont les esters sont déprotégés est obtenu.
Extrait sec : 23,9 mg/g
DP (estimé d'après la RMN ¹H) : 10
D'après la RMN ¹H : i = 0,1
La masse molaire moyenne calculée du co-polyaminoacide B17 est de 2576 g/mol. HPLC-SEC aqueuse (calibrant PEG) : Mn = 1000 g/mol.

### Co-polyaminoacide B18 : poly-L-glutamate de sodium modifié par la molécule A16 dont les esters sont déprotégés et ayant une masse molaire en nombre (Mn) de 5000 g/mol

Par un procédé de couplage similaire à celui utilisé pour la préparation du co-polyaminoacide B3 appliqué à la molécule A16 (31,06 g, 42,08 mmol) et au co-polyaminoacide B2-1 (36,80 g), un solide beige est obtenu après l'étape de précipitation acide. Ce solide est dilué dans du TFA (100 g/L) et le mélange est agité à température ambiante pendant 3 h. La solution est ensuite coulée goutte à goutte sur de l'eau (3 V) sous agitation. Après 16 h d'agitation, le précipité est récupéré par filtration puis lavé avec de l'eau. Le solide obtenu est solubilisé dans de l'eau en ajustant le pH à 7 par ajout d'une solution aqueuse de soude 10 N. Une fois la solubilisation complète, le pH est ajusté à pH 12 pendant 1 h par ajout d'une solution de NaOH 1 N. Après neutralisation à pH 7 par ajout d'une solution de HCl 1 N, le produit est purifié par un procédé similaire à celui utilisé pour la préparation du co-polyaminoacide B3 (carbofiltration et ultrafiltration). Un poly-L-glutamate de sodium modifié par la molécule A16 dont les esters sont déprotégés est obtenu.
Extrait sec : 28,2 mg/g
DP (estimé d'après la RMN ¹H) : 40
D'après la RMN ¹H : i = 0,15
La masse molaire moyenne calculée du co-polyaminoacide B18 est de 9884 g/mol. HPLC-SEC organique (calibrant PEG) : Mn = 5000 g/mol.

### Co-polyaminoacide B19 : poly-L-glutamate de sodium modifié par la molécule A17 dont les esters sont déprotégés et ayant une masse molaire en nombre (Mn) de 4900 g/mol

Par un procédé similaire à celui utilisé pour la préparation du co-polyaminoacide B3 appliqué au sel de chlorhydrate de la molécule A17 (7,35 g, 13,09 mmol) et au co-polyaminoacide B2-1 (11,45 g), avec une étape de saponification à pH 13 pendant 3 h dans un mélange d'eau contenant 30 % massique d'éthanol, un poly-L-glutamate de sodium modifié par la molécule A17 dont les esters sont déprotégés est obtenu.
Extrait sec : 25,7 mg/g
DP (estimé d'après la RMN ¹H) : 40
D'après la RMN ¹H : i = 0,15
La masse molaire moyenne calculée du co-polyaminoacide B19 est de 9062 g/mol. HPLC-SEC organique (calibrant PEG) : Mn = 4900 g/mol.

### Co-polyaminoacide B20 : poly-L-glutamate de sodium modifié par la molécule A18 dont les esters sont déprotégés et ayant une masse molaire en nombre (Mn) de 5800 g/mol

Par un procédé similaire à celui utilisé pour la préparation du co-polyaminoacide B3 appliqué au sel de chlorhydrate de la molécule A18 (5,43 g, 6,86 mmol) et au co-polyaminoacide B2-1 (6,00 g), avec une étape de saponification à pH 13 pendant 3 h dans un mélange d'eau contenant 30 % massique d'éthanol, un poly-L-glutamate de sodium modifié par la molécule A18 dont les esters sont déprotégés est obtenu.
Extrait sec : 22,0 mg/g
DP (estimé d'après la RMN ¹H) : 40
D'après la RMN ¹H : i = 0,15
La masse molaire moyenne calculée du co-polyaminoacide B20 est de 10444 g/mol. HPLC-SEC organique (calibrant PEG) : Mn = 5800 g/mol.

### Co-polyaminoacide B21 : poly-L-glutamate de sodium modifié par la molécule A19 dont les esters sont déprotégés et ayant une masse molaire en nombre (Mn) de 5000 g/mol

Par un procédé similaire à celui utilisé pour la préparation du co-polyaminoacide B18 appliqué à la molécule A19 (32,64 g, 45,97 mmol) et au co-polyaminoacide B2-1 (40,20 g), un poly-L-glutamate de sodium modifié par la molécule A19 dont les esters sont déprotégés est obtenu.
Extrait sec : 26,2 mg/g
DP (estimé d'après la RMN ¹H) : 40
D'après la RMN ¹H : i = 0,15
La masse molaire moyenne calculée du co-polyaminoacide B21 est de 9716 g/mol. HPLC-SEC organique (calibrant PEG) : Mn = 5000 g/mol.

### Co-polyaminoacide B22 : poly-L-glutamate de sodium modifié à l'une de ses extrémités par la molécule A20 et ayant une masse molaire moyenne en nombre (Mn) de 1900 g/mol

Par un procédé similaire à celui utilisé pour la préparation du co-polyaminoacide B14 appliqué à la molécule A20 (13,28 g, 12,51 mmol) dans du CHCl₃ (53 mL) et au γ-benzyl-L-glutamate *N*-carboxyanhydride (72,46 g, 275,2 mmol) dans du DMF (270 mL), avec une étape de saponification à pH 12 pendant 1 h 30, un poly-L-glutamate de sodium modifié à l'une de ses extrémités par la molécule A20 est obtenu.
Extrait sec : 27,3 mg/g
DP (estimé d'après la RMN ¹H) : 20
D'après la RMN ¹H : i = 0,05
La masse molaire moyenne calculée du co-polyaminoacide B22 est de 4087 g/mol. HPLC-SEC aqueuse (calibrant PEG) : Mn = 1900 g/mol.

### ii) Co-polyaminoacides de formule XXXb

| **N°** | **CO-POLYAMINOACIDES PORTEUR DE CHARGES CARBOXYLATES ET DE RADICAUX HYDROPHOBES** |
|---|---|
| B7' | 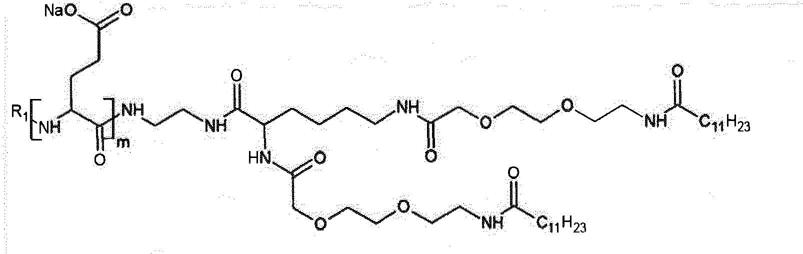 |
| | i = 0,042, DP (m) = 24 |
| | R₁ = H ou pyroglutamate |
| B8 | 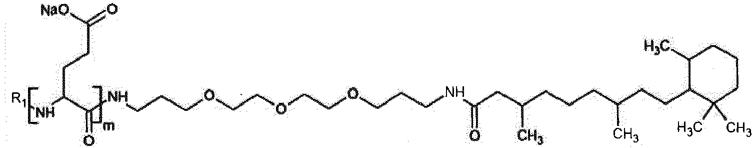 |
| | i = 0,043, DP (m) = 23 |
| | R₁ = H ou pyroglutamate |
| B10 | 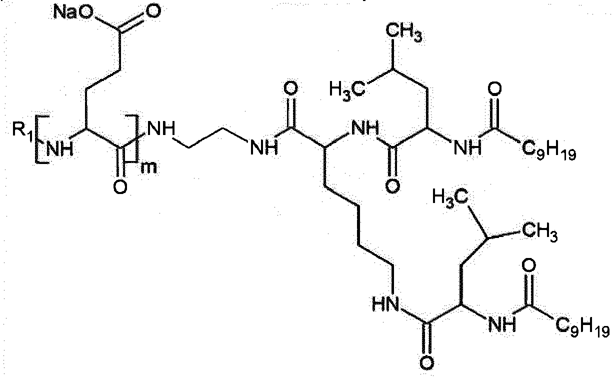 |
| | i = 0,032, DP (m) = 31 |
| | R₁ = H ou pyroglutamate |
| B11 | 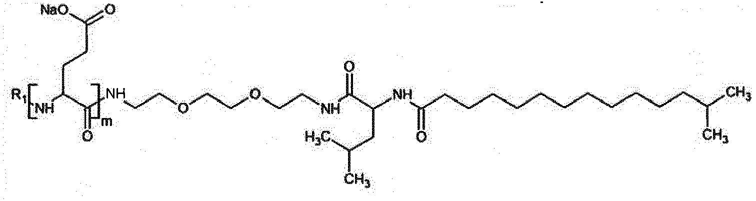 |
| | i = 0,034, DP (m) = 29 |
| | R₁ = H ou pyroglutamate |
| B12 | 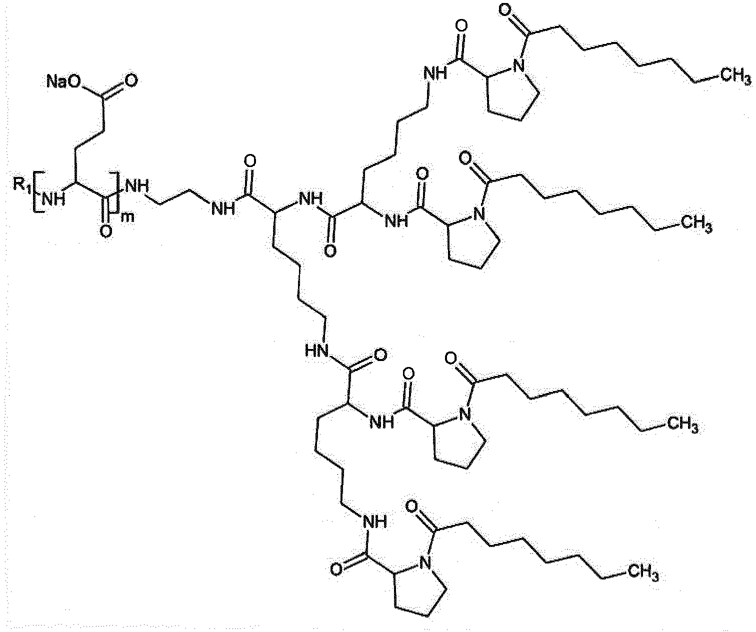 |
| | i = 0,042, DP (m) = 24 |
| | R₁ = H ou pyroglutamate |

### Co-polyaminoacide B7' : poly-L-glutamate de sodium modifié à l'une de ses extrémités par la molécule A5a et ayant une masse molaire moyenne en nombre (Mn) de 2600 g/mol

### Co-polyaminoacide B7'-1 : poly-L-benzylglutamate modifié à l'une de ses extrémités par la molécule A5a.

Dans un ballon préalablement séché à l'étuve, du γ-benzyl-L-glutamate *N*-carboxyanhydride (10,1 g, 38,4 mmol) est solubilisé dans du DMF anhydre (19 mL). Le mélange est alors agité jusqu'à complète dissolution, refroidi à 0 °C, puis une solution de la molécule A5a (1,47 g, 1,74 mmol) dans le chloroforme (3,7 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (0,29 L) sous agitation. Le précipité blanc est récupéré par filtration, lavé deux fois avec du diisopropyléther (5 × 50 mL) puis séché sous vide à 30 °C pour obtenir un solide blanc.

### Co-polyaminoacide B7'

Le co-polyaminoacide B7'-1 (8,33 g, 33,0 mmol) est dilué dans de l'acide trifuloroacétique (TFA, 132 mL), puis la solution est refroidie à 4 °C. Une solution de HBr à 33 % dans l'acide acétique (92,5 mL, 0,528 mol) est alors ajoutée goutte-à-goutte. Le mélange est agité à température ambiante pendant 2 h, puis coulé goutte-à-goutte sur un mélange 1:1 (v/v) de diisopropyléther et d'eau sous agitation (0,8 L). Après 2 h d'agitation, le mélange hétérogène est laissé au repos pendant une nuit. Le précipité blanc est récupéré par filtration, lavé avec de l'IPE (2 × 66 mL) puis avec de l'eau (2 × 66 mL). Le solide obtenu est alors solubilisé dans de l'eau (690 mL) en ajustant le pH à 7 par ajout d'une solution aqueuse de soude 1 N. Après solubilisation, la concentration théorique est ajustée à 20 g/L théorique par addition d'eau (310 mL), la solution est filtrée sur filtre 0,45 µm puis purifiée par ultrafiltration contre une solution de NaCl 0,9 %, puis de l'eau jusqu'à ce que la conductimétrie du perméat soit inférieure à 50 µS/cm. La solution obtenue est filtrée sur filtre 0,2 µm et stockée à 2-8 °C.
Extrait sec : 17,3 mg/g
DP (estimé d'après la RMN ¹H) : 24
D'après la RMN ¹H : i = 0,042
La masse molaire moyenne calculée du co-polyaminoacide B7' est de 4430 g/mol. HPLC-SEC organique (calibrant PEG) : Mn = 2600 g/mol.

### Exemple B8 : co-polyaminoacide B8 - poly-L-glutamate de sodium modifié à l'une de ses extrémités par la molécule A6a et ayant une masse molaire moyenne en nombre (Mn) de 2400 g/mol

### Co-polyaminoacide B8-1 : poly-L-benzylglutamate modifié à l'une de ses extrémités par la molécule A6.

Dans un ballon préalablement séché à l'étuve, du γ-benzyl-L-glutamate *N*-carboxyanhydride (19,0 g, 72,2 mmol) est solubilisé dans du DMF anhydre (19 mL). Le mélange est alors agité jusqu'à complète dissolution, refroidi à 0 °C, puis une solution de la molécule A6a (1,68 g, 3,28 mmol) dans le chloroforme (3,7 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (0,29 L) sous agitation. Le précipité blanc est récupéré par filtration, lavé deux fois avec du diisopropyléther (5 × 50 mL) puis séché sous vide à 30 °C pour obtenir un solide blanc.

### Co-polyaminoacide B8

Par un procédé similaire à celui utilisé pour la préparation du co-polyaminoacide B7' appliqué au co-polyaminoacide B8-1 (14,6 g, 61,5 mmol), un poly-L-glutamate de sodium modifié à l'une de ses extrémités par la molécule A6a est obtenu.
Extrait sec : 21,3 mg/g
DP (estimé d'après la RMN ¹H) : 23
D'après la RMN ¹H : i = 0,043
La masse molaire moyenne calculée du co-polyaminoacide B8 est de 3948 g/mol. HPLC-SEC organique (calibrant PEG) : Mn = 2400 g/mol.

### Co-polyaminoacide B10 : poly-L-glutamate de sodium modifié à l'une de ses extrémités par la molécule A8 et ayant une masse molaire moyenne en nombre (Mn) de 3100 g/mol

### Co-polyaminoacide B10-1 : poly-L-benzylglutamate modifié à l'une de ses extrémités par la molécule A8.

Dans un contenant adapté sont introduits successivement le sel de chlorhydrate de la molécule A8 (2,308 g, 3,04 mmol), du chloroforme (120 mL), du tamis moléculaire 4 Å (1,5 g), ainsi que de la résine échangeuse d'ion Amberlite IRN 150 (1,5 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (40 mL) pour être utilisé directement dans la réaction de polymérisation.

Dans un ballon préalablement séché à l'étuve, du γ-benzyl-L-glutamate *N*-carboxyanhydride (20,0 g, 76,0 mmol) est solubilisé dans du DMF anhydre (19 mL). Le mélange est alors agité jusqu'à complète dissolution, refroidi à 0 °C, puis une solution de la molécule A8, préalablement préparée, dans le chloroforme (3,7 mL) est introduite rapidement. Le mélange est agité entre 0 °C et température ambiante pendant 2 jours, puis chauffé à 65 °C pendant 2 h. Le mélange réactionnel est alors refroidi à température ambiante puis versé goutte-à-goutte dans du diisopropyléther (0,29 L) sous agitation. Le précipité blanc est récupéré par filtration, lavé deux fois avec du diisopropyléther (5 × 50 mL) puis séché sous vide à 30 °C pour obtenir un solide blanc.

### Co-polyaminoacide B10

Par un procédé similaire à celui utilisé pour la préparation du co-polyaminoacide B7' appliqué au co-polyaminoacide B10-1 (15,2 g, 60,8 mmol), un poly-L-glutamate de sodium modifié à l'une de ses extrémités par la molécule A8 est obtenu.
Extrait sec : 34,1 mg/g
DP (estimé d'après la RMN ¹H) : 31
D'après la RMN ¹H : i = 0,032
La masse molaire moyenne calculée du co-polyaminoacide B10 est de 5367 g/mol. HPLC-SEC organique (calibrant PEG) : Mn = 3100 g/mol.

### Exemple B11 : co-polyaminoacide B11 - poly-L-glutamate de sodium modifié à l'une de ses extrémités par la molécule A9 et ayant une masse molaire moyenne en nombre (Mn) de 3000 g/mol

### Co-polyaminoacide B11-1 : poly-L-benzylglutamate modifié à l'une de ses extrémités par la molécule A9.

Dans un contenant adapté sont introduits successivement le sel de chlorhydrate de la molécule A9 (2,023 g, 3,87 mmol), du chloroforme (120 mL), du tamis moléculaire 4 Å (1,5 g), ainsi que de la résine échangeuse d'ion Amberlite IRN 150 (1,5 g). Après 1 h d'agitation sur rouleaux, le milieu est filtré et la résine est rincée avec du chloroforme. Le mélange est évaporé puis co-évaporé avec du toluène. Le résidu est solubilisé dans du DMF anhydre (40 mL) pour être utilisé directement dans la réaction de polymérisation.

Par un procédé similaire à celui utilisé pour la préparation du co-polyaminoacide B8-1 appliqué à la solution de la molécule A9 préalablement préparée et au γ-benzyl-L-glutamate *N*-carboxyanhydride (25,5 g, 96,8 mmol), le co-polyaminoacide B11-1 est obtenu.

### Co-polyaminoacide 811

Par un procédé similaire à celui utilisé pour la préparation du co-polyaminoacide B7' appliqué au co-polyaminoacide B11-1 (18,4 g, 77,3 mmol), un poly-L-glutamate de sodium modifié à l'une de ses extrémités par la molécule A9 est obtenu.
Extrait sec : 28,0 mg/g
DP (estimé d'après la RMN ¹H) : 29
D'après la RMN ¹H : i = 0,034
La masse molaire moyenne calculée du co-polyaminoacide B11 est de 4828 g/mol. HPLC-SEC organique (calibrant PEG) : Mn = 3000 g/mol.

### Co-polyaminoacide B12 : poly-L-glutamate de sodium modifié à l'une de ses extrémités par la molécule A10 et ayant une masse molaire moyenne en nombre (Mn) de 2700 g/mol

### Co-polyaminoacide B12-1 : poly-L-benzylglutamate modifié à l'une de ses extrémités par la molécule A10.

Par un procédé similaire à celui utilisé pour la préparation du co-polyaminoacide B10-1 appliqué à la molécule A10 (3,0 g, 2,24 mmol) et au γ-benzyl-L-glutamate *N*-carboxyanhydride (12,99 g, 49,3 mmol), le co-polyaminoacide B12-1 est obtenu.

### Co-polyaminoacide B12

Par un procédé similaire à celui utilisé pour la préparation du co-polyaminoacide B7' appliqué au co-polyaminoacide B12-1 (13,2 g, 48,0 mmol), un poly-L-glutamate de sodium modifié à l'une de ses extrémités par la molécule A10 est obtenu.
Extrait sec : 13,2 mg/g
DP (estimé d'après la RMN ¹H) : 24
D'après la RMN ¹H : i = 0,042
La masse molaire moyenne calculée du co-polyaminoacide B12 est de 4924 g/mol. HPLC-SEC organique (calibrant PEG) : Mn = 2700 g/mol.

### PARTIE C : COMPOSITIONS

Le glucagon utilisé est du glucagon humain issu d'un processus de synthèse peptidique. Il provient de la société Bachem (référence 4074733).

### Exemple C1 : Solution de Glucagon à 2 mq/mL

Dans un tube Falcon de 50 mL est introduit 94,7 mg de glucagon DS en poudre suivi de 45 mL d'une solution d'acide chlorhydrique à 0,003 N contenant 2 mg/mL de L-méthionine. La poudre de glucagon est mélangée par des inversions répétées du tube jusqu'à complète dissolution du glucagon. La solution de glucagon à 2 mg/mL est alors filtrée sur membrane (0,22 µm).

### Exemple C2 : Solution de Glucagon à 4 mg/ml

Du glucagon (160 mg) en poudre est introduit dans un tube falcon de 45 ml puis 40 mL de la solution aqueuse d'acide chlorhydrique à 0,006 N contenant 2 mg/mL de L-méthionine est ajoutée. La poudre de glucagon est mélangée par des inversions répétées du tube jusqu'à complète dissolution du glucagon. La solution de glucagon à 4 mg/ml est alors filtrée sur membrane (0,22 µm).

### Exemple CA0 : Préparation d'une solution de glucagon à 1 mg/ml et contenant différents co-polyaminoacides de l'invention, un tampon phosphate (2 mm) et de la glycérine à pH 7,2.

Dans un flacon contenant des solutions concentrées d'excipients (phosphate, glycérol (pour obtenir 300 mOsmole/kg dans la formulation finale)) et potentiellement des additifs (m-crésol, citrate), est ajoutée une solution de co-polyaminoacide. La composition est brièvement agitée jusqu'à dissolution du co-polyaminoacide, puis la solution est filtrée sur membrane (0,22 µm).
Le mélange équivolumique de cette solution avec la solution de glucagon fraichement préparée, telle que décrite à l'exemple C1, conduit aux compositions finales CA1 à CA32 et CA15' et CA26' contenant 1 mg/mL de glucagon. Le pH de la solution est ajusté à pH 7,2 ± 0,1 par ajout de NaOH/HCl 1 N puis filtrée sur membrane (0,22µm). Le détail des compositions est récapitulé dans les tableaux ci-dessous.

Une inspection visuelle est effectuée pour déterminer si l'on obtient ou non une solution limpide (Par comparaison, la solution de glucagon à pH neutre n'est pas soluble au-delà de 0,2 mg/mL). L'inspection visuelle des échantillons est effectuée afin de détecter les particules visibles, ou une turbidité. Cette inspection est réalisée selon les recommandations de la Pharmacopée Européenne (EP 2.9.20) : les échantillons sont soumis à un éclairage d'au moins 2000 Lux et sont observés face à un fond blanc et un fond noir. Quand des particules sont visibles dans la moitié des échantillons la composition est estimée non limpide.

Les résultats sont présentés dans les Tableaux 1 et 1a : Compositions et aspect visuel des solutions de glucagon à 1 mg/mL à pH 7,2 à différentes concentrations en co-polyaminoacide contenant 2 mM de tampon phosphate et 1 mg/mL de L-méthionine.

**Tableau 1 : Compositions et aspect visuel des solutions de glucagon à 1 mg/mL à pH 7,2 à différentes concentrations en co-polyaminoacide contenant 2 mM de tampon phosphate et 1 mg/mL de L-méthionine.**

| **Composition** | **Co-polyaminoacide** | **Concentration de co-polyaminoacides (mg/mL)** | **Ratio molaire co-polyaminoacide/gluac gon** | **Additifs** | **Glycérol (mM)** | **Aspect visuel de la solution** |
|---|---|---|---|---|---|---|
| CA1 | B3 | 3,43 | 1,33 | | 290 | limpide |
| CA2 | | 4,02 | 1,56 | | 290 | limpide |
| CA3 | | 3,61 | 1,4 | 10 mM citrate | 250 | limpide |
| CA4 | | 4,13 | 1,6 | 10 mM citrate | 250 | limpide |
| CA5 | B2 | 6,95 | 2,6 | 10 mM citrate | 250 | limpide |
| CA6 | | 7,75 | 2,9 | 10 mM citrate | 250 | limpide |
| CA7 | | 7,75 | 2,9 | | 294 | limpide |
| CA8 | | 8,55 | 3,2 | | 294 | limpide |
| CA9 | | 9,62 | 3,6 | | 294 | limpide |
| CA10 | B1 | 4,3 | 3 | 27 mM m-crésol | 259 | limpide |
| CA11 | | 7,2 | 5 | 27 mM m-crésol | 253 | limpide |
| CA11C | | 2,61 | 2 | 10 mM citrate | 260 | limpide |
| CA11D | | 2,87 | 2,2 | 10 mM citrate | 260 | limpide |
| CA11E | | 4,95 | 3,8 | 10 mM citrate | 260 | limpide |
| CA11F | | 2,61 | 2 | | 290 | limpide |
| CA11G | | 2,87 | 2,2 | | 290 | limpide |
| CA12 | B3 | 2,58 | 1 | | 290 | limpide |
| CA13 | | 3,09 | 1,2 | | 290 | limpide |
| CA14 | | 2,58 | 1 | 10 mM citrate | 260 | limpide |
| CA15 | | 3,09 | 1,2 | 10 mM citrate | 260 | limpide |
| CA16 | B2 | 2,67 | 1 | 10 mM citrate | 260 | limpide |
| CA17 | | 3,20 | 1,2 | 10 mM citrate | 260 | limpide |
| CA18 | | 2,14 | 0,8 | | 300 | limpide |
| CA19 | | 2,67 | 1 | | 300 | limpide |
| CA20 | | 3,20 | 1,2 | | 300 | limpide |
| CA21 | B5 | 6,28 | 2,2 | 10 mM citrate | 260 | limpide |
| CA22 | | 7,13 | 2,5 | 10 mM citrate | 260 | limpide |
| CA23 | B20 | 4,27 | 1,5 | 10 mM citrate | 260 | limpide |
| CA24 | | 5,70 | 2,0 | 10 mM citrate | 260 | limpide |
| CA25 | B14 | 3,79 | 2,8 | 10 mM citrate | 248 | limpide |
| CA26 | | 4,06 | 3,0 | 10 mM citrate | 248 | limpide |
| CA27 | B19 | 3,12 | 1,2 | 10 mM citrate | 249 | limpide |
| CA28 | | 3,64 | 1,4 | 10 mM citrate | 249 | limpide |
| CA29 | B17 | 2,22 | 3,2 | 10 mM citrate | 249 | limpide |
| CA30 | | 2,37 | 3,0 | 10 mM citrate | 249 | limpide |
| CA31 | B18 | 3,46 | 1,2 | 10 mM citrate | 260 | limpide |
| CA32 | | 4,03 | 1,4 | 10 mM citrate | 260 | limpide |

**Tableau 1a : Compositions et aspect visuel des solutions de glucagon à 1 mg/mL à pH 7,2 à différentes concentrations en co-polyaminoacide contenant du tampon phosphate (2 mM) et 1 mg/mL de L-méthionine.**

| **Composition** | **Copolyaminoacide** | **Concentration de copolyaminoacide (mg/mL)** | **Ratio Copolyamino-acide/ glucagon** | **Additifs** | **Glycérine (mM)** | **Aspect visuel de la solution** |
|---|---|---|---|---|---|---|
| CA15' | B11 | 20,2 | 14,6 | 23 mM m-crésol | 230 | limpide |
| CA26' | B8 | 2,50 | 2,2 | | 292 | limpide |

### Les compositions formulées ci-dessus sont limpides, alors que le glucagon formulé dans ces conditions, sans co-polyaminoacide, n'est pas soluble.

### Exemple CB0 : Préparation d'une solution de co-polyaminoacide et de glucagon a 2 mg/ml à pM 7,2

De manière analogue à l'exemple CA0, des compositions de glucagon à 2 mg/mL contenant différents co-polyaminoacides, du glycérol (pour obtenir 300 mOsmol/kg dans la formulation finale), un tampon phosphate (2 mM) et des additifs sont préparées. Elles sont présentées dans le tableau 1b suivant :

**Tableau 1b : Compositions et aspect visuel des solutions de glucagon à 2 mg/mL à pH 7,2 à différentes concentrations en co-polyaminoacide contenant 2 mM de tampon phosphate et 1 mg/mL de L-méthionine.**

| **Composition** | **copolyaminoacide** | **Concentration de copolyaminoacides (mg/mL)** | **Ratio copolyaminoacide/ glucagon** | **Additif** | **glycérol (mM)** | **Aspect visuel de la solution** |
|---|---|---|---|---|---|---|
| CB1 | B3 | 6,9 | 1,33 | | 290 | limpide |
| CB2 | | 10,3 | 2 | 10 mM citrate | 250 | limpide |
| CB3 | B2 | 16 | 3 | 10 mM citrate | 250 | limpide |
| CB4 | | 16 | 3 | | 294 | limpide |

### Stabilité physique des compositions

Les compositions précédemment préparées ont été transférées dans des cartouches (easy-to-fill de OMPI de 3 ml - Ref P40B4100.3250) à raison de 1 mL par cartouche et placées en conditions statiques à 37 °C.

L'inspection visuelle des échantillons placés en conditions statiques à 37 °C est effectuée à 0, 1, 2, 3, 4, 5, 6 semaines à 37 °C afin de détecter l'apparition de particules visibles, de fibrilles ou d'une turbidité. Cette inspection est réalisée selon les recommandations de la Pharmacopée Européenne (EP 2.9.20) : les échantillons sont soumis à un éclairage d'au moins 2000 Lux et sont observés face à un fond blanc et un fond noir pour respecter les recommandations de la pharmacopée Européenne. Quand des particules sont visibles dans la moitié des échantillons la composition est estimée non stable. Stable signifie donc qu'au jour de l'inspection au moins la moitié des échantillons étaient dépourvus de particules, de fibrilles ou d'une turbidité.

Les résultats des inspections visuelles sont reportés dans le tableau suivant.
L'étude des stabilités physiques des compositions décrites dans le tableau ci-dessous a été menée sur des volumes de 1 mL de composition dans des cartouches de contenance de 3 mL (OMPI - ref : P40B4100.3250). Par comparaison, la solution de glucagon à pH acide à 1 mg/mL n'est stable que 2 jours à 37 °C.

**Tableau 2 : Stabilité physique à 37 °C de compositions comprenant B1, B2 ou B3 en cartouche**

| **Composition** | **co-polyaminoacide** | **Concentration de co-polyaminoacides (mg/mL)** | **Additif** | **Stabilité (semaine)** |
|---|---|---|---|---|
| CA2 | B3 | 4,02 | | >2 |
| | | | | >5 |
| CA3 | | 3,61 | 10 mM citrate | >2 |
| | | | | >4 |
| CA4 | | 4,13 | 10 mM citrate | >2 |
| | | | | >20 |
| CA5 | B2 | 6,95 | 10 mM citrate | >2 |
| CA6 | | 7,75 | 10 mM citrate | >2 |
| CA7 | | 7,75 | | >2 |
| CA8 | | 8,55 | | >2 |
| CA9 | | 9,62 | | >2 |
| CA11E | B1 | 3,8 | 10 mM citrate | >10 |

La composition CA11 a été transféré dans un vial de 3 mL (Adelphi - réf :VCDIN2RDLS1) à raison de 1 mL par vial et placées en conditions statiques à 37 °C. Les résultats des inspections visuelles sont reportés dans le tableau suivant.

**Tableau 3 : Stabilité physique à 37 °C de la composition B1 en vial.**

| **Composition** | **co-polyaminoacide** | **Concentration de co-polyaminoacides (mg/mL)** | **Additif** | **Stabilité à 37 °C (semaine)** |
|---|---|---|---|---|
| CA11 | B1 | 7,2 | 27 mM m-crésol | >2 |
| | | | | >6 |

Les solutions selon l'invention présentent une stabilité physique à 37 °C en conditions statiques en cartouche supérieure à deux semaines à 37 °C. L'addition de co-polyaminoacide B1 permet de solubiliser et de stabiliser le glucagon à pH neutre alors que le glucagon en solution a pH acide n'est stable que quelques jours à 37 °C (2 jours).

### RESULTATS DES OBSERVATIONS VISUELLES AU MELANGE ET DES MESURES DE FIBRILLATION PAR THT

Les compositions précédemment préparées ont été aliquoté dans une plaque 96 puits en triplicat (3*150µL) et placées en conditions statiques à 37 °C.

### Principe

La mauvaise stabilité d'un peptide peut conduire à la formation de fibrilles amyloïdes, définies comme des structures macromoléculaires ordonnées. Celles-ci peuvent éventuellement résulter à la formation de gel au sein de l'échantillon.

L'essai de suivi de la fluorescence de la Thioflavine T (ThT) est utilisé pour analyser la stabilité physique des solutions. La thioflavine est une petite molécule sonde ayant une signature de fluorescence caractéristique lorsqu'elle se lie à des fibrilles de type amyloïdes (Naiki et al. (1989) Anal. BioChem. 177, 244-249 ; LeVine (1999) Methods. Enzymol. 309, 274-284).

Cette méthode permet de suivre la formation de fibrilles pour de faibles concentrations de ThT au sein de solutions non diluées. Ce suivi est réalisé dans des conditions de stabilité accélérées : sous agitation et à 37 °C.

### Conditions expérimentales

Les échantillons ont été préparés juste avant le début de la mesure. La préparation de chaque composition est décrite dans l'exemple associé. La Thioflavine T a été ajoutée dans la composition à partir d'une solution mère concentrée de manière à induire une dilution négligeable de la composition. La concentration de Thioflavine T dans la composition est de 40 µM.

Un volume de 150 µL de la composition a été introduit au sein d'un puit d'une plaque 96 puits puis 2,7 µL de solution concentrée de ThT a été introduite. Chaque composition a été analysée en trois essais (triplicat) au sein d'une même plaque. La plaque a été scellée par du film transparent afin d'éviter l'évaporation de la composition. Cette plaque a ensuite été placée dans l'enceinte d'un lecteur de plaques (Xenius XC, SAFAS). La température est réglée à 37 °C, et une agitation latérale de 960 rpm avec 1 mm d'amplitude est imposée.

Une lecture de l'intensité de fluorescence dans chaque puit est réalisée avec une longueur d'onde d'excitation de 442 nm, et une longueur d'onde d'émission de 482 nm au cours du temps.

Le processus de fibrillation se manifeste par une forte augmentation de la fluorescence après un délai appelé temps de latence.

Le lag time est déterminé graphiquement, en prenant le temps où la tangente à la phase linéaire de croissance coupe l'axe des abscisses.
La valeur de temps de latence reportée correspond à la moyenne des mesures de temps de latence faites sur trois puits.

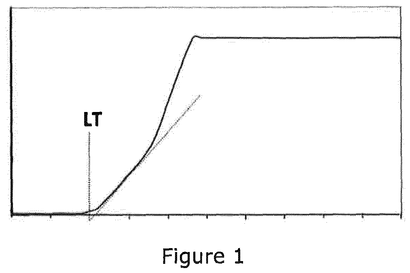

Un exemple de détermination graphique est représenté à la figure 1.

Sur cette figure est représentée de façon graphique la détermination du temps de latence ou « lag time » (LT) par suivi de la fluorescence de la Thioflavine T, sur une courbe ayant en ordonnées la valeur de la fluorescence (en u.a. unités arbitraires) et en abscisses le temps en minutes.

Les résultats de temps de latence obtenus sont présentés dans le tableau ci-dessous. Par comparaison, le glucagon seul est insoluble en solution à pH physiologique et la solution de glucagon à pH acide à 1 mg/mL montre un temps de fibrillation de 0,5 h environ.

**Tableau 4 : Mesure du temps de latence des compositions CA11D à CA32.**

| **Composition** | **co-polyaminoacide** | **Concentration de co-polyaminoacides (mg/mL)** | **Additif** | **Temps de fibrillation (h)** |
|---|---|---|---|---|
| CA11D | | 2,87 | 10 mM citrate | >10 |
| CA11G | | 2,87 | - | 2<t<4 |
| CA12 | B3 | 2,58 | - | >60 |
| CA13 | | 3,09 | - | >90 |
| CA14 | | 2,58 | 10 mM citrate | >60* |
| CA15 | | 3,09 | 10 mM citrate | >60* |
| CA16 | B2 | 2,67 | 10 mM citrate | >10* |
| CA17 | | 3,20 | 10 mM citrate | >20 |
| CA18 | | 2,14 | - | >9 |
| CA19 | | 2,67 | - | >10 |
| CA20 | | 3,20 | - | >10 |
| CA21 | B5 | 6,28 | 10 mM citrate | >50 |
| CA22 | | 7,13 | 10 mM citrate | >70 |
| CA23 | B20 | 4,27 | 10 mM citrate | >60 |
| CA24 | | 5,70 | 10 mM citrate | >70 |
| CA26 | B14 | 4,06 | 10 mM citrate | >10 |
| CA28 | B19 | 3,64 | 10 mM citrate | >15 |
| CA30 | B17 | 2,37 | 10 mM citrate | >15 |
| CA31 | B18 | 3,46 | 10 mM citrate | >10 |
| CA32 | | 4,03 | 10 mM citrate | >20 |

| | | | | |
|---|---|---|---|---|
| *Les essais ont été arrêtés avant qu'il y est fibrillation | | | | |

Les compositions contenant des co-polyaminoacides permettent d'augmenter considérablement le temps de latence par rapport à la solution de glucagon seul à pH acide qui n'est stable que quelques minutes dans ces conditions de mesures.

## Revendications

1. Composition sous forme d'une solution aqueuse injectable, dont le pH est compris entre 6,0 et 8,0, comprenant au moins :
a) du glucagon humain ;
b) un co-polyaminoacide porteur de charges carboxylates et de radicaux hydrophobes Hy, ledit co-polyaminoacide étant constitué d'unités glutamiques ou aspartiques et lesdits radicaux hydrophobes Hy étant de formule X suivante : 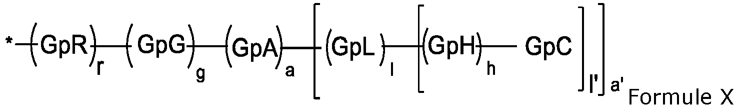 dans laquelle
- GpR est choisi parmi les radicaux de formules VII, VII' ou VII" : 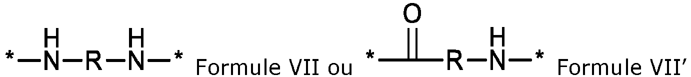 ou 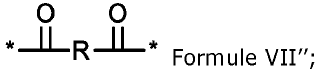
- GpG et GpH identiques ou différents sont choisis parmi les radicaux de formules XI ou XI': 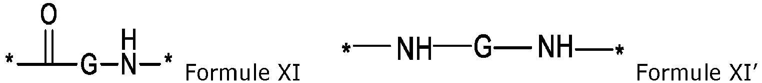
- GpA est choisi parmi les radicaux de formule VIII 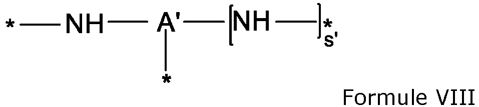 dans laquelle A' est choisi parmi les radicaux de formule VIII',VIII" ou VIII‴ 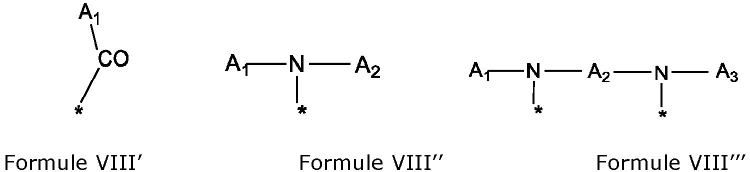
- -GpL est choisi parmi les radicaux de formule XII 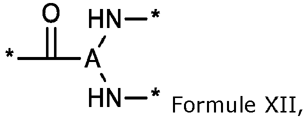
- GpC est un radical de formule IX : 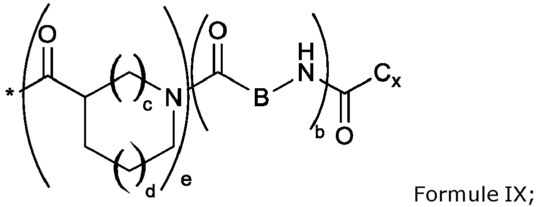
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3 ;
- b est un entier égal à 0 ou à 1 ;
- c est un entier égal à 0 ou à 1, et si c est égal à 0 alors d est égal à 1 ou à 2 ;
- d est un entier égal à 0, à 1 ou à 2 ;
- e est un entier égal à 0 ou à 1 ;
- g est un entier égal à 0, à 1, à 2, à 3, à 4, à 5 ou à 6 ;
- h est un entier égal à 0, à 1, à 2, à 3, à 4, à 5 ou à 6 ;
- l est un entier égal à 0 ou 1 et l' = 1 si l = 0 et l' = 2 si l = 1 ;
- r est un entier égal à 0, 1 ou à 2 ;
- s' est un entier égal à 0 ou 1 ;
- et si e est différent de 0 alors au moins un des g, h ou l est différent de 0 ;
- et si a = 0 alors l = 0 ;
- A, A₁, A₂ et A₃ identiques ou différents sont des radicaux alkyles linéaires ou ramifiés comprenant de 1 à 8 atomes de carbone et éventuellement substitué par un radical issu d'un cycle saturé, insaturé ou aromatique ;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone ;
- Cₓ est un radical alkyl monovalent linéaire ou ramifié, éventuellement comprenant une partie cyclique, dans lequel x indique le nombre d'atomes de carbone et :
• Lorsque le radical hydrophobe -Hy porte 1 -GpC, alors 9 ≤ x ≤ 25,
• Lorsque le radical hydrophobe -Hy porte 2 -GpC, alors 9 ≤ x ≤ 15,
• Lorsque le radical hydrophobe -Hy porte 3 -GpC, alors 7 ≤ x ≤ 13,
• Lorsque le radical hydrophobe -Hy porte 4 -GpC, alors 7 ≤ x ≤ 11,
• Lorsque le radical hydrophobe -Hy porte au moins 5 -GpC alors, 6 ≤ x ≤ 11 ;
- G est un radical alkyle ramifié de 1 à 8 atomes de carbone ledit radical alkyle portant une ou plusieurs fonction(s) acide carboxylique libre ;
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions
- CONH₂ ou un radical éther ou polyéther non substitué comprenant de 4 à 14 atomes de carbone et de 1 à 5 atomes d'oxygène ;
- le ou les radicaux hydrophobes -Hy de formule X étant liés au PLG:
∘ via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur Hy' du radical hydrophobe -Hy, et
∘ via une liaison covalente entre un atome d'azote du radical hydrophobe
- Hy et un carbonyle porté par le PLG formant ainsi une fonction amide issue de la réaction d'une fonction amine du précurseur Hy' du radical hydrophobe -Hy et une fonction acide portée par le PLG ;
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents ;
- le degré de polymérisation DP en unités glutamiques ou aspartiques pour les chaines PLG est compris entre 5 et 250 ;
- les fonctions acides carboxyliques libres étant sous forme de sel de cation alcalin choisi dans le groupe constitué par Na⁺ et K⁺.

2. Composition selon la revendication 1, **caractérisée en ce que** le co-polyaminoacide porteur de charges carboxylates et de radicaux hydrophobes est choisi parmi les co-polyaminoacides de formule XXX suivante : 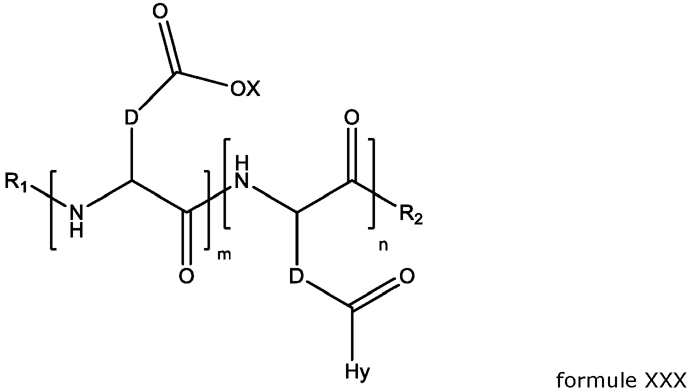 dans laquelle,
• D représente, indépendamment, soit un groupe -CH₂- (unité aspartique) soit un groupe -CH₂-CH₂- (unité glutamique),
• Hy est un radical hydrophobe choisi parmi les radicaux hydrophobes de formules X, dans lesquelles r = 1 et GpR est un radical de Formule VII,
• R₁ est un radical hydrophobe choisi parmi les radicaux hydrophobes de formules X dans lesquelles r=0 ou r=1 et GpR est un radical de Formule VII', ou un radical choisi dans le groupe constitué par un H, un groupe acyle linéaire en C2 à C10, un groupe acyle ramifié en C3 à C10, un benzyle, une unité « acide aminé » terminale et un pyroglutamate,
• R₂ est un radical hydrophobe choisi parmi les radicaux hydrophobes de formules X dans lesquelles r = 1 et GpR est un radical de Formule VII, ou un radical -NR'R", R' et R" identiques ou différents étant choisis dans le groupe constitué par H, les alkyles linéaires ou ramifiés ou cycliques en C2 à C10, le benzyle et lesdits R' et R" alkyles pouvant former ensemble un ou des cycles carbonés saturés, insaturés et/ou aromatiques et/ou pouvant comporter des hétéroatomes, choisis dans le groupe constitué par O, N et S,
• X représente un H ou une entité cationique choisie dans le groupe comprenant les cations métalliques ;
• n + m représente le degré de polymérisation DP du co-polyaminoacide, c'est-à-dire le nombre moyen d'unités monomériques par chaîne de co-polyaminoacide et 5 ≤ n + m ≤ 250 ;
• n + m représente le degré de polymérisation DP du co-polyaminoacide, c'est-à-dire le nombre moyen d'unités monomériques par chaîne de co-polyaminoacide et 5 ≤ n + m ≤ 250.

3. Composition selon l'une quelconque des revendications précédentes, **caractérisée en ce que** le co-polyaminoacide porteur de charges carboxylates et de radicaux hydrophobes est choisi parmi les co-polyaminoacides de formule XXX dans laquelle n = 0 de formule XXXb suivante : 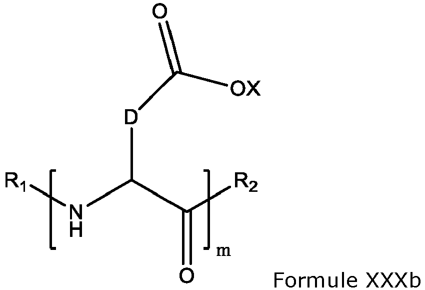 dans laquelle m, X, D, R₁ et R₂ ont les définitions données précédemment et au moins R₁ ou R₂ est un radical hydrophobe de formule X.

4. Composition selon la revendication 3, **caractérisée en ce que** le co-polyaminoacide porteur de charges carboxylates et de radicaux hydrophobes est choisi parmi les co-polyaminoacides de formule XXXb dans laquelle R₂ est un radical hydrophobe de formule X dans lesquelles r = 1 et GpR est de Formule VII'.

5. Composition selon l'une quelconque des revendications 1 à 4, **caractérisée en ce que** le co-polyaminoacide porteur de charges carboxylates et de radicaux hydrophobes est choisi parmi les co-polyaminoacides de formules XXX, XXXb dans lesquels le au moins un co-polyaminoacide est choisi parmi les co-polyaminoacides dans lesquels le groupe D est un groupe -CH₂- (unité aspartique).

6. Composition selon l'une quelconque des revendications 1 à 4, **caractérisée en ce que** le co-polyaminoacide porteur de charges carboxylates et de radicaux hydrophobes est choisi parmi les co-polyaminoacides de formules XXX, XXXb dans lesquels le au moins un co-polyaminoacide est choisi parmi les co-polyaminoacides dans lesquels le groupe D est un groupe -CH₂-CH₂- (unité glutamique).

7. Composition selon l'une quelconque des revendications précédentes, **caractérisée en ce que** la concentration en co-polyaminoacide porteur de charges carboxylates et de radicaux hydrophobes est au plus de 40 mg/mL.

8. Composition selon l'une quelconque des revendications précédentes, **caractérisée en ce que** la concentration en glucagon humain est comprise entre 0,25 et 5 mg/mL.

9. Composition selon l'une quelconque des revendications précédentes, **caractérisée en ce que** le ratio molaire [radical hydrophobe]/[glucagon humain] est inférieur à 15.

10. Composition selon l'une quelconque des revendications précédentes, **caractérisée en ce qu'**elle comprend en outre un composé polyanionique.

11. Composition selon l'une quelconque des revendications précédentes, **caractérisée en ce qu'**elle comprend en outre un sel de zinc.

12. Composition selon l'une quelconque des revendications précédentes, **caractérisée en ce qu'**elle comprend en outre une hormone gastrointestinale.

13. Co-polyaminoacide porteur de charges carboxylates et de radicaux hydrophobes Hy, choisi parmi les radicaux de formule X telle que définie ci-dessous : 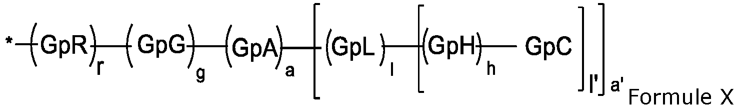 dans laquelle
- GpR est choisi parmi les radicaux de formules VII, VII' ou VII" : 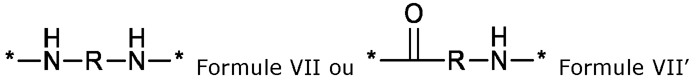 ou 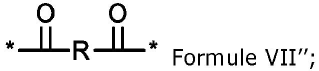
- GpG et GpH identiques ou différents sont choisis parmi les radicaux de formules XI ou XI': 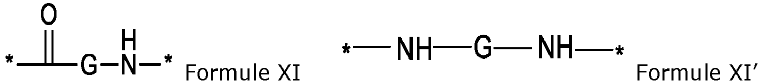
- GpA est choisi parmi les radicaux de formule VIII 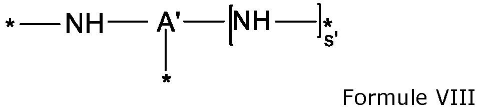 Dans laquelle A' est choisi parmi les radicaux de formule VIII',VIII" ou VIII‴ 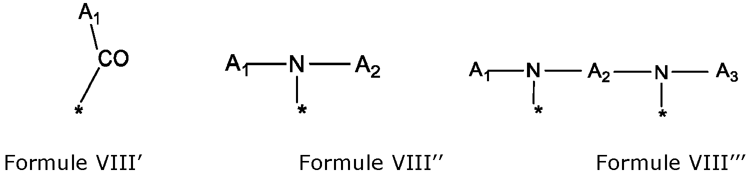
- GpL est choisi parmi les radicaux de formule XII 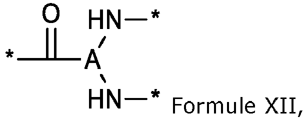
- GpC est un radical de formule IX : 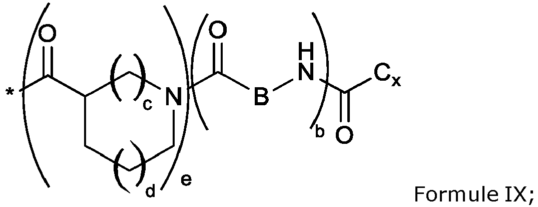
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3 ;
- b est un entier égal à 0 ou à 1 ;
- c est un entier égal à 0 ou à 1, et si c est égal à 0 alors d est égal à 1 ou à 2 ;
- d est un entier égal à 0, à 1 ou à 2 ;
- e est un entier égal à 0 ou à 1 ;
- g est un entier égal à 0, à 1, à 2, à 3, à 4, à 5 ou à 6 ;
- h est un entier égal à 0, à 1, à 2, à 3, à 4, à 5 ou à 6 ;
- l est un entier égal à 0 ou 1 et l' = 1 si l = 0 et l' = 2 si l = 1 ;
- r est un entier égal à 0, 1 ou à 2 ;
- s' est un entier égal à 0 ou 1 ;
- et si e est différent de 0 alors au moins un des g, h ou l est différent de 0 ;
- et si a = 0 alors l = 0 ;
- A, A₁, A₂ et A₃ identiques ou différents sont des radicaux alkyles linéaires ou ramifiés comprenant de 1 à 8 atomes de carbone et éventuellement substitué par un radical issu d'un cycle saturé, insaturé ou aromatique ;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone ;
- Cₓ est un radical alkyl monovalent linéaire ou ramifié, éventuellement comprenant une partie cyclique, dans lequel x indique le nombre d'atomes de carbone et :
• Lorsque le radical hydrophobe -Hy porte 1 -GpC, alors 9 ≤ x ≤ 25,
• Lorsque le radical hydrophobe -Hy porte 2 -GpC, alors 9 ≤ x ≤ 15,
• Lorsque le radical hydrophobe -Hy porte 3 -GpC, alors 7 ≤ x ≤ 13,
• Lorsque le radical hydrophobe -Hy porte 4 -GpC, alors 7 ≤ x ≤ 11,
• Lorsque le radical hydrophobe -Hy porte au moins 5 -GpC alors, 6 ≤ x ≤ 11 ;
- G est un radical alkyle ramifié de 1 à 8 atomes de carbone ledit radical alkyle portant une ou plusieurs fonction(s) acide carboxylique libre ;
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions
- CONH₂ ou un radical éther ou polyéther non substitué comprenant de 4 à 14 atomes de carbone et de 1 à 5 atomes d'oxygène ;
- le ou les radicaux hydrophobes -Hy de formule X étant liés au PLG:
o via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur Hy' du radical hydrophobe -Hy, et
∘ via une liaison covalente entre un atome d'azote du radical hydrophobe
- Hy et un carbonyle porté par le PLG formant ainsi une fonction amide issue de la réaction d'une fonction amine du précurseur Hy' du radical hydrophobe -Hy et une fonction acide portée par le PLG ;
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents ;
- le degré de polymérisation DP en unités glutamiques ou aspartiques pour les chaines PLG est compris entre 5 et 250 ;
- les fonctions acides carboxyliques libres étant sous forme de sel de cation alcalin choisi dans le groupe constitué par Na⁺ et K⁺.

14. Précurseur Hy' du radical hydrophobe -Hy de formule X' : 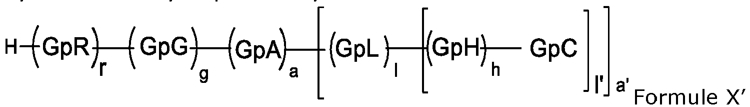 dans laquelle
- GpR est choisi parmi les radicaux de formules VII, VII' ou VII" : 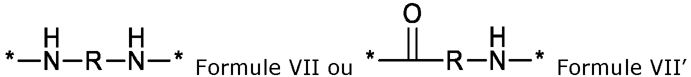 ou 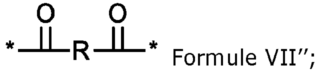
- GpG et GpH identiques ou différents sont choisis parmi les radicaux de formules XI ou XI': 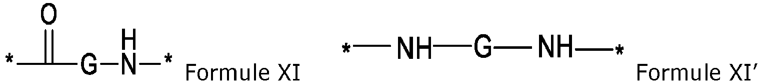
- GpA est choisi parmi les radicaux de formule VIII 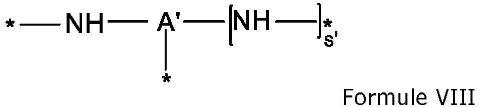 Dans laquelle A' est choisi parmi les radicaux de formule VIII',VIII" ou VIII'" 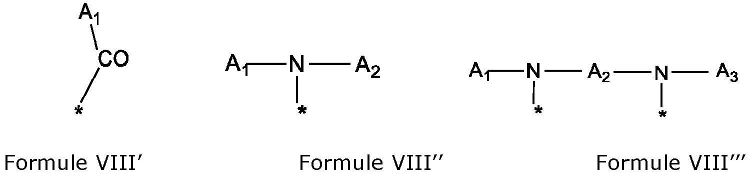
- GpL est choisi parmi les radicaux de formule XII 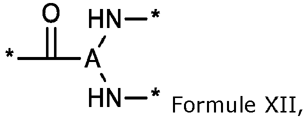
- GpC est un radical de formule IX : 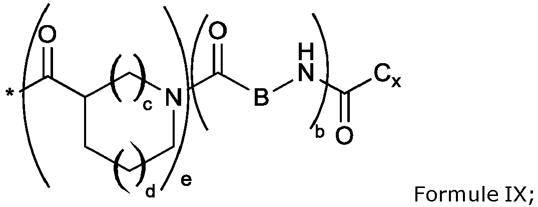
- les * indiquent les sites de rattachement des différents groupes liés par des fonctions amides ;
- a est un entier égal à 0 ou à 1 et a' = 1 si a = 0 et a' = 1, 2 ou 3 si a = 1 ;
- a' est un entier égal à 1, à 2 ou à 3 ;
- b est un entier égal à 0 ou à 1 ;
- c est un entier égal à 0 ou à 1, et si c est égal à 0 alors d est égal à 1 ou à 2 ;
- d est un entier égal à 0, à 1 ou à 2 ;
- e est un entier égal à 0 ou à 1 ;
- g est un entier égal à 0, à 1, à 2, à 3, à 4, à 5 ou à 6 ;
- h est un entier égal à 0, à 1, à 2, à 3, à 4, à 5 ou à 6 ;
- l est un entier égal à 0 ou 1 et l' = 1 si l = 0 et l' = 2 si l = 1 ;
- r est un entier égal à 0, 1 ou à 2 ;
- s'est un entier égal à 0 ou 1 ;
- et si e est différent de 0 alors au moins un des g, h ou l est différent de 0 ;
- et si a = 0 alors l = 0 ;
- A, A₁, A₂ et A₃ identiques ou différents sont des radicaux alkyles linéaires ou ramifiés comprenant de 1 à 8 atomes de carbone et éventuellement substitué par un radical issu d'un cycle saturé, insaturé ou aromatique ;
- B est un radical éther ou polyéther non substitué comprenant de 4 à 14 atomes de carbone et de 1 à 5 atomes d'oxygène ou un radical alkyle linéaire ou ramifié, éventuellement comprenant un noyau aromatique, comprenant de 1 à 9 atomes de carbone ;
- Cₓ est un radical alkyl monovalent linéaire ou ramifié, éventuellement comprenant une partie cyclique, dans lequel x indique le nombre d'atomes de carbone et :
• Lorsque le radical hydrophobe -Hy porte 1 -GpC, alors 9 ≤ x ≤ 25,
• Lorsque le radical hydrophobe -Hy porte 2 -GpC, alors 9 ≤ x ≤ 15,
• Lorsque le radical hydrophobe -Hy porte 3 -GpC, alors 7 ≤ x ≤ 13,
• Lorsque le radical hydrophobe -Hy porte 4 -GpC, alors 7 ≤ x ≤ 11,
• Lorsque le radical hydrophobe -Hy porte au moins 5 -GpC alors, 6 ≤ x ≤ 11 ;
- G est un radical alkyle ramifié de 1 à 8 atomes de carbone ledit radical alkyle portant une ou plusieurs fonction(s) acide carboxylique libre ;
- R est un radical choisi dans le groupe constitué par un radical alkyle divalent, linéaire ou ramifié comprenant de 1 à 12 atomes de carbone, un radical alkyle divalent, linéaire ou ramifié comprenant de 1 à 12 atomes de carbone portant une ou plusieurs fonctions
- CONH₂ ou un radical éther ou polyéther non substitué comprenant de 4 à 14 atomes de carbone et de 1 à 5 atomes d'oxygène ;
- le ou les radicaux hydrophobes -Hy de formule X étant liés au PLG:
o via une liaison covalente entre un carbonyle du radical hydrophobe -Hy et un atome d'azote porté par le PLG formant ainsi une fonction amide issue de la réaction d'une fonction amine portée par le PLG et une fonction acide portée par le précurseur Hy' du radical hydrophobe -Hy, et
∘ via une liaison covalente entre un atome d'azote du radical hydrophobe
- Hy et un carbonyle porté par le PLG formant ainsi une fonction amide issue de la réaction d'une fonction amine du précurseur Hy' du radical hydrophobe -Hy et une fonction acide portée par le PLG ;
- le ratio M entre le nombre de radicaux hydrophobes et le nombre d'unités glutamiques ou aspartiques étant compris entre 0 < M ≤ 0,5 ;
- lorsque plusieurs radicaux hydrophobes sont portés par un co-polyaminoacide alors ils sont identiques ou différents ;
- le degré de polymérisation DP en unités glutamiques ou aspartiques pour les chaines PLG est compris entre 5 et 250 ;
- les fonctions acides carboxyliques libres étant sous forme de sel de cation alcalin choisi dans le groupe constitué par Na⁺ et K⁺.

## Patentansprüche

1. Zusammensetzung in Form einer injizierbaren wässrigen Lösung, deren pH zwischen 6,0 und 8,0 beträgt, wenigstens umfassend :
a) menschliches Glucagon;
b) eine Co-Polyaminosäure, die Carboxylatladungen und hydrophobe Radikale Hy trägt, wobei die Co-Polyaminosäure aus Glutaminsäureeinheiten oder Asparaginsäureeinheiten besteht und die hydrophoben Radikale Hy die folgende Formel X aufweisen: 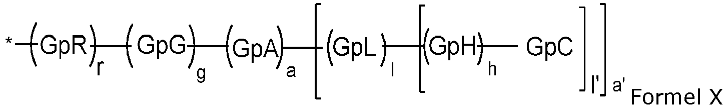 wobei
- GpR ausgewählt ist aus den Radikalen gemäß Formel VII, VII' oder VII" : 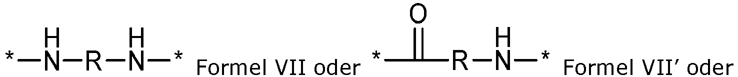 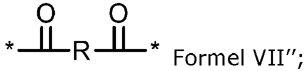
- GpG und GpH identisch oder verschieden sind und ausgewählt sind aus den Radikalen gemäß Formel XI oder XI': 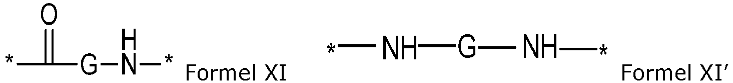
- GpA ausgewählt ist aus den Radikalen gemäß Formel VIII 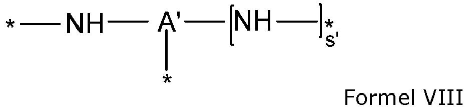 wobei A' ausgewählt ist aus den Radikalen gemäß Formel VIII',VIII" oder VIII‴ 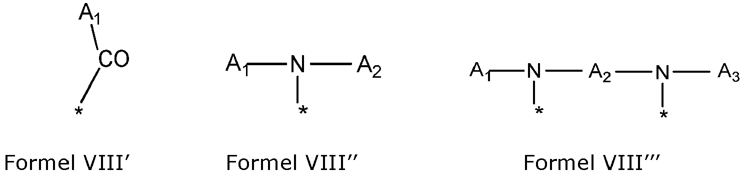
- -GpL ausgewählt ist aus den Radikalen gemäß Formel XII 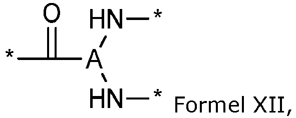
- GpC ein Radikal gemäß Formel IX ist: 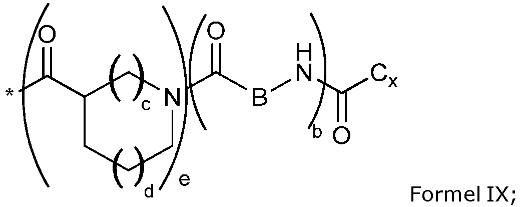
- die * die Bindungsstellen der verschiedenen, durch Amidfunktionen verbundenen Gruppen anzeigt;
- a eine ganze Zahl von 0 oder 1 ist, und a' = 1 ist, wenn a = 0 ist und a' = 1, 2 oder 3 ist, wenn a = 1 ist;
- a' eine ganz Zahl von 1, 2 oder 3 ist;
- b eine ganze Zahl von 0 oder 1 ist;
- c eine ganze Zahl von 0 oder 1 ist, und wenn c gleich 0 ist, dann ist d gleich 1 oder 2 ;
- d eine ganze Zahl von 0, 1 oder 2 ist;
- e eine ganze Zahl von 0 oder 1 ist;
- g eine ganze Zahl von 0, 1, 2, 3, 4, 5 oder 6 ist;
- h eine ganze Zahl von 0, 1, 2, 3, 4, 5 oder 6 ist;
- l eine ganze Zahl von 0 oder 1 ist und l' = 1 ist, wenn l = 0 ist und l' = 2 ist, wenn l = 1 ist;
- r eine ganze Zahl von 0, 1 oder 2 ist;
- s' eine ganze Zahl von 0 oder 1 ist;
- und wenn e verschieden von 0 ist, dann ist wenigstens einer von g, h oder l verschieden von 0;
- und wenn a = 0 ist, dann ist l = 0;
- A, A₁, A₂ und A₃ identisch oder verschieden sind und lineare oder verzweigte Alkylradikale sind umfassend 1 bis 8 Kohlenstoffatome und optional substituiert sind durch ein Radikal eines gesättigten, ungesättigten oder aromatischen Ringes;
- B ein unsubstituiertes Ether- oder Polyetherradikal ist umfassend 4 bis 14 Kohlenstoffatome und 1 bis 5 Sauerstoffatome oder ein lineares oder verzweigtes Alkylradikal ist umfassend 1 bis 9 Kohlenstoffatome, das optional einen aromatischen Kern umfasst;
- Cₓ ein lineares oder verzweigtes monovalentes Alkylradikal ist, das optional einen zyklischen Teil umfasst, in dem x die Anzahl an Kohlenstoffatomen angibt, und:
▪ wenn das hydrophobe Radikal -Hy 1 -GpC trägt, dann ist 9 ≤ x ≤ 25,
▪ wenn das hydrophobe Radikal -Hy 2 -GpC trägt, dann ist 9 ≤ x ≤ 15,
▪ wenn das hydrophobe Radikal -Hy 3 -GpC trägt, dann ist 7 ≤ x ≤ 13,
▪ wenn das hydrophobe Radikal -Hy 4 -GpC trägt, dann ist 7 ≤ x ≤ 11,
▪ wenn das hydrophobe Radikal -Hy mindestens 5 -GpC trägt, dann ist 6 ≤ x ≤ 11 ;
- G ein verzweigtes Alkylradikal ist mit 1 bis 8 Kohlenstoffatomen, wobei das Alkylradikal eine oder mehrere freie Carbonsäurefunktionen trägt;
- R ein Radikal ist, das ausgewählt ist aus der Gruppe bestehend aus einem divalenten linearen oder verzweigten Alkylradikal umfassend 1 bis 12 Kohlenstoffatome, einem divalenten linearen oder verzweigten Alkylradikal umfassend 1 bis 12 Kohlenstoffatome tragend eine oder mehrere -CONH₂-Funktionen oder einem unsubstitutierten Ether- oder Polyetherradikal umfassend 4 bis 14 Kohlenstoffatome und 1 bis 5 Sauerstoffatome;
- das oder die hydrophoben Radikale -Hy gemäß Formel X mit dem PLG verbunden sind:
o vermittels einer kovalenten Bindung zwischen einem Carbonyl des hydrophoben Radikals -Hy und einem von dem PLG getragenen Stickstoffatom, wodurch eine Amidfunktion gebildet ist aus der Reaktion einer von dem PLG getragenen Aminfunktion und einer von dem Vorläufer Hy' des hydrophoben Radikals -Hy getragenen Säurefunktion, und
o vermittels einer kovalenten Bindung zwischen einem Stickstoffatom des hydrophoben Radikals -Hy und einem von dem PLG getragenen Carbonyl, wodurch eine Amidfunktion gebildet ist aus der Reaktion einer von dem Vorläufer Hy' des hydrophoben Radikals -Hy getragenen Aminfunktion und einer von dem PLG getragenen Säurefunktion;
- das Verhältnis M aus der Anzahl an hydrophoben Radikalen und der Anzahl an Glutaminsäureeinheiten oder Asparaginsäureeinheiten 0 < M ≤ 0,5 beträgt;
- wenn mehrere hydrophobe Radikale von einer Co-Polyaminosäure getragen sind, dann sind sie identisch oder verschieden;
- der Polymerisationsgrad PD von Glutaminsäureeinheiten oder Asparaginsäureeinheiten für die PLG-Ketten zwischen 5 und 250 beträgt;
- die freien Carbonsäurefunktionen in der Form eines Salzes eines alkalischen Kations vorliegen, das ausgewählt ist aus der Gruppe bestehend aus Na⁺ und K⁺.

2. Zusammensetzung nach Anspruch 1, **dadurch gekennzeichnet, dass** die Carboxylatladungen und hydrophobe Radikale tragende Co-Polyaminosäure ausgewählt ist aus den Co-Polyaminosäuren gemäß der foglenden Formel XXX: 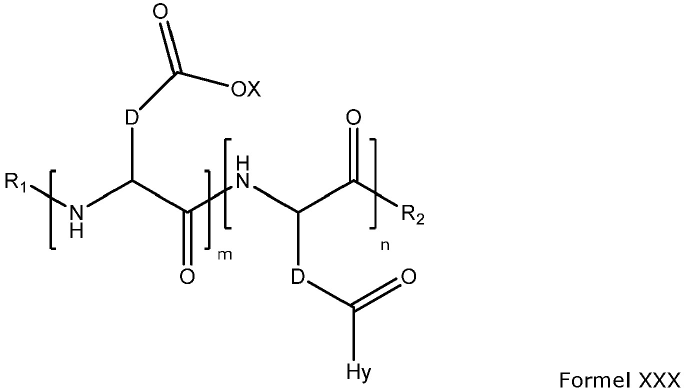 wobei
• D unabhängig entweder eine -CH₂- -Gruppe (Asparaginsäureeinheit) oder eine -CH₂-CH₂- -Gruppe (Glutaminsäureeinheit) darstellt,
• Hy ein hydrophobes Radikal ist, das ausgewählt ist aus den hydrophoben Radikalen gemäß den Formeln X, in denen r = 1 und GpR ein Radikal gemäß Formel VII ist,
• R₁ ein hydrophobes Radikal ist, das ausgewählt ist aus den hydrophoben Radikalen gemäß den Formeln X, wobei r=0 oder r=1 ist und GpR ein Radikal gemäß Formel VII' ist, oder ein Radikal ist, das ausgewählt ist aus der Gruppe bestehend aus einem H, einer linearen C2- bis C10-Acylgruppe, einer verzweigten C3- bis C10-Acylgruppe, einem Benzyl, einer terminalen « Aminosäure »-Einheit und einem Pyroglutamat,
• R₂ ein hydrophobes Radikal ist, das ausgewählt ist aus den hydrophoben Radikalen gemäß den Formeln X, worin r = 1 ist und GpR ein Radikal gemäß Formel VII ist, oder ein Radikal -NR'R" ist, wobei R' und R" identisch oder verschieden sind und ausgewählt sind aus der Gruppe bestehend aus H, linearen, verzweigten oder zyklischen C2- bis C10-Alkylen, Benzyl und die Alkyle R' und R" zusammen einen oder mehrere gesättigte, ungesättigte und/oder aromatische Zyklen bilden können und/oder Heteroatome tragen können, die ausgewählt sind aus der Gruppe bestehend aus O, N und S,
• X ein H oder eine kationische Einheit darstellt, die ausgewählt ist aus der Gruppe umfassend Metallkationen;
• n + m den Polymerisationsgrad DP der Co-Polyaminosäure darstellt, d.h. die durchschnittliche Anzahl von monomeren Einheiten pro Co-Polyaminosäurekette und 5 ≤ n + m ≤ 250 ist.

3. Zusammensetzung nach einem der vorangehenden Ansprüche, **dadurch gekennzeichnet, dass** die Carboxylatladungen und hydrophobe Radikale tragende Co-Polyaminosäure ausgewählt ist aus den Co-Polyaminosäuren gemäß Formel XXX, wobei n = 0 ist gemäß der folgenden Formel XXXb: 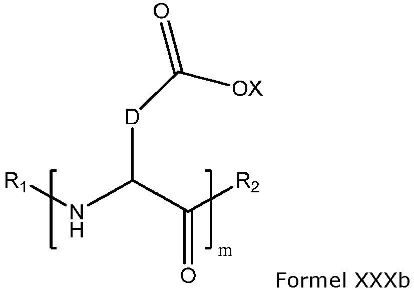 wobei m, X, D, R₁ und R₂ die zuvor angegebenen Definitionen aufweisen und mindestens R₁ oder R₂ ein hydrophobes Radikal gemäß Formel X ist.

4. Zusammensetzung nach Anspruch 3, **dadurch gekennzeichnet, dass** die Carboxylatladungen und hydrophobe Radikale tragende Co-Polyaminosäure ausgewählt ist aus den Co-Polyaminosäuren gemäß Formel XXXb, wobei R₂ ein hydrophobes Radikal gemäß Formel X ist, wobei r = 1 und GpR gemäß Formel VII' ist.

5. Zusammensetzung nach einem der Ansprüche 1 bis 4, **dadurch gekennzeichnet, dass** die Carboxylatladungen und hydrophobe Radikale tragende Co-Polyaminosäure ausgewählt ist aus den Co-Polyaminosäuren gemäß den Formeln XXX, XXXb, in denen mindestens eine Co-Polyaminosäure ausgewählt ist aus den Co-Polyaminosäuren, in denen die Gruppe D eine -CH₂- -Gruppe ist (Asparaginsäureeinheit).

6. Zusammensetzung nach einem der Ansprüche 1 bis 4, **dadurch gekennzeichnet, dass** die Carboxylatladungen und hydrophobe Radikale tragende Co-Polyaminosäure ausgewählt ist aus den Co-Polyaminosäuren gemäß den Formeln XXX, XXXb, in denen mindestens eine Co-Polyaminosäure ausgewählt ist aus den Co-Polyaminosäuren, in denen die Gruppe D eine-CH₂-CH₂- -Gruppe ist (Glutaminsäureeinheit).

7. Zusammensetzung nach einem der vorangehenden Ansprüche, **dadurch gekennzeichnet, dass** die Konzentration an Carboxylatladungen und hydrophobe Radikale tragender Co-Polyaminosäure höchstens 40 mg/mL beträgt.

8. Zusammensetzung nach einem der vorangehenden Ansprüche, **dadurch gekennzeichnet, dass** die Konzentration an menschlichem Glucagon 0,25 bis 5 mg/mL beträgt.

9. Zusammensetzung nach einem der vorangehenden Ansprüche, **dadurch gekennzeichnet, dass** das Molverhältnis [hydrophobes Radikal]/[menschliches Glucagon] kleiner als 15 ist.

10. Zusammensetzung nach einem der vorangehenden Ansprüche, **dadurch gekennzeichnet, dass** sie zusätzlich eine polyanionische Verbindung umfasst.

11. Zusammensetzung nach einem der vorangehenden Ansprüche, **dadurch gekennzeichnet, dass** sie zusätzlich ein Zinksalz umfasst.

12. Zusammensetzung nach einem der vorangehenden Ansprüche, **dadurch gekennzeichnet, dass** sie zusätzlich ein gastrointestinales Hormon umfasst.

13. Co-Polyaminosäure, die Carboxylatladungen und hydrophobe Radikale Hy trägt, ausgewählt aus den Radikalen gemäß Formel X, wie im Folgenden definiert: 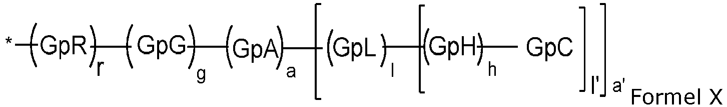 wobei
- GpR ausgewählt ist aus den Radikalen gemäß Formel VII, VII' oder VII" : 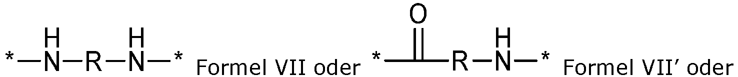 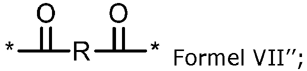
- GpG und GpH identisch oder verschieden sind und ausgewählt sind aus den Radikalen gemäß Formel XI oder XI': 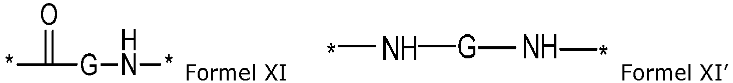
- GpA ausgewählt ist aus den Radikalen gemäß Formel VIII 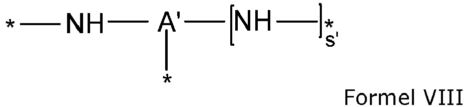 wobei A' ausgewählt ist aus den Radikalen gemäß Formel VIII',VIII" oder VIII‴ 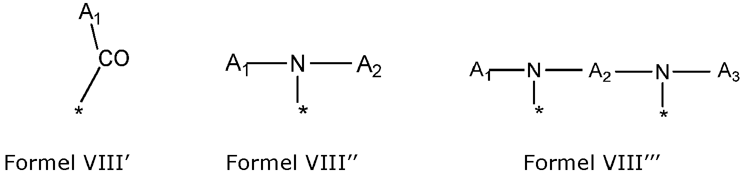
- GpL ausgewählt ist aus den Radikalen gemäß Formel XII 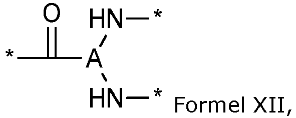
- GpC ein Radikal gemäß Formel IX ist: 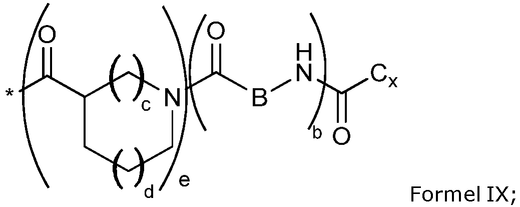
- die * die Bindungsstellen der verschiedenen, durch Amidfunktionen verbundenen Gruppen anzeigt;
- a eine ganze Zahl von 0 oder1 ist, und a' = 1 ist, wenn a = 0 ist und a' = 1, 2 oder 3 ist, wenn a = 1 ist;
- a' eine ganz Zahl von 1, 2 oder 3 ist;
- b eine ganze Zahl von 0 oder 1 ist;
- c eine ganze Zahl von 0 oder 1 ist, und wenn c gleich 0 ist, dann ist d gleich 1 oder 2;
- d eine ganze Zahl von 0, 1 oder 2 ist;
- e eine ganze Zahl von 0 oder 1 ist;
- g eine ganze Zahl von 0, 1, 2, 3, 4, 5 oder 6 ist;
- h eine ganze Zahl von 0, 1, 2, 3, 4, 5 oder 6 ist;
- l eine ganze Zahl von 0 oder 1 ist und l' = 1 ist, wenn l = 0 ist und l' = 2 ist, wenn l = 1 ist;
- r eine ganze Zahl von 0, 1 oder 2 ist;
- s' eine ganze Zahl von 0 oder 1 ist;
- und wenn e verschieden von 0 ist, dann ist wenigstens einer von g, h oder l verschieden von 0;
- und wenn a = 0 ist, dann ist l = 0;
- A, A₁, A₂ und A₃ identisch oder verschieden sind und lineare oder verzweigte Alkylradikale sind umfassend 1 bis 8 Kohlenstoffatome und optional substituiert sind durch ein Radikal eines gesättigten, ungesättigten oder aromatischen Ringes;
- B ein unsubstituiertes Ether- oder Polyetherradikal ist umfassend 4 bis 14 Kohlenstoffatome und 1 bis 5 Sauerstoffatome oder ein lineares oder verzweigtes Alkylradikal ist umfassend 1 bis 9 Kohlenstoffatome, das optional einen aromatischen Kern umfasst;
- Cₓ ein lineares oder verzweigtes monovalentes Alkylradikal ist, das optional einen zyklischen Teil umfasst, in dem x die Anzahl an Kohlenstoffatomen angibt und:
▪ wenn das hydrophobe Radikal -Hy 1 -GpC trägt, dann ist 9 ≤ x ≤ 25,
▪ wenn das hydrophobe Radikal -Hy 2 -GpC trägt, dann ist 9 ≤ x ≤ 15,
▪ wenn das hydrophobe Radikal -Hy 3 -GpC trägt, dann ist 7 ≤ x ≤ 13,
▪ wenn das hydrophobe Radikal -Hy 4 -GpC trägt, dann ist 7 ≤ x ≤ 11,
▪ wenn das hydrophobe Radikal -Hy mindestens 5 -GpC trägt, dann ist 6 ≤ x ≤ 11;
- G ein verzweigtes Alkylradikal ist mit 1 bis 8 Kohlenstoffatomen, wobei das Alkylradikal eine oder mehrere freie Carbonsäurefunktionen trägt;
- R ein Radikal ist, das ausgewählt ist aus der Gruppe bestehend aus einem divalenten linearen oder verzweigten Alkylradikal umfassend 1 bis 12 Kohlenstoffatome, einem divalenten linearen oder verzweigten Alkylradikal umfassend 1 bis 12 Kohlenstoffatome tragend eine oder mehrere -CONH₂-Funktionen oder einem unsubstituierten Ether- oder Polyetherradikal umfassend 4 bis 14 Kohlenstoffatome und 1 bis 5 Sauerstoffatome ;
- das oder die hydrophoben Radikale -Hy der Formel X mit dem PLG verbunden sind:
o vermittels einer kovalenten Bindung zwischen einem Carbonyl des hydrophoben Radikals -Hy und einem von dem PLG getragenen Stickstoffatom, wodurch eine Amidfunktion gebildet ist aus der Reaktion einer von dem PLG getragenen Aminfunktion und einer von dem Vorläufer Hy' des hydrophoben Radikals -Hy getragenen Säurefunktion, und
o vermittels einer kovalenten Bindung zwischen einem Stickstoffatom des hydrophoben Radikals -Hy und einem von dem PLG getragenen Carbonyl, wodurch eine Amidfunktion gebildet ist aus der Reaktion einer von dem Vorläufer Hy' des hydrophoben Radikals -Hy getragenen Aminfunktion und einer von dem PLG getragenen Säurefunktion;
- das Verhältnis M aus der Anzahl an hydrophoben Radikalen und der Anzahl an Glutaminsäureeinheiten oder Asparaginsäureeinheiten 0 < M ≤ 0,5 beträgt;
- wenn mehrere hydrophobe Radikale von einer Co-Polyaminosäure getragen sind, dann sind sie identisch oder verschieden;
- der Polymerisationsgrad PD von Glutaminsäureeinheiten oder Asparaginsäureeinheiten für die PLG-Ketten zwischen 5 und 250 beträgt;
- die freien Carbonlsäurefunktionen in der Form eines Salzes eines alkalischen Kations vorliegen, das ausgewählt ist aus der Gruppe bestehend aus Na⁺ und K⁺.

14. Vorläufer Hy' des hydrophoben Radikals -Hy gemäß Formel X' : 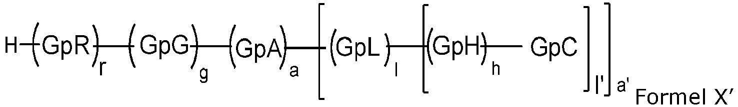 wobei
- GpR ausgewählt ist aus den Radikalen gemäß Formel VII, VII' oder VII" : 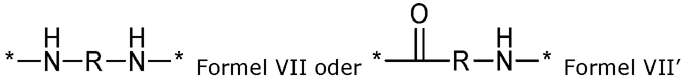 oder 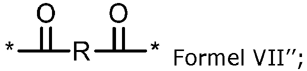
- GpG und GpH identisch oder verschieden sind und ausgewählt sind aus den Radikalen gemäß Formel XI oder XI': 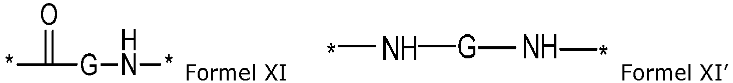
- GpA ausgewählt ist aus den Radikalen gemäß Formel VIII 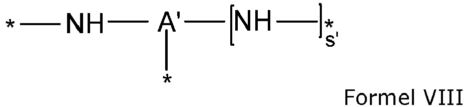 wobei A' ausgewählt ist aus den Radikalen gemäß Formel VIII', VIII" oder VIII‴ 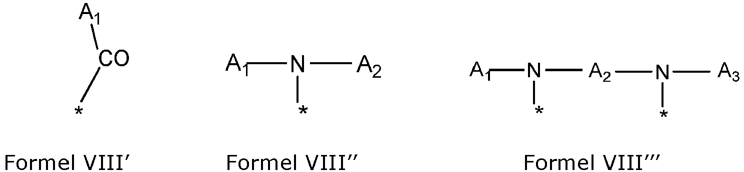
- GpL ausgewählt ist aus den Radikalen gemäß Formel XII 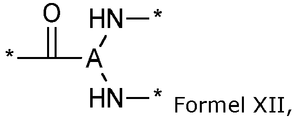
- GpC ein Radikal gemäß Formel IX ist: 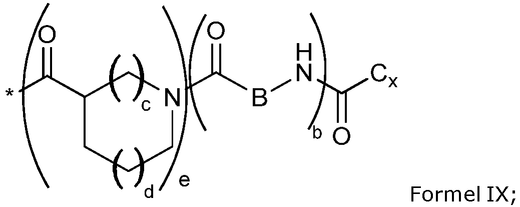
- die * die Bindungsstellen der verschiedenen, durch Amidfunktionen verbundenen Gruppen anzeigt;
- a eine ganze Zahl von 0 oder 1 ist, und a' = 1 ist, wenn a = 0 ist und a' = 1, 2 oder 3 ist, wenn a = 1 ist;
- a' eine ganz Zahl von 1, 2 oder 3 ist;
- b eine ganze Zahl von 0 oder 1 ist;
- c eine ganze Zahl von 0 oder 1 ist, und wenn c gleich 0 ist, dann ist d gleich 1 oder 2;
- d eine ganze Zahl von 0, 1 oder 2 ist;
- e eine ganze Zahl von 0 oder 1 ist;
- g eine ganze Zahl von 0, 1, 2, 3, 4, 5 oder 6 ist;
- h eine ganze Zahl von 0, 1, 2, 3, 4, 5 oder 6 ist;
- l eine ganze Zahl von 0 oder 1 ist und l' = 1 ist, wenn l = 0 ist und l' = 2 ist, wenn l = 1 ist;
- r eine ganze Zahl von 0, 1 oder 2 ist;
- s' eine ganze Zahl von 0 oder 1 ist;
- und wenn e verschieden von 0 ist, dann ist wenigstens einer von g, h oder l verschieden von 0;
- und wenn a = 0 ist, dann ist l = 0;
- A, A₁, A₂ und A₃ identisch oder verschieden sind und lineare oder verzweigte Alkylradikale sind umfassend 1 bis 8 Kohlenstoffatome und optional substituiert sind durch ein Radikal eines gesättigten, ungesättigten oder aromatischen Ringes;
- B ein unsubstituiertes Ether- oder Polyetherradikal ist umfassend 4 bis 14 Kohlenstoffatome und 1 bis 5 Sauerstoffatome oder ein lineares oder verzweigtes Alkylradikal ist umfassend 1 bis 9 Kohlenstoffatome, das optional einen aromatischen Kern umfasst;
- Cₓ ein lineares oder verzweigtes monovalentes Alkylradikal ist, das optional einen zyklischen Teil umfasst, in dem x die Anzahl an Kohlenstoffatomen angibt und:
▪ wenn das hydrophobe Radikal -Hy 1 -GpC trägt, dann ist 9 ≤ x ≤ 25,
▪ wenn das hydrophobe Radikal -Hy 2 -GpC trägt, dann ist 9 ≤ x ≤ 15,
▪ wenn das hydrophobe Radikal -Hy 3 -GpC trägt, dann ist 7 ≤ x ≤ 13,
▪ wenn das hydrophobe Radikal -Hy 4 -GpC trägt, dann ist 7 ≤ x ≤ 11,
▪ wenn das hydrophobe Radikal -Hy mindestens 5 -GpC trägt, dann ist 6 ≤ x ≤ 11;
- G ein verzweigtes Alkylradikal ist mit 1 bis 8 Kohlenstoffatomen, wobei das Alkylradikal eine oder mehrere freie Carbonsäurefunktionen trägt;
- R ein Radikal ist, das ausgewählt ist aus der Gruppe bestehend aus einem divalenten linearen oder verzweigten Alkylradikal umfassend 1 bis 12 Kohlenstoffatome, einem divalenten linearen oder verzweigten Alkylradikal umfassend 1 bis 12 Kohlenstoffatome tragend eine oder mehrere -CONH₂-Funktionen oder einen unsubstitutierten Ether- oder Polyetherradikal umfassend 4 bis 14 Kohlenstoffatome und 1 bis 5 Sauerstoffatome;
- das oder die hydrophoben Radikale -Hy gemäß Formel X mit dem PLG verbunden sind:
o vermittels einer kovalenten Bindung zwischen einem Carbonyl des hydrophoben Radikals -Hy und einem von dem PLG getragenen Stickstoffatom, wodurch eine Amidfunktion gebildet ist aus der Reaktion einer von dem PLG getragenen Aminfunktion und einer von dem Vorläufer Hy' des hydrophoben Radikals -Hy getragenen Säurefunktion, und
o vermittels einer kovalenten Bindung zwischen einem Stickstoffatom des hydrophoben Radikals -Hy und einem von dem PLG getragenen Carbonyl, wodurch eine Amidfunktion gebildet ist aus der Reaktion einer von dem Vorläufer Hy' des hydrophoben Radikals -Hy getragenen Aminfunktion und einer von dem PLG getragenen Säurefunktion;
- das Verhältnis M aus der Anzahl an hydrophoben Radikalen und der Anzahl an Glutaminsäureeinheiten oder Asparaginsäureeinheiten 0 < M ≤ 0,5 beträgt;
- wenn mehrere hydrophobe Radikale von einer Co-Polyaminosäure getragen sind, dann sind sie identisch oder verschieden;
- der Polymerisationsgrad PD an Glutaminsäureeinheiten oder Asparaginsäureeinheiten für die PLG-Ketten zwischen 5 und 250 beträgt;
- die freien Carbonlsäurefunktionen in der Form eines Salzes eines alkalischen Kations vorliegen, das ausgewählt ist aus der Gruppe bestehend aus Na⁺ und K⁺.

## Claims

1. Composition in the form of an injectable aqueous solution, for which the pH is comprised from 6.0 to 8.0, comprising at least:
a) human glucagon;
b) a co-polyamino acid bearing carboxylate charges and hydrophobic radicals Hy, said co-polyamino acid being constituted of glutamic or aspartic units and said hydrophobic radicals Hy chosen according to formula X as defined below: 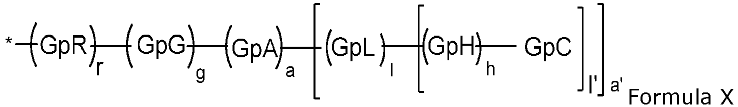 in which
- GpR is chosen among the radicals according to formulasaccording to formulas VII, VII' or VII": 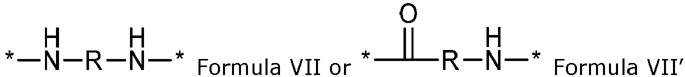 or 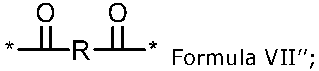
- Identical or different GpG and GpH are chosen among the radicals according to formulas XI or XI'; 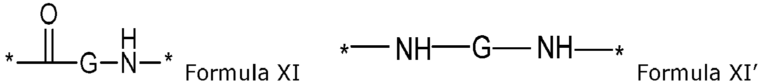
- GpA is chosen among the radicals according to formula VIII 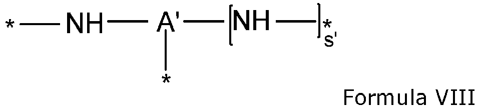 In which A' is chosen among the radicals according to formulas VIII', VIII" or VIII'" 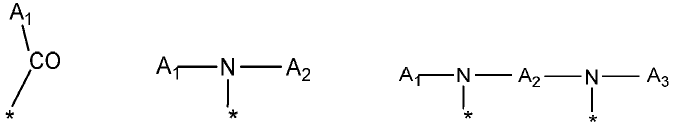 Formula VIII' or Formula VIII" or Formula VIII‴
- -GpL is chosen among the radicals according to formula XII 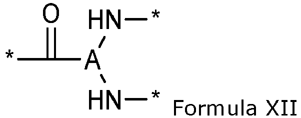
- GpC is a radical according to formula IX: 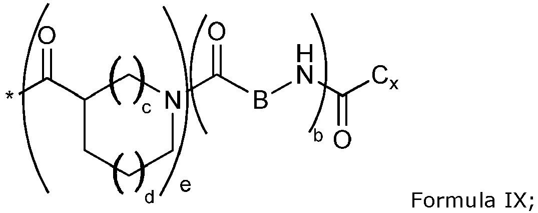
- *indicate the attachment sites of the different groups bound by amide functions;
- a is an integer equal to 0 or to 1 and a' = 1 if a = 0 and a' = 1, 2 or 3 if a =1;
- a' is an integer equal to 1, to 2 or to 3;
- b is an integer equal to 0 or to 1;
- c is an integer equal to 0 or to 1, and if c is equal to 0, then d is equal to 1 or to 2;
- d is an integer equal to 0, to 1 or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6;
- l is an integer equal to 0 or to 1 and l' = 1 if l = 0 and l' = 2 if l = 1;
- r is an integer equal to 0, 1 or to 2;
- s' is an integer equal to 0 or to 1;
- And if e is different from 0, then at least one of g, h or l is different from 0;
- And if a = 0, then l = 0;
- A, A₁, A₂ and A₃ identical or different, are linear or branched alkyl radicals comprising from 1 to 8 carbon atoms and, optionally, substituted by a radical from a saturated, unsaturated or aromatic ring;
- B is a radical ether or polyether, unsubstituted, comprising from 4 to 14 carbon atoms and from 1 to 5 oxygen atoms, or a linear or branched alkyl radical, optionally comprising an aromatic ring, comprising from 1 to 9 carbon atoms.;
- Cₓ is a linear or branched, monovalent alkyl radical optionally comprising a cyclic part, in which x indicates the number of carbon atoms, and:
▪ When the hydrophobic radical -Hy bears 1 -GpC, then 9 ≤ x ≤ 25,
▪ When the hydrophobic radical -Hy bears 2 -GpC, then 9 ≤ x ≤ 15,
▪ When the hydrophobic radical -Hy bears 3 -GpC, then 7 ≤ x ≤ 13,
▪ When the hydrophobic radical -Hy bears 4 -GpC, then 7 ≤ x ≤ 11,
▪ When the hydrophobic radical -Hy bears at least 5 -GpC; 6 ≤ x ≤ 11;
- G is a branched alkyl radical of 1 to 8 carbon atoms, said alkyl radical bears one or more free carboxylic acid functions.
- R is a radical chosen from the group consisting of a divalent, linear or branched alkyl radical comprising from 1 to 12 carbon atoms, a divalent, linear or branched alkyl radical comprising from 1 to 12 carbon atoms bearing one or more -CONH₂ functions or an unsubstituted ether or polyether radical comprising from 4 to 14 carbon atoms and from 1 to 5 oxygen atoms.
- the hydrophobic radical(s) - Hy according to formula X being bound to the PLG:
o via a covalent bond between a carbonyl of hydrophobic radical -Hy and a nitrogen atom borne by the PLG, thus forming an amide function resulting from the reaction of an amine function borne by the PLG and an acid function borne by the precursor Hy' of the hydrophobic radical -Hy, and
∘ via a covalent bond between a nitrogen atom of the hydrophobic radical -Hy and a carbonyl borne by the PLG; thus forming an amide function resulting from the reaction of an amine function of the precursor -Hy' of the hydrophobic radical -Hy and an acid function borne by the PLG.
- The ratio M between the number of hydrophobic radicals and the number of glutamic or aspartic unites being between 0 < M ≤ 0.5;
- when several hydrophobic radicals are borne by a co-polyamino acid, then they are identical or different,
- The degree of polymerization DP in glutamic or aspartic units for the PLG chains is comprised from 5 to 250;
- Free carboxylic acids being in the form of an alkaline cation salt chosen from the group consisting of Na⁺ and K⁺.

2. Composition according to claim 1, **characterized in that** the co-polyamino acid bearing carboxylate charges and hydrophobic radicals is chosen among the co-polyamino acids according to formula XXX below: 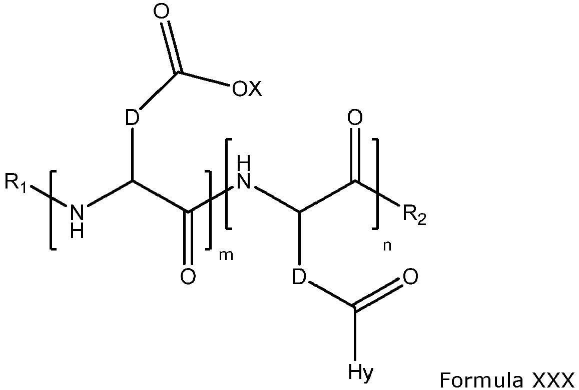 in which,
• D represents, independently, either a -CH₂- group (aspartic unit) of a -CH₂-CH₂- group (glutamic unit),
• Hy is a hydrophobic radical chosen among the hydrophobic radicals according to formula X, in which r = 1 and GpR is a radical according to formula VII.
• R₁ is a hydrophobic radical chosen among the hydrophobic radicals according to formula X in which r=0 or r=1 and GpR is a radical according to formula VII', or a radical chosen from the group consisting of an H, a linear acyl group in C2 to C10, a branched acyl group in C3 to C10, a benzyl, an end "amino acid" unit and a pyroglutamate,
• R₂ is a hydrophobic radical chosen among the hydrophobic radicals according to formula X in which r = 1 and GpR is a radical according to formula VII or an -NR'R", R' and R" radical, identical or different, being chosen from the group consisting of H, the linear, branched or cyclic alkyls in C2 to C10, benzyl and said R' and R" alkyls being able to together form one or more saturated, unsaturated and/or aromatic rings and/or being able to comprise heteroatoms, chosen from the group consisting of O, N and S,
• X represents a H or a cationic entity chosen from the group comprising the metallic cations;
• n + m represents the degree of polymerization DP of the co-polyamino acid, that is the average number of monomeric units per co-polyamino acid chain and 5 ≤ n + m ≤ 250;
• n + m represents the degree of polymerization DP of the co-polyamino acid, that is the average number of monomeric units per co-polyamino acid chain and 5 ≤ n + m ≤ 250.

3. Composition according to any one of the preceding claims, **characterized in that** the co-polyamino acid bearing carboxylate charges and hydrophobic radicals is chosen among the co-polyamino acids according to formula XXX, in which n = 0 according to formula XXXb below: 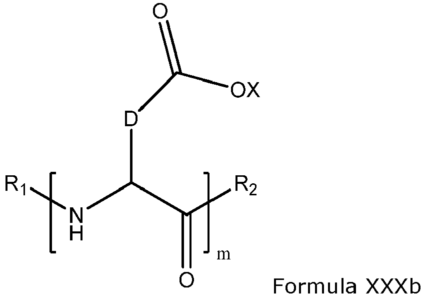 in which m, X, D, R₁ and R₂ have the definitions given above and at least R₁ or R₂ is a hydrophobic radical according to formula X.

4. Composition according to claim 3, **characterized in that** the co-polyamino acid bearing carboxylate charges and hydrophobic radicals is chosen among the co-polyamino acids according to formula XXXb, in which R₂ is a hydrophobic radical according to formula X in which r = 1 and GpR is according to formula VII'.

5. Composition according to any one of claims 1 to 4, **characterized in that** the co-polyamino acid bearing carboxylate charges and hydrophobic radicals is chosen among the co-polyamino acids according to formula XXX, XXXb, in which at least one co-polyamino acid is chosen among the co-polyamino acids in which the D group is a -CH₂- group (aspartic unit).

6. Composition according to any one of claims 1 to 4, **characterized in that** the co-polyamino acid bearing carboxylate charges and hydrophobic radicals is chosen among the co-polyamino acids according to formula XXX, XXXb, in which at least one co-polyamino acid is chosen among the co-polyamino acids in which the D group is a -CH₂-CH₂- group (glutamic unit).

7. Composition according to any one of the preceding claims, **characterized in that** the concentration of co-polyamino acid bearing carboxylate charges and hydrophobic radicals is at most of 40 mg/mL.

8. Composition according to any one of the preceding claims, **characterized in that** the concentration of human glucagon is comprised from 0.25 to 5 mg/mL.

9. Composition according to any one of the preceding claims, **characterized in that** the[hydrophobic radical]/[human glucagon] molar ratio is greater than or equal to 15.

10. Composition according to any one of the preceding claims, **characterized in that** it also comprises a polyanionic compound.

11. Composition according to any one of the preceding claims, **characterized in that** it also comprises a zinc salt.

12. Composition according to any one of the preceding claims, **characterized in that** it also comprises a gastro-intestinal hormone.

13. Co-polyamino acid bearing carboxylate charges and hydrophobic radicals Hy, chosen among the radicals according to formula X as defined below: 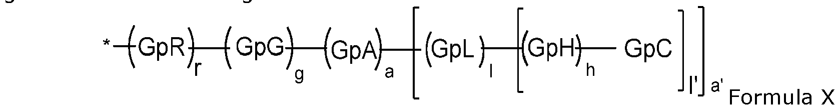 in which
- GpR is chosen among the radicals according to formulas VII, VII' or VII": 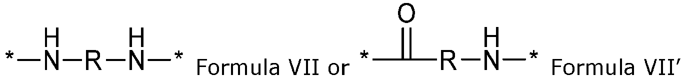 or 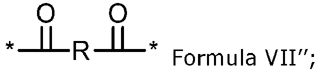
- Identical or different GpG and GpH are chosen among the radicals according to formulas XI or XI'; 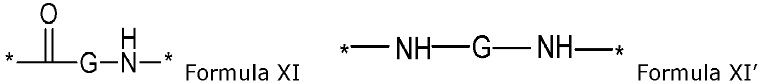
- GpA is chosen among the radicals according to formulas VIII 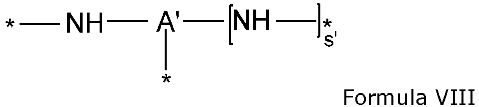 In which A' is chosen among the radicals according to formulas VIII', VIII" or VIII'" 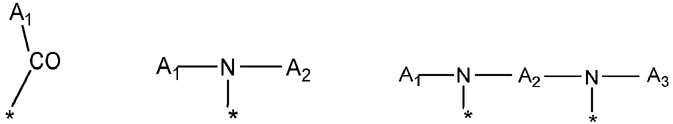 Formula VIII' or Formula VIII" or Formula VIII‴
- GpL is chosen among the radicals according to formula XII 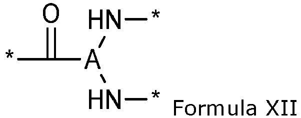
- GpC is a radical according to formula IX: 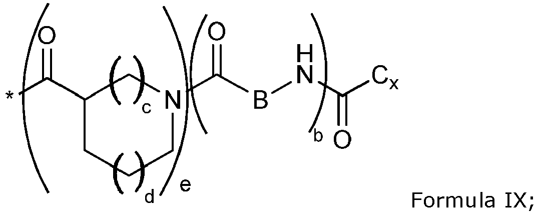
- *indicate the attachment sites of the different groups bound by amide functions;
- a is an integer equal to 0 or to 1 and a' = 1 if a = 0 and a' = 1, 2 or 3 if a =1;
- a' is an integer equal to 1, to 2 or to 3;
- b is an integer equal to 0 or to 1;
- c is an integer equal to 0 or to 1, and if c is equal to 0, then d is equal to 1 or to 2;
- d is an integer equal to 0, to 1 or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6;
- l is an integer equal to 0 or to 1 and l' = 1 if l = 0 and l' = 2 if l = 1;
- r is an integer equal to 0, 1 or to 2;
- s' is an integer equal to 0 or to 1;
- And if e is different from 0, then at least one of g, h or l is different from 0;
- And if a = 0, then l = 0;
- A, A₁, A₂ and A₃ identical or different, are linear or branched alkyl radicals comprising from 1 to 8 carbon atoms and, optionally, substituted by a radical from a saturated, unsaturated or aromatic ring;
- B is a radical ether or polyether, unsubstituted, comprising from 4 to 14 carbon atoms and from 1 to 5 oxygen atoms, or a linear or branched alkyl radical, optionally comprising an aromatic ring, comprising from 1 to 9 carbon atoms.;
- Cₓ is a linear or branched, monovalent alkyl radical optionally comprising a cyclic part, in which x indicates the number of carbon atoms, and:
▪ When the hydrophobic radical -Hy bears 1 -GpC, then 9 ≤ x ≤ 25,
▪ When the hydrophobic radical -Hy bears 2 -GpC, then 9 ≤ x ≤ 15,
▪ When the hydrophobic radical -Hy bears 3 -GpC, then 7 ≤ x ≤ 13,
▪ When the hydrophobic radical -Hy bears 4 -GpC, then 7 ≤ x ≤ 11,
▪ When the hydrophobic radical -Hy bears at least 5 -GpC; 6 ≤ x ≤ 11;
- G is a branched alkyl radical of 1 to 8 carbon atoms, said alkyl radical bears one or more free carboxylic acid functions.
- R is a radical chosen from the group consisting of a divalent, linear or branched alkyl radical comprising from 1 to 12 carbon atoms, a divalent, linear or branched alkyl radical comprising from 1 to 12 carbon atoms bearing one or more -CONH₂ functions or an unsubstituted ether or polyether radical comprising from 4 to 14 carbon atoms and from 1 to 5 oxygen atoms.
- the hydrophobic radical(s) - Hy according to formula X being bound to the PLG:
o via a covalent bond between a carbonyl of hydrophobic radical -Hy and a nitrogen atom borne by the PLG, thus forming an amide function resulting from the reaction of an amine function borne by the PLG and an acid function borne by the precursor Hy' of the hydrophobic radical -Hy, and
∘ via a covalent bond between a nitrogen atom of the hydrophobic radical -Hy and a carbonyl borne by the PLG; thus forming an amide function resulting from the reaction of an amine function of the precursor -Hy' of the hydrophobic radical -Hy and an acid function borne by the PLG.
- The ratio M between the number of hydrophobic radicals and the number of glutamic or aspartic unites being between 0 < M ≤ 0.5;
- when several hydrophobic radicals are borne by a co-polyamino acid, then they are identical or different,
- The degree of polymerization DP in glutamic or aspartic units for the PLG chains is comprised from 5 to 250;
- Free carboxylic acids being in the form of an alkaline cation salt chosen from the group consisting of Na⁺ and K⁺.

14. Hy' precursor of the -Hy hydrophobic radical according to formula X': 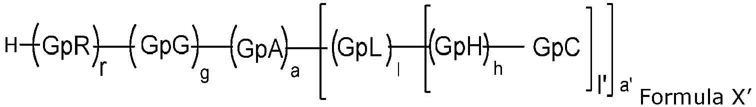 in which
- GpR is chosen among the radicals according to formulas VII, VII' or VII": 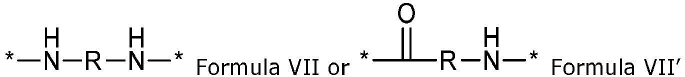 or 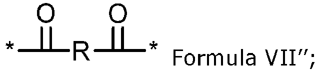
- Identical or different GpG and GpH are chosen among the radicals according to formulas XI or XI'; 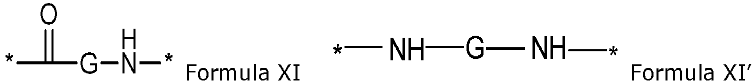
- GpA is chosen among the radicals according to formulas VIII 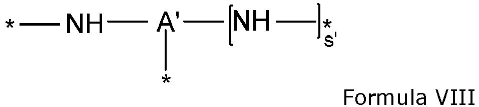 In which A' is chosen among the radicals according to formulas VIII', VIII" or VIII'" 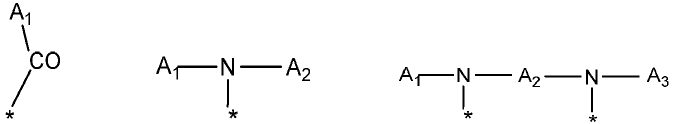 Formula VIII' or Formula VIII" or Formula VIII‴
- -GpL is chosen among the radicals according to formulas XII 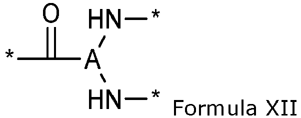
- GpC is a radical according to formula IX: 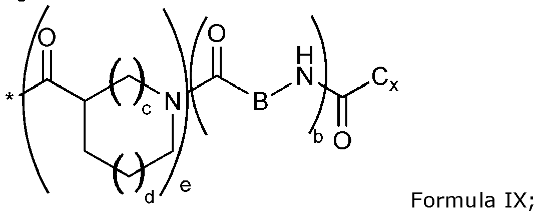
- *indicate the attachment sites of the different groups linked by amide functions;
- a is an integer equal to 0 or to 1 and a' = 1 if a = 0 and a' = 1, 2 or 3 if a =1;
- a' is an integer equal to 1, to 2 or to 3;
- b is an integer equal to 0 or to 1;
- c is an integer equal to 0 or to 1, and if c is equal to 0, then d is equal to 1 or to 2;
- d is an integer equal to 0, to 1 or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6;
- l is an integer equal to 0 or to 1 and l' = 1 if l = 0 and l' = 2 if l = 1;
- r is an integer equal to 0, 1 or to 2;
- s' is an integer equal to 0 or to 1;
- And if e is different from 0, then at least one of g, h or l is different from 0;
- And if a = 0, then l = 0;
- A, A₁, A₂ and A₃ identical or different, are linear or branched alkyl radicals comprising from 1 to 8 carbon atoms and, optionally, substituted by a radical from a saturated, unsaturated or aromatic ring;
- B is a radical ether or polyether, unsubstituted, comprising from 4 to 14 carbon atoms and from 1 to 5 oxygen atoms, or a linear or branched alkyl radical, optionally comprising an aromatic ring, comprising from 1 to 9 carbon atoms.;
- Cₓ is a linear or branched, monovalent alkyl radical optionally comprising a cyclic part, in which x indicates the number of carbon atoms, and:
▪ When the hydrophobic radical -Hy bears 1 -GpC, then 9 ≤ x ≤ 25,
▪ When the hydrophobic radical -Hy bears 2 -GpC, then 9 ≤ x ≤ 15,
▪ When the hydrophobic radical -Hy bears 3 -GpC, then 7 ≤ x ≤ 13,
▪ When the hydrophobic radical -Hy bears 4 -GpC, then 7 ≤ x ≤ 11,
▪ When the hydrophobic radical -Hy bears at least 5 -GpC; 6 ≤ x ≤ 11;
- G is a branched alkyl radical of 1 to 8 carbon atoms, said alkyl radical bears one or more free carboxylic acid functions.
- R is a radical chosen from the group consisting of a divalent, linear or branched alkyl radical comprising from 1 to 12 carbon atoms, a divalent, linear or branched alkyl radical comprising 1 to 12 carbon atoms bearing one or more -CONH₂ functions or an unsubstituted ether or polyether radical comprising from 4 to 14 carbon atoms and from 1 to 5 oxygen atoms.
- the hydrophobic radical(s) - Hy according to formula X being bound to the PLG:
o via a covalent bond between a carbonyl of hydrophobic radical -Hy and a nitrogen atom borne by the PLG, thus forming an amide function resulting from the reaction of an amine function borne by the PLG and an acid function borne by theprecursor Hy' of the hydrophobic radical -Hy, and
∘ via a covalent bond between a nitrogen atom of the hydrophobic radical -Hy and a carbonyl borne by the PLG; thus forming an amide function resulting from the reaction of an amine function of the precursor -Hy' of the hydrophobic radical -Hy and an acid function borne by the PLG.
- The ratio M between the number of hydrophobic radicals and the number of glutamic or aspartic unites being comprised from 0 < M ≤ 0.5;
- when several hydrophobic radicals are borne by a co-polyamino acid, then they are identical or different,
- The degree of polymerization DP in glutamic or aspartic units for the PLG chains is comprised from 5 to 250;
- Free carboxylic acids being in the form of an alkaline cation salt chosen from the group consisting of Na⁺ and K⁺.
